# EUROPEAN PATENT APPLICATION

(11) **EP 2 410 062 A1**
(43) Date of publication of application: **25.01.2012**
(21) Application number: 11008204.7
(22) Date of filing: 03.02.2009
(51) Int. Cl.: C12Q 1/48, G01N 33/573

(54) **Selectivity profiling of PI3K interacting molecules against multiple targets**

(30) Priority: 04.02.2008 EP 08002053
(62) Divisional of application: 09709335.5
(71) Applicant: CELLZOME AG, 69117 Heidelberg (DE)
(72) Inventor: Cansfield, Andrew, David, Cambridge, CB22 7QT (GB); Bergamini Moore, Giovanna, 69121 Heidelberg (DE); Neubauer, Gitte, Dr., 68259 Mannheim (DE)
(74) Representative: Lahrtz, Fritz

(57) **Abstract**

The present invention relates to methods wherein a PI3K interacting compound is identified by incubating a PI3K containing protein preparation with phenylthiazole ligand 1.

## Description

The present invention relates to methods for the identification and characterization of PI3K interacting molecules and for the purification of PI3K using phenylthiazole ligand 1 as a ligand for PI3K. Furthermore, the present invention relates to pharmaceutical compositions comprising said interacting molecules e.g. for the treatment of cancer, metabolic diseases or autoimmune/inflammatory disorders.

Kinases catalyze the phosphorylation of proteins, lipids, sugars, nucleosides and other cellular metabolites and play key roles in all aspects of eukryotic cell physiology. Especially, protein kinases and lipid kinases participate in the signaling events which control the activation, growth, differentiation and survival of cells in response to extracellular mediators or stimuli such as growth factors, cytokines or chemokines. In general, protein kinases are classified in two groups, those that preferentially phosphorylate tyrosine residues and those that preferentially phosphorylate serine and/or threonine residues.

Inappropriately high protein kinase activity is involved in many diseases including cancer, metabolic diseases and autoimmune/inflammatory disorders. This can be caused either directly or indirectly by the failure of control mechanisms due to mutation, overexpression or inappropriate activation of the enzyme. In all of these instances, selective inhibition of the kinase is expected to have a beneficial effect.

One group of lipid kinases that has become a recent focus of drug discovery is the phosphoinositide 3-kinase (PI3K) family. Members of the PI3K family are lipid kinases that catalyse the transfer of the gamma-phosphate from ATP to the 3'-hydroxyl group of phophatidylinositol and its derivatives, collectively called phosphoinositides. Eight members (isoforms) of the PI3K family have been isolated from mammalian cells so far and grouped into three classes according to their primary structure and substrate specificity (class IA: PI3K alpha, beta and delta; class IB: PI3K gamma; class II: PI3KC2 alpha, beta and gamma; class III: Vps34 yeast homologue) (Fruman et al., 1998. Phosphoinositide kinases. Annual Review Biochemistry 67, 481-507; Cantley, L.C., 2002, Science 296, 1655-1657).

Mammalian cells are known to express three isoforms of the catalytic subunit of PI3K IA class (p110 alpha, p110 beta and p110 delta, synonym "PI3K delta"). Class IB contains only one member (catalytic subunit) which has been named p110 gamma or PI3K gamma. In addition to its lipid kinase activity PI3K gamma exhibits also a serine/threonine protein kinase acitivity as demonstrated by autophosphorylation.

The study of genetically manipulated mice in which the genes encoding PI3K gamma or delta were deleted give important information about the physiological function of these kinases and their potential utility as drug targets. Mice lacking PI3K gamma or delta are viable and exhibit distinctive phenotypes suggesting several potential therapeutic indications. PI3K gamma appears to be a major mediator of the innate immune system. For example, PI3K gamma deficient macrophages and neutrophilic granulocytes display an impaired ability to infiltrate the inflamed peritoneum. Mast cells represent another cell type affected in PI3K gamma deficient mice. The phenotype of mice lacking PI3K delta is characterized by an impairment of lymphocyte functions and point to a dominant function in the control of the adaptive immune response (Wetzker and Rommel, Current Pharmaceutical Design, 2004, 10, 1915-1922).

In contrast to the widely expressed PI3K alpha and beta isoforms the hematopoietic specific isoforms PI3K gamma and delta suggest important therapeutic indications. Both isoforms appear as ideal targets for the treatment of autoimune/inflammatory diseases mediated by hyperactive phagocytes, mast cells, B-and T-lymphocytes (e.g. rheumatoid arthritis, asthma or allergic reactions). In order to avoid unwanted side effects highly isoform selective inhibitors are necessary (Ohashi and Woodgett 2005, Nature Medicine 11, 924-925).

Members of the phosphatidylinositol kinase-related kinase (PIKK) family are high molecular mass kinases involved in cell cycle progression, DNA recombination, and the detection of DNA damage. The human ATM gene, which is defective in cells of patients with ataxia-telangiectasia and is involved in detection and response of cells to damaged DNA, is a member of this family. Another is mTOR (synonym FRAP), which is involved in a rapamycin-sensitive pathway leading to G1 cell cycle progression (Shilo, 2003. Nature Reviews Cancer 3, 155-168).

One prerequisite for the identification and characterization of PI3K inhibitors is the provision of suitable assays, preferably physiological forms of the protein target. In the art, several strategies have been proposed to address this issue.

Conventionally, PI3K lipid kinase activity can be measured using purified or recombinant enzyme in a solution-based assay with phopholipid vesicles. The reaction is terminated by the addition of acidified organic solvents and subsequent phase separation by extraction or thin layer chromatography analysis (Carpenter et al., 1990, J. Biol. Chem. 265, 19704-19711).

Another assay described in the art is based on the phosphate transfer from radiolabeled ATP to phosphatidylinositol immobilized on plates. This assay type also uses recombinant PI3K gamma enzyme but can be performed in a high throughput mode (Fuchikami et al., 2002, J. Biomol. Screening 7, 441-450).

Yet another biochemical screening assay is based on a competitive fluorescence polarization (FP) format using fluorophore-labeled phosphoinositide (Drees et al., 2003, Comb. Chem. High Throughput Screening 6, 321-330).

Finally, a cell-based Akt-EGFP redistribution assay was reported based on fluorescence microscopic imaging and automated image analysis. To this end Chinese Hamster Ovary (CHO) cells were stably transfected with the human insulin receptor and an Akt1-enhanced green fluorescent protein (EGFP) fusion construct. After stimulation with insulin-like growth factor-1 (IGF-1) PI3K was activated and the Akt1-EGFP protein was recruited to the cell membrane. The validation of the redistribution assay with PI3K isoform selective inhibitors showed that PI3K alpha is the main isoform activated in CHO host cells after IGF-1 stimulation (Wolff et al., Comb. Chem. High Throughput Screen. 9, 339-350).

Another, although not in all instances necessary prerequisite for the identification of selective kinase inhibitors is a method that allows to determine the target selectivity of these molecules. For example, it can be intended to provide molecules that bind to and inhibit a particular drug target but do not interact with a closely related target, inhibition of which could lead to side effects. Conventionally large panels of individual enzyme assays are used to assess the inhibitory effect of a compound for kinases (Knight et al., 2004. Bioorganic and Medicinal Chemistry 12, 4749-4759; Knight et al., 2006, Cell 125, 733-747). More recently, kinases or kinase domains displayed on bacteriophages have been employed to assess the ability of a given compound to interact with a large set of kinases (Karaman et al., 2008. Nature Biotechnology 26, 127-132). In addition, chemical proteomics methods have been described which allow the profiling of kinase inhibitors against the proteome (WO 2006/134056; Bantscheff et al., 2007. Nature Biotechnology 25, 1035-1044; Patricelly et al., 2007. Biochemistry 46, 350-358; Gharbi et al., 2007. Biochem. J. 404, 15-21; WO2008/015013).

In view of the above, there is a need for providing effective methods for the identification and selectivity profiling of PI3K interacting compounds as well as for methods for the purification of PI3K.

To comply with this need, the invention provides in a first aspect a method for the identification of a PI3K interacting compound, comprising the steps of
a) providing a protein preparation containing PI3K,
b) contacting the protein preparation with phenylthiazole ligand 1 immobilized on a solid support under conditions allowing the formation of a phenylthiazole ligand 1 - PI3K complex,
c) incubating the phenylthiazole ligand 1 - PI3K complex with a given compound,
d) determining whether the compound is able to separate PI3K from the immobilized phenylthiazole ligand 1, and
e) determining whether the compound is able to separate also ATM, ATR, DNAPK and/or mTOR from the immobilized phenylthiazole ligand 1.

In a second aspect, the present invention relates to a method for the identification of a PI3K interacting compound, comprising the steps of
a) providing a protein preparation containing PI3K,
b) contacting the protein preparation with phenylthiazole ligand 1 immobilized on a solid support and with a given compound under conditions allowing the formation of a phenylthiazole ligand 1 - PI3K complex,
c) detecting the phenylthiazole ligand 1 - PI3K complex formed in step b), and
d) detecting whether also a complex between phenylthiazole ligand 1 and ATM, ATR, DNAPK and or mTOR has been formed in step b).

In a third aspect, the invention provides a method for the identification of a PI3K interacting compound, comprising the steps of:
a) providing two aliquots of a protein preparation containing PI3K,
b) contacting one aliquot with the phenylthiazole ligand 1 immobilized on a solid support under conditions allowing the formation of a phenylthiazole ligand 1 - PI3K complex,
c) contacting the other aliquot with the phenylthiazole ligand 1 immobilized on a solid support and with a given compound under conditions allowing the formation of a phenylthiazole ligand 1 - PI3K complex,
d) determining the amount of the phenylthiazole ligand 1 - PI3K complex formed in steps b) and c), and
e) determining whether also a complex between phenylthiazole ligand 1 and ATM, ATR, DNAPK and or mTOR has been formed in steps b) and c).

In a fourth aspect, the invention relates to a method for the identification of a PI3K interacting compound, comprising the steps of:
a) providing two aliquots comprising each at least one cell containing PI3K,
b) incubating one aliquot with a given compound,
c) harvesting the cells of each aliquot,
d) lysing the cells in order to obtain protein preparations,
e) contacting the protein preparations with the phenylthiazole ligand 1 immobilized on a solid support under conditions allowing the formation of a phenylthiazole ligand 1 - PI3K complex, and
f) determining the amount of the phenylthiazole ligand 1 - PI3K complex formed in each aliquot in step e), and
g) determining whether also a complex between phenylthiazole ligand 1 and ATM, ATR, DNAPK and or mTOR has been formed in step e).

In the context of the present invention, it has been surprisingly found that phenylthiazole ligand 1 is a PI3K ligand and a ligand of other members of the PIKK family, namely ATM, ATR, DNAPK and mTOR (FRAP). This enables the use of phenylthiazole ligand 1 in screening assays, e.g. in competitive screening assays as well as in methods for the purification of PI3K.

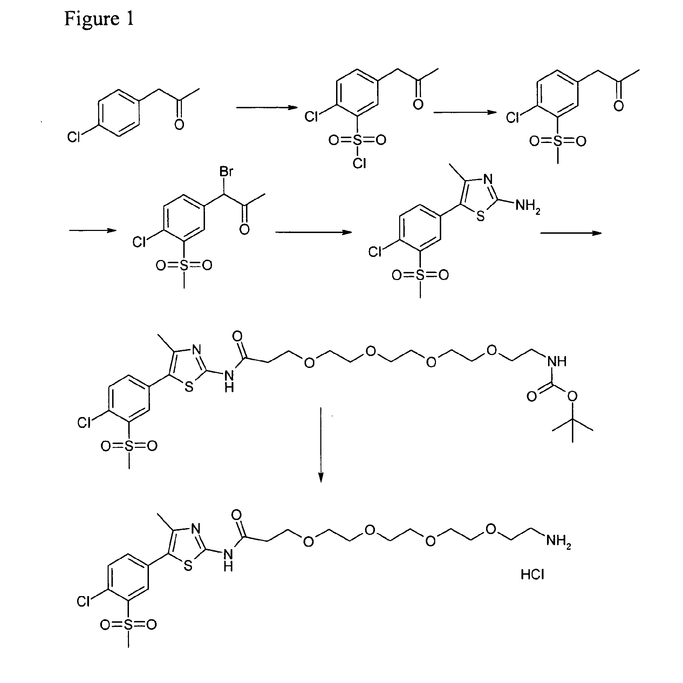

The structure of phenylthiazole ligand 1 is given in Figure 1. This compound is a substituted thiazole (3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide) which according to Figure 1 has hydrochloride as the anion in liquid solution. However, further counter ions are also envisaged in the context of the present invention. The phenylthiazole ligand 1 can be covalently coupled to a suitable solid support material via the primary amino group and be used for the isolation of binding proteins. The synthesis of phenylthiazole ligand 1 is described in Example 1. According to the invention, the expression "phenylthiazole ligand 1" also includes compounds comprising the identical core but which have another linker, preferably coupled to the nitrogen not being part of the cyclic structures, for linkage to the solid support. Typically linkers have backbone of 8, 9 or 10 atoms. The linkers may contain either a carboxy-, hydroxy or amino-active group.

Therefore, in a preferred embodiment, the expression "phenylthiazole ligand 1" also includes compounds having the same N-[5-(4-chloro-3-methanesulfonyl-phenyl)-4-methyl-thiazol-2yl]-propionamide core but comprise another linker at the N-atom, e.g. a C1-C8 alkylcarbonyl or a C1-C8 alkylaminocarbonyl, either of which being optionally substituted by halogen, hydroxy, amino, C1-C8-alkylamino, C1-C8-alkoxycarbonyl, C1-C8-alkoxy optionally substituted by hydroxyl or C1-C8-alkyl optionally substituted by hydroxyl or halogen. Furthermore, this expression also includes compounds as described above which have instead of the 4-chloro residue another halogen, e.g. bromide or which are further substituted at the phenyl ring, e.g. by halogen. Furthermore, instead of the methane sulfonyl group, also another group like a hydroxyl, carboxyl or C1-C8 alkyl group, optionally substituted by halogen, may be present.

In an especially preferred embodiment, compounds falling under the expression "phenylthiazole ligand 1" are selected from the group consisting of 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide hydrochloride, 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide, and compounds with the same N-[5-(4-chloro-3-methanesulfonyl-phenyl)-4-methyl-thiazol-2yl]-propionamide core which are only further substituted at the N- atom by C1-C8 alkylcarbonyl or C1-C8 alkylaminocarbonyl, either of which being optionally substituted by halogen, hydroxy, amino, C1-C8-alkylamino, C1-C8-alkoxycarbonyl, C1-C8-alkoxy optionally substituted by hydroxyl or C1-C8-alkyl optionally substituted by hydroxyl or halogen

According to the present invention "PI3K" comprises all members of the PI3K family comprising class IA (e.g. PI3K alpha, beta and delta), class IB (e.g. PI3K gamma), class II (e.g. PI3KC2 alpha, beta and gamma) and class III (e.g. Vps34 yeast homologue).

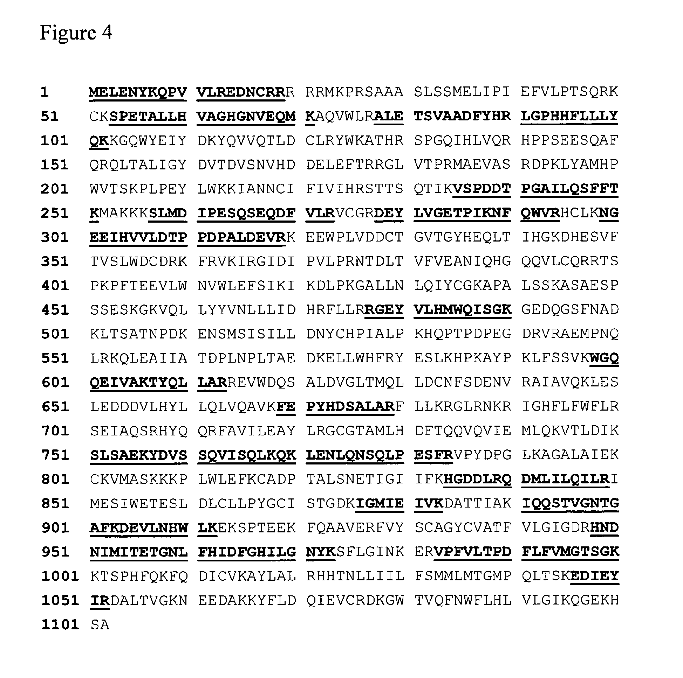

The sequence of human PI3K gamma (the so far only known member of class IB) is given in Figure 4.

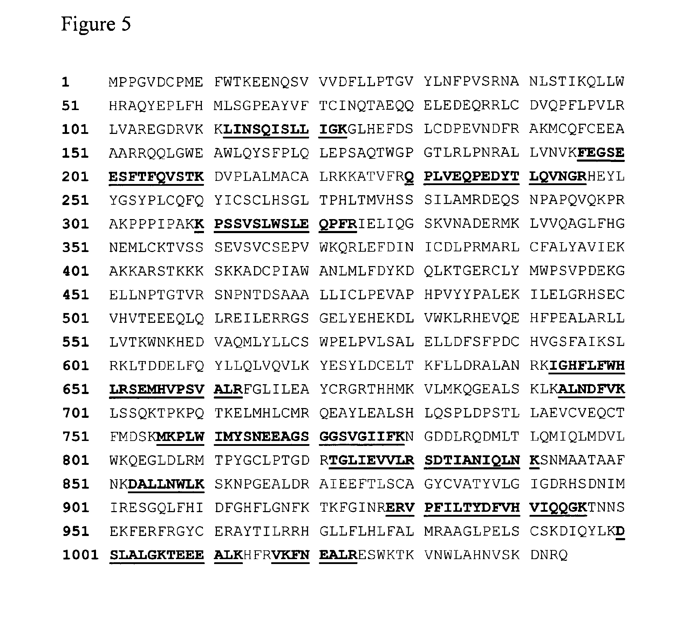

The sequence of human PI3K delta (a member of class IA) is given in Figure 5.

According to the present invention, the expression "PI3K" relates to both human and other proteins of this family. The expression especially includes functionally active derivatives thereof, or functionally active fragments thereof, or a homologues thereof, or variants encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCI (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

According to the present invention, "ATM" means Ataxia Telangiectasia Mutated protein. The ATM protein is a member of the phosphatidylinositol-3 kinase family of proteins that respond to DNA damage by phosphorylating key substrates involved in DNA repair and/or cell cycle control (Shilo, 2003. Nature Reviews Cancer 3, 155-168).

According to the present invention, "ATR" means Ataxia Telangiectasia and RAD3-Related protein (synonym FRAP-related protein 1, FRP1).

According to the present invention, "DNAPK" means DNA-dependent protein kinase. The PRKDC gene encodes the catalytic subunit of a nuclear DNA-dependent serine/threonine protein kinase (DNA-PK). The second component is the autoimmune antigen Ku (152690), which is encoded by the G22P1 gene on chromosome 22q. On its own, the catalytic subunit of DNA-PK is inactive and relies on the G22P 1 component to direct it to the DNA and trigger its kinase activity; PRKDC must be bound to DNA to express its catalytic properties.

According to the present invention, "mTOR" means mammalian target of rapamycin (mTOR, also known as FRAP or RAFT1) (Tsang et al., 2007, Drug Discovery Today 12, 112-124). The mTOR protein is a large kinase of 289 kDA which occurs in all eukaryotic organisms sequenced so far. The sequence of the carboxy-terminal "phosphatidylinositol 3-kinase (PI3K)-related kinase" (PIKK) domain is highly conserved between species and exhibits serine and threonine kinase activity but no detectable lipid kinase activity.

According to the present invention, the expressions "ATM", "ATR", "DNAPK" or "mTOR" relate to both human and other proteins of this family (Shilo, 2003. Nature Reviews Cancer 3, 155-168). The expression especially includes functionally active derivatives thereof, or functionally active fragments thereof, or a homologues thereof, or variants encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

Phenylthiazole ligand I is a ligand for all isoforms of PI3K (see above). However, throughout the invention, it is preferred that PI3K is PI3K gamma or PI3K delta, especially the human isoforms thereof.

In some aspects of the invention, first a protein preparation containing PI3K is provided. The methods of the present invention can be performed with any protein preparation as a starting material, as long as the PI3K is solubilized in the preparation. Examples include a liquid mixture of several proteins, a cell lysate, a partial cell lysate which contains not all proteins present in the original cell or a combination of several cell lysates, in particular in cases where not every target protein of interest is present in every cell lysate. The term "protein preparation" also includes dissolved purified protein.

The presence of PI3K protein species in a protein preparation of interest can be detected on Western blots probed with antibodies that are specifically directed against P13K. In case that PI3K is a specific isoform (e.g. PIK3 gamma and/or PI3K delta), the presence of said isoform can be determined by an isoform-specific antibody. Such antibodies are known in the art (Sasaki et al., 2000, Nature 406, 897-902; Deora et al., 1998, J. Biol. Chem. 273, 29923-29928). Alternatively, also mass spectrometry (MS) could be used (see below).

The presence of ATM, ATR, DNAPK and/or mTOR protein in a protein preparation of interest can be detected on Western blots probed with antibodies that are specific for said protein.

Cell lysates or partial cell lysates can be obtained by isolating cell organelles (e.g. nucleus, mitochondria, ribosomes, golgi etc.) first and then preparing protein preparations derived from these organelles. Methods for the isolation of cell organelles are known in the art (Chapter 4.2 Purification of Organelles from Mammalian Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

In addition, protein preparations can be prepared by fractionation of cell extracts thereby enriching specific types of proteins such as cytoplasmic or membrane proteins (Chapter 4.3 Subcellular Fractionation of Tissue Culture Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

Furthermore protein preparations from body fluids can be used (e.g. blood, cerebrospinal fluid, peritoneal fluid and urine).

For example whole embryo lysates derived from defined development stages or adult stages of model organisms such as C. elegans can be used. In addition, whole organs such as heart dissected from mice can be the source of protein preparations. These organs can also be perfused in vitro in order to obtain a protein preparation.

Furthermore, the protein preparation may be a preparation containing PI3K which has been recombinantely produced. Methods for the production of recombinant proteins in prokaryotic and eukaryotic cells are widely established (Chapter 5 Production of Recombinant Proteins in "Current Protocols in Protein Science", Editors: John. E. Coligan, Ben M. Dunn, Hidde L. Ploegh, David W. Speicher, Paul T. Wingfield; Wiley, 1995, ISBN: 0-471-14098-8).

In a preferred embodiment of the methods of the invention, the provision of a protein preparation includes the steps of harvesting at least one cell containing PI3K and lysing the cell.

Suitable cells for this purpose are e.g. those cells or tissues were members of the PIK3 family are expressed. Members of the PI3K family are expressed in most cells and tissues. PI3K gamma is preferentially expressed in cells of the hematopoietic system (e.g. granulocytes, macrophages, mast cells and platelets) but also in cardiomyocytes, vascular smooth muscle and vascular epithelium cells. PI3K delta is ubiquitously expressed with pronounced expression in lymphocytes, granulocytes and mast cells.

Therefore, in a preferred embodiment, cells isolated from peripheral blood represent a suitable biological material. Procedures for the preparation and culture of human lymphocytes and lymphocyte subpopulations obtained from peripheral blood (PBLs) are widely known (W.E Biddison, Chapter 2.2 "Preparation and culture of human lymphocytes" in Current Protocols in Cell Biology, 1998, John Wiley & Sons, Inc.). For example, density gradient centrifugation is a method for the separation of lymphocytes from other blood cell populations (e.g. erythrocytes and granulocytes). Human lymphocyte subpopulations can be isolated via their specific cell surface receptors which can be recognized by monoclonal antibodies. The physical separation method involves coupling of these antibody reagents to magnetic beads which allow the enrichment of cells that are bound by these antibodies (positive selection). The isolated lymphocyte cells can be further cultured and stimulated by adding antibodies directed against the T-cell receptor or co-receptors such as CD-3 to initiate T-cell recptor signaling and subsequently phosphorylation of PI3K (Houtman et al., 2005, The Journal of Immunology 175(4), 2449-2458).

As an alternative to primary human cells cultured cell lines (e.g. MOLT-4 cells or rat basophilic leukemia (RBL-2H3) cells) can be used. RBL-2H3 cells can be stimulated by cross-linking the high-affinity receptor for IgE (FcepsilonRI) by multivalent antigens to induce activation of PI3K (Kato et al., 2006, J. Immunol. 177(1): 147-154).

In a preferred embodiment, the cell is part of a cell culture system and methods for the harvest of a cell out of a cell culture system are known in the art (literature supra).

The choice of the cell will mainly depend on the expression of PI3K, since it has to be ensured that the protein is principally present in the cell of choice. In order to determine whether a given cell is a suitable starting system for the methods of the invention, methods like Westernblot, PCR-based nucleic acids detection methods, Northernblots and DNA-microarray methods ("DNA chips") might be suitable in order to determine whether a given protein of interest is present in the cell.

The choice of the cell may also be influenced by the purpose of the study. If the in vivo efficacy for a given drug needs to be analyzed then cells or tissues may be selected in which the desired therapeutic effect occurs (e.g. granulocytes or mast cells). By contrast, for the elucidation of protein targets mediating unwanted side effects the cell or tissue may be analysed in which the side effect is observed (e.g. cardiomycytes, vascular smooth muscle or epithelium cells).

Furthermore, it is envisaged within the present invention that the cell containing PI3K may be obtained from an organism, e.g. by biopsy. Corresponding methods are known in the art. For example, a biopsy is a diagnostic procedure used to obtain a small amount of tissue, which can then be examined miscroscopically or with biochemical methods. Biopsies are important to diagnose, classify and stage a disease, but also to evaluate and monitor drug treatment.

It is encompassed within the present invention that by the harvest of the at least one cell, the lysis is performed simultaneously. However, it is equally preferred that the cell is first harvested and then separately lysed.

Methods for the lysis of cells are known in the art (Karwa and Mitra: Sample preparation for the extraction, isolation, and purification of Nuclei Acids; chapter 8 in "Sample Preparation Techniques in Analytical Chemistry", Wiley 2003, Editor: Somenath Mitra, print ISBN: 0471328456; online ISBN: 0471457817). Lysis of different cell types and tissues can be achieved by homogenizers (e.g. Potter-homogenizer), ultrasonic desintegrators, enzymatic lysis, detergents (e.g. NP-40, Triton X-100, CHAPS, SDS), osmotic shock, repeated freezing and thawing, or a combination of these methods.

According to the methods of the invention, the protein preparation containing PI3K is contacted with the phenylthiazole ligand 1 immobilized on a solid support under conditions allowing the formation of a phenylthiazole ligand 1 - PI3K complex.

In the present invention, the term "a phenylthiazole ligand 1 - PI3K complex" denotes a complex where phenylthiazole ligand 1 interacts with PI3K, e.g. by covalent or, most preferred, by non-covalent binding. The same definition applies also for complexes between phenylthiazole ligand 1 and ATM, ATR, DNAPK or mTOR.

The skilled person will know which conditions can be applied in order to enable the formation of the phenylthiazole ligand 1 - PI3K complex.

In the context of the present invention, the term "under conditions allowing the formation of the complex" includes all conditions under which such formation, preferably such binding is possible. This includes the possibility of having the solid support on an immobilized phase and pouring the lysate onto it. In another preferred embodiment, it is also included that the solid support is in a particulate form and mixed with the cell lysate. In the context of non-covalent binding, the binding between phenylthiazole ligand 1 and PI3K is, e.g., via salt bridges, hydrogen bonds, hydrophobic interactions or a combination thereof.

In a preferred embodiment, the steps of the formation of the phenylthiazole ligand 1-PI3K complex are performed under essentially physiological conditions. The physical state of proteins within cells is described in Petty, 1998 (Howard R. Petty, Chapter 1, Unit 1.5 in: Juan S. Bonifacino, Mary Dasso, Joe B. Harford, Jennifer Lippincott-Schwartz, and Kenneth M. Yamada (eds.) Current Protocols in Cell Biology Copyright © 2003 John Wiley & Sons, Inc. All rights reserved. DOI: 10.1002/0471143030.cb0101s00Online Posting Date: May, 2001Print Publication Date: October, 1998).

The contacting under essentially physiological conditions has the advantage that the interactions between the ligand, the cell preparation (i. e. the kinase to be characterized) and optionally the compound reflect as much as possible the natural conditions. "Essentially physiological conditions" are *inter alia* those conditions which are present in the original, unprocessed sample material. They include the physiological protein concentration, pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

For example, "essentially physiological conditions" may comprise 50-200 mM NaCl or KCl, pH 6.5-8.5, 20-37°C, and 0.001-10 mM divalent cation (e.g. Mg++, Ca++,); more preferably about 150 m NaCl or KCl, pH7.2 to 7.6, 5 mM divalent cation and often include 0.01-1.0 percent non-specific protein (e.g. BSA). A non-ionic detergent (Tween, NP-40, Triton-X100) can often be present, usually at about 0.001 to 2%, typically 0.05-0.2% (volume/volume). For general guidance, the following buffered aequous conditions may be applicable: 10-250 mM NaCI, 5-50 mM Tris HCl, pH5-8, with optional addition of divalent cation(s) and/or metal chelators and/or non-ionic detergents.

Preferably, "essentially physiological conditions" mean a pH of from 6.5 to 7.5, preferably from 7.0 to 7.5, and / or a buffer concentration of from 10 to 50 mM, preferably from 25 to 50 mM, and / or a concentration of monovalent salts (e.g. Na or K) of from 120 to 170 mM, preferably 150 mM. Divalent salts (e.g. Mg or Ca) may further be present at a concentration of from 1 to 5 mM, preferably 1 to 2 mM, wherein more preferably the buffer is selected from the group consisting of Tris-HCl or HEPES.

In the context of the present invention, phenylthiazole ligand 1 is immobilized on a solid support. Throughout the invention, the term "solid support" relates to every undissolved support being able to immobilize a small molecule ligand on its surface.

According to a further preferred embodiment, the solid support is selected from the group consisting of agarose, modified agarose, sepharose beads (e.g. NHS-activated sepharose), latex, cellulose, and ferro- or ferrimagnetic particles.

Phenylthiazole ligand 1 may be coupled to the solid support either covalently or non-covalently. Non-covalent binding includes binding via biotin affinity ligands binding to steptavidin matrices.

Preferably, the phenylthiazole ligand 1 is covalently coupled to the solid support.

Before the coupling, the matrixes can contain active groups such as NHS, Carbodimide etc. to enable the coupling reaction with the phenylthiazole ligand 1. The phenylthiazole ligand 1 can be coupled to the solid support by direct coupling (e.g. using functional groups such as amino-, sulfhydryl-, carboxyl-, hydroxyl-, aldehyde-, and ketone groups) and by indirect coupling (e.g. via biotin, biotin being covalently attached to phenylthiazole ligand 1 and non-covalent binding of biotin to streptavidin which is bound to solid support directly).

The linkage to the solid support material may involve cleavable and non-cleavable linkers. The cleavage may be achieved by enzymatic cleavage or treatment with suitable chemical methods.

Preferred binding interfaces for binding phenylthiazole ligand 1 to solid support material are linkers with a C-atom backbone. Typically linkers have backbone of 8, 9 or 10 atoms. The linkers contain either a carboxy- or amino-active group.

The skilled person will appreciate that between the individual steps of the methods of the invention, washing steps may be necessary. Such washing is part of the knowledge of the person skilled in the art. The washing serves to remove non-bound components of the cell lysate from the solid support. Nonspecific (e.g. simple ionic) binding interactions can be minimized by adding low levels of detergent or by moderate adjustments to salt concentrations in the wash buffer.

According to the identification methods of the invention, the read-out system is either the detection or determination of PI3K (first aspect of the invention), the detection of the phenylthiazole ligand 1 - PI3K complex (second aspect of the invention), or the determination of the amount of the phenylthiazole ligand 1 - PI3K complex (second, third and forth aspect of the invention).

Throughout the invention, the same read-out systems used for the determination or detection of PI3K, the detection of the phenylthiazole ligand 1 - PI3K complex or the determination of the amount of the phenylthiazole ligand 1 - PI3K complex can be used for the detection of ATM, ATR, DNAPK or mTOR or the detection or the determination of the amount of a complex between phenylthiazole ligand 1 and said proteins. This implies that in cases where an agent specific for PI3K (e.g. an antibody) is used, an agent specific for ATM, ATR, DNAPK, or mTOR has to be used instead. Consequently, the embodiments and explanations given below also apply to the the detection of ATM, ATR, DNAPK or mTOR or to the detection of the complex or to the determination of the amount of a complex between phenylthiazole ligand 1 and said proteins.

In the method according to the first aspect of the invention, the detection or determination of separated PI3K is preferably indicative for the fact that the compound is able to separate PI3K from the immobilized phenylthiazole ligand 1. This capacity indicates that the respective compound interacts, preferably binds to P13K, which is indicative for its therapeutic potential.

In one embodiment of the method according to the second aspect of the invention, the phenylthiazole ligand 1 -PI3K complex formed during the method of the invention is detected. The fact that such complex is formed preferably indicates that the compound does not completely inhibit the formation of the complex. On the other hand, if no complex is formed, the compound is presumably a strong interactor with PI3K, which is indicative for its therapeutic potential.

According to the methods of the second, third and forth aspect of the invention the amount of the phenylthiazole ligand I -PI3K complex formed during the method is determined. In general, the less complex in the presence of the respective compound is formed, the stronger the respective compound interacts with PI3K, which is indicative for its therapeutic potential.

The detection of the phenylthiazole ligand 1 - PI3K complex according to the second aspect of the invention can be performed by using labeled antibodies directed against PI3K and a suitable readout system.

According to a preferred embodiment of the second aspect of the invention, the phenylthiazole ligand 1 - PI3K complex complex is detected by determining its amount.

In the course of the second, third and forth aspect of the invention, it is preferred that PI3K is separated from the immobilized phenylthiazole ligand 1 in order to determine the amount of the phenylthiazole ligand 1 - PI3K complex.

According to invention, separating means every action which destroys the interactions between phenylthiazole ligand I and PI3K. This includes in a preferred embodiment the elution of PI3K from the immobilized phenylthiazole ligand 1.

The elution can be achieved by using non-specific reagents as described in detail below (ionic strength, pH value, detergents). In addition, it can be tested whether a compound of interest can specifically elute the PI3K from phenylthiazole ligand 1. Such PI3K interacting compounds are described further in the following sections.

Such non-specific methods for destroying the interaction are principally known in the art and depend on the nature of the ligand enzyme interaction. Principally, change of ionic strength, the pH value, the temperature or incubation with detergents are suitable methods to dissociate the target enzymes from the immobilized ligand. The application of an elution buffer can dissociate binding partners by extremes of pH value (high or low pH; e.g. lowering pH by using 0.1 M citrate, pH2-3), change of ionic strength (e.g. high salt concentration using NaI, KI, MgC12, or KCl), polarity reducing agents which disrupt hydrophobic interactions (e.g. dioxane or ethylene glycol), or denaturing agents (chaotropic salts or detergents such as Sodium-docedyl-sulfate, SDS; Review: Subramanian A., 2002, Immunoaffinty chromatography).

In some cases, the solid support has preferably to be separated from the released material. The individual methods for this depend on the nature of the solid support and are known in the art. If the support material is contained within a column the released material can be collected as column flowthrough. In case the support material is mixed with the lysate components (so called batch procedure) an additional separation step such as gentle centrifugation may be necessary and the released material is collected as supernatant. Alternatively magnetic beads can be used as solid support so that the beads can be eliminated from the sample by using a magnetic device.

In step d) of the method according to the first aspect of the invention, it is determined if PI3K has been separated from the immobilized phenylthiazole ligand 1. This may include the detection of PI3K or the determination of the amount PI3K.

Consequently, at least in preferred embodiments of all identification methods of the invention, methods for the detection of separated PI3K or for the determination of its amount are used. Such methods are known in the art and include physico-chemical methods such as protein sequencing (e.g. Edmann degradation), analysis by mass spectrometry methods or immunodetection methods employing antibodies directed against PI3K.

Throughout the invention, if an antibody is used in order to detect PI3K or in order to determine its amount (e.g. via ELISA), the skilled person will understand that, if a specific isoform of PI3K is to be detected or if the amount of a specific isoform of PI3K is to be determined, an isoform-specific antibody may be used. As indicated above, such antibodies are known in the art. Furthermore, the skilled person is aware of methods for producing the same.

Preferably, PI3K, ATM, ATR, DNAPK and/or mTOR are detected or the amount of said proteins is determined by mass spectrometry or immunodetection methods. In the following, this will be explained in more detail by reference to PI3K, but the embodiments and explanation described below also apply to ATM, ATR, DNAPK or mTOR.

The identification of proteins with mass spectrometric analysis (mass spectrometry) is known in the art (Shevchenko et al., 1996, Analytical Chemistry 68: 850-858; Mann et al., 2001, Analysis of proteins and proteomes by mass spectrometry, Annual Review of Biochemistry 70, 437-473) and is further illustrated in the example section.

Preferably, the mass spectrometry analysis is performed in a quantitative manner, for example by using iTRAQ technology (isobaric tags for relative and absolute quatification) or cICAT (cleavable isotope-coded affinity tags) (Wu et al., 2006. J. Proteome Res. 5, 651-658).

According to a further preferred embodiment of the present invention, the characterization by mass spectrometry (MS) is performed by the identification of proteotypic peptides of PI3K. The idea is that PI3K is digested with proteases and the resulting peptides are determined by MS. As a result, peptide frequencies for peptides from the same source protein differ by a great degree, the most frequently observed peptides that "typically" contribute to the identification of this protein being termed "proteotypic peptide". Therefore, a proteotypic peptide as used in the present invention is an experimentally well observable peptide that uniquely identifies a specific protein or protein isoform.

According to a preferred embodiment, the characterization is performed by comparing the proteotypic peptides obtained in the course of practicing the methods of the invention with known proteotypic peptides. Since, when using fragments prepared by protease digestion for the identification of a protein in MS, usually the same proteotypic peptides are observed for a given enzyme, it is possible to compare the proteotypic peptides obtained for a given sample with the proteotypic peptides already known for enzymes of a given class of enzymes and thereby identifying the enzyme being present in the sample. As an alternative to mass spectrometry analysis, the eluted PI3K (including coeluted binding partners or scaffold proteins), can be detected or its amount can be determined by using a specific antibody directed against PI3K (or against an isoform of PI3K, see above).

Furthermore, in another preferred embodiment, once the identity of the coeluted binding partner has been established by mass spectrometry analysis, each binding partner can be detected with specific antibodies directed against this protein.

Suitable antibody-based assays include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

These assays can not only be configured in a way to detect and quantify a PI3K interacting protein of interest (e.g. a catalytic or regulatory subunit of a PI3K complex), but also to analyse posttranslational modification patterns such as phosphorylation or ubiquitin modification.

Furthermore, the identification methods of the invention involve the use of compounds which are tested for their ability to be an PI3K interacting compound.

Principally, according to the present invention, such a compound can be every molecule which is able to interact with PI3K, eg. by inhibiting its binding to phenylthiazole ligand 1. Preferably, the compound has an effect on PI3K, e.g. a stimulatory or inhibitory effect.

Preferably, said compound is selected from the group consisting of synthetic or naturally occurring chemical compounds or organic synthetic drugs, more preferably small molecules, organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 1000 Da, more preferred less than 750 Da, most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, Manger M, Scheck M, Waldmann H. Natural product guided compound library development. Curr Med Chem. 2002 Dec;9(23):2129-45, wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfill the specific requirements of the individual binding pocket within a domain family it may be necessary to optimise the natural product structure by chemical variation. Solid-phase chemistry is said to become an efficient tool for this optimisation process, and recent advances in this field are highlighted in this review article. Other related references include Edwards PJ, Morrell AI. Solid-phase compound library synthesis in drug design and development. Curr Opin Drug Discov Devel. 2002 Jul;5(4):594-605.; Merlot C, Domine D, Church DJ. Fragment analysis in small molecule discovery. Curr Opin Drug Discov Devel. 2002 May;5(3):391-9. Review; Goodnow RA Jr. Current practices in generation of small molecule new leads. J Cell Biochem Suppl. 2001;Suppl 37:13-21; which describes that the current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

In a preferred embodiment of the second and third aspect of the invention, the PI3K containing protein preparation is first incubated with the compound and then with the immobilized phenylthiazole ligand 1. However, the simultaneous incubation of the compound and the immobilized phenylthiazole ligand 1 (coincubation) with the PI3K containing protein preparation is equally preferred (competitive binding assay).

In case that the incubation with the compound is first, the PI3K is preferably first incubated with the compound for 10 to 60 minutes, more preferred 30 to 45 minutes at a temperature of 4°C to 37°C, more preferred 4°C to 25°C, most preferred 4°C. Preferably compounds are used at concentrations ranging from 1 µM to 1 mM, preferably from 10 to 100 µM. The second step, contacting with the immobilized ligand, is preferably performed for 10 to 60 minutes at 4°C.

In case of simultaneous incubation, the PI3K is preferably simultaneously incubated with the compound and phenylthiazole ligand 1 for 30 to 120 minutes, more preferred 60 to 120 minutes at a temperature of 4°C to 37°C, more preferred 4°C to 25°C, most preferred 4°C. Preferably compounds are used at concentrations ranging from 1 µM to 1 mM, preferably from 10 to 100 µM.

Furthermore, steps a) to c) of the second aspect of the invention may be performed with several protein preparations in order to test different compounds. This embodiment is especially interesting in the context of medium or high troughput screenings (see below).

In a preferred embodiment of the method of the invention according to the third or forth aspect, the amount of the phenylthiazole ligand 1 - PI3K complex formed in step c) is compared to the amount formed in step b)

In a preferred embodiment of the method of the invention according to the third or forth aspect, a reduced amount of the phenylthiazole ligand 1 - PI3K complex formed in step c) in comparison to step b) indicates that PI3K is a target of the compound. This results from the fact that in step c) of this method of the invention, the compound competes with the ligand for the binding of PI3K. If less PI3K is present in the aliquot incubated with the compound, this means preferably that the compound has competed with the inhibitor for the interaction with the enzyme and is, therefore, a direct target of the protein and vice versa.

Preferably, the identification methods of the invention are performed as a medium or high throughput screening.

The interaction compound identified according to the present invention may be further characterized by determining whether it has an effect on PI3K, for example on its kinase activity (Carpenter et al., 1990, J. Biol. Chem. 265, 19704-19711). Such assays are known in the art, also in a format that allows medium to high throughput screening (Fuchikami et al., 2002, J. Biomol. Screening 7, 441-450).

In addition, the interaction compound identified according to the present invention may be further characterized by determining whether it has an effect on ATM, ATR, DNAPK or mTOR for example on their kinase activities (Knight et al., 2004. Bioorganic and Medicinal Chemistry 12, 4749-4759; Knight et al., 2006, Cell 125, 733-747).

Briefly, PI3K lipid kinase activity can be measured using solution-based assays with phopholipid vesicles. The reaction is terminated by the addition of acidified organic solvents and subsequent phase separation by extraction or thin layer chromatography analysis (Carpenter et al., 1990, J. Biol. Chem. 265, 19704-19711).

Alternatively, a fluorescence polarization assay format can be used. Briefly, PI3K is incubated with a suitable phosphoinositol substrate. After the reaction is complete the reaction products are mixed with a specfic phosphoinositol detector protein and a fluorescent phosphoinositol probe. The polarization (mP) values decrease as probe binding to the phosphoinositol detector protein is displaced by the reaction product. The degree of polarization of the fluorescent probe is inversely proportional to the amount of the product of the PI3K reaction (Drees et al., 2003, Comb. Chem. High Throughput Screening 6, 321-330).

For the determination of PI3K protein kinase activity a fluorescence polarization assay with a suitable peptide substratecan be used. Briefly, a fluorescein-labeled peptide substrate may be incubated with PI3K (e.g. PI3K delta), ATP and an anti-phosphoserine antibody. As the reaction proceeds, the phosphorylated peptide binds to the anti-phosphoserine antibody, resulting in an increase in the polarization signal. Compounds that inhibit the kinase result in a low polarization signal.

The compounds identified according to the present invention may further be optimized (lead optimisation). This subsequent optimisation of such compounds is often accelerated because of the structure-activity relationship (SAR) information encoded in these lead generation libraries. Lead optimisation is often facilitated due to the ready applicability of high-throughput chemistry (HTC) methods for follow-up synthesis.

One example of such a library and lead optimization is described for PI3K gamma (Pomel et al., 2006, J. Med. Chem. 49, 3857-3871).

The methods of the invention comprise a method step wherein it is determined whether the compound is able to separate also ATM, ATR, DNAPK and/or mTOR from the immobilized phenylthiazole ligand 1 (first aspect of the invention) or whether also a complex between phenylthiazole ligand 1 and ATM, ATR, DNAPK and/or mTOR has been formed. As indicated above, these steps can essentially be performed as described above for PI3K, where agents, e.g. antibodies specific for the given kinase are used when required.

The rational behind these method steps is that it is possible to determine the specificity of the identified PI3K interacting compound. It is preferred, in the context of the present invention, to identify PI3K interacting compounds which are specific for PI3K, i.e. which bind to a lesser extend to ATM, ATR, DNAPK and/or mTOR, or, even more preferred, do not bind to one of or all of these proteins.

The invention further relates to a method for the preparation of a pharmaceutical composition comprising the steps of
a) identifying a PI3K interacting compound as described above, and
b) formulating the interacting compound to a pharmaceutical composition.

Therefore, the invention provides a method for the preparation of pharmaceutical compositions, which may be administered to a subject in an effective amount. In a preferred aspect, the therapeutic is substantially purified. The subject to be treated is preferably an animal including, but not limited to animals such as cows, pigs, horses, chickens, cats, dogs, etc., and is preferably a mammal, and most preferably human. In a specific embodiment, a non-human mammal is the subject.

The compounds identified according to the invention are useful for the prevention or treatment of diseases where PI3K plays a role such as cancer (e.g. breast, colon or ovary cancer), metabolic disorders (e.g. diabetes or obesity) or autoimmune/inflammatory disorders (e.g. rheumatic arthritis, psoriasis, Crohn's disease, ulcerative colitis, asthma or allergic reactions).

Consequently, the present invention also relates to the use of a compound identified by the methods of the invention for the preparation of a medicament for the treatment of one or more of the above mentioned diseases. Furthermore, the present invention relates to a pharmaceutical composition comprising said compound.

In general, the pharmaceutical compositions of the present invention comprise a therapeutically effective amount of a therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc..

The amount of the therapeutic of the invention which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems. In general, suppositories may contain active ingredient in the range of 0.5% to 10% by weight; oral formulations preferably contain 10% to 95% active ingredient. Various delivery systems are known and can be used to administer a therapeutic of the invention, e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the therapeutic can be delivered in a vesicle, in particular a liposome (Langer, 1990, Science 249:1527-1533).

In yet another embodiment, the therapeutic can be delivered via a controlled release system. In one embodiment, a pump may be used (Langer, supra). In yet another embodiment, a controlled release system can be placed in proximity of the therapeutic target, i.e., the brain, thus requiring only a fraction of the systemic dose

In the context of the present invention, it has been found that phenylthiazole ligand 1 is a ligand for ATM, ATR, DNAPK, and mTOR. Therefore, the present invention also relates to methods for the identification of compounds interacting with ATM, ATR, DNAPK and/or mTOR. These methods are performed as described above in the context of the identification of PI3K interacting coumpounds. Furthermore, it is envisaged within the present invention that these methods for the identification of ATM, ATR, DNAPK or mTOR-interacting compounds may or may not contain the step of determining whether a given compound may be able to interact also with other PIKK kinases as defined in the present invention

The invention further relates to a method for the purification of ATM, ATR, DNAPK and/or mTOR, comprising the steps of
a) providing a protein preparation containing one or more of said proteins,
b) contacting the protein preparation with phenylthiazole ligand 1 immobilized on a solid support under conditions allowing the formation of an phenylthiazole ligand 1 - protein complex, and
c) separating the protein from the immobilized phenylthiazole ligand 1.

As mentioned above, it has been surprisingly found that phenylthiazole ligand 1 is a ligand which recognizes these proteins. This enables efficient purification methods for these proteins.

The embodiments as defined above for the identification methods of the invention also apply to the purification method of the invention.

Preferably, said purification is performed using an isoform specific antibody as explained above.

In a preferred embodiment, the purification method of the invention further comprises after step c) the identification of proteins being capable of binding to ATM, ATR, DNAPK and/or mTOR. This is especially interesting when the formation of the complex is performed under essentially physiological conditions, because it is then possible to preserve the natural condition of the enzyme which includes the existence of binding partners, enzyme subunits or post-translational modifications, which can then be identified with the help of mass spectrometry (MS).

Consequently, in a preferred embodiment, the purification method of the invention further comprises after step c) the determination whether the given protein is further posttranslationally modified, e.g. by ubiquitin modification.

The invention further relates to the use of phenylthiazole ligand 1 for the identification of ATM, ATR, DNAPK and/or mTOR interacting compounds and for the purification of PI3K. The embodiments as defined above also apply to the uses of the invention.

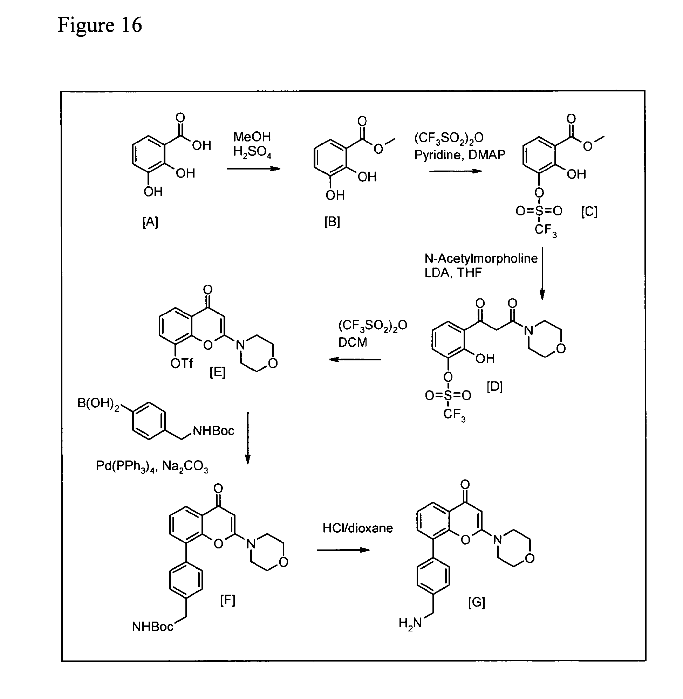

In a preferred embodiment of the present invention, not only phenlythiazole ligand 1, but in addition also another ligand, namely the phenylmorpholin-chromen ligand (8-(4-aminomethyl-phenyl)-2-morpholin-4-yl-chromen-4-one) as shown in Fig. 16, or derivatives thereof, e.g. compounds comprising the identical core but which have another linker, preferably coupled to the nitrogen not being part of the cyclic structures, for linkage to the solid support, may be used for the identification of the interacting compounds, Consequently, in these embodiments of the invention, both ligands are immobilized. In this context, it is included that, in case that beads are used, both ligands are immobilized on the same bead or that one ligand is immobilized on one bead and the other ligand is immobilized on the other bead. In this context, typically linkers have backbone of 8, 9 or 10 atoms. The linkers may contain either a carboxy-, hydroxy or amino-active group.

The invention is further illustrated by the following figures and examples, which are not considered as being limiting for the scope of protection conferred by the claims of the present application.

### Short description of the figures

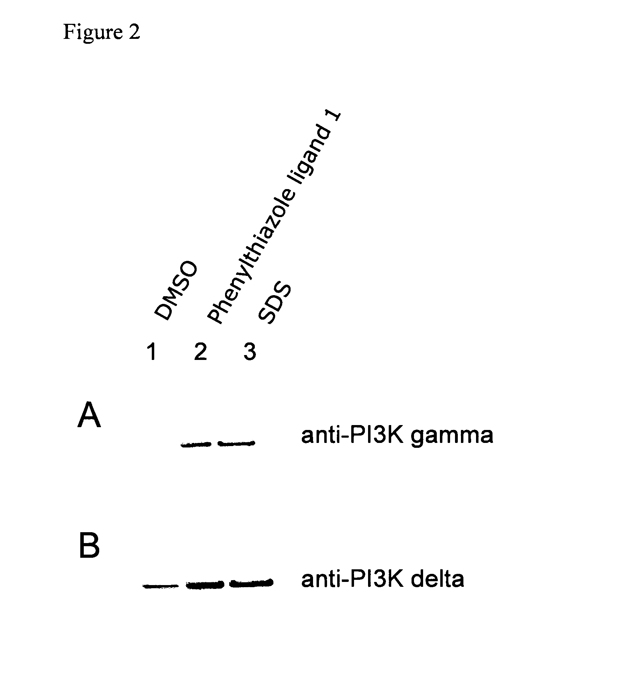

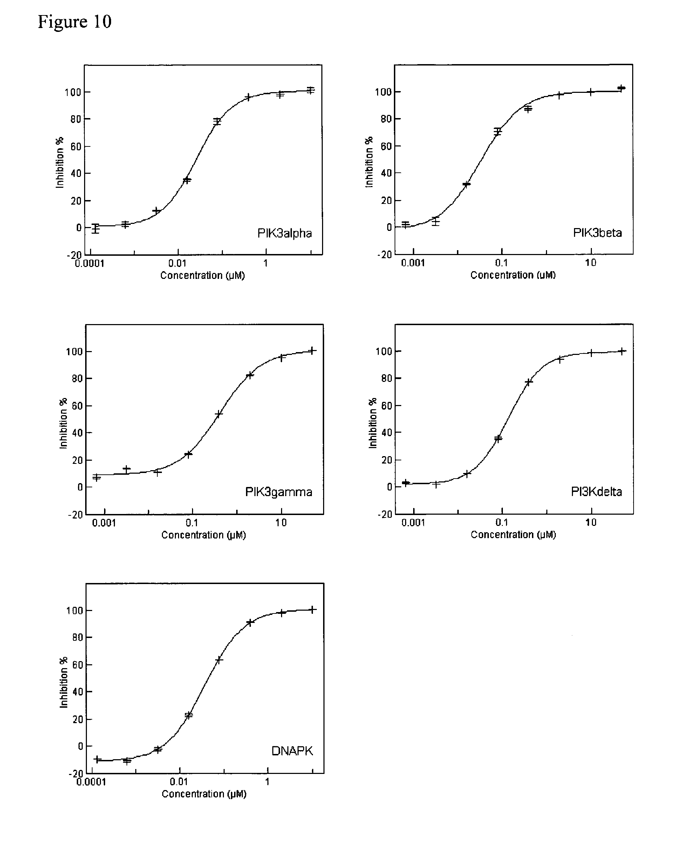

**Figure 1**: Synthesis and structure of phenylthiazole ligand 1.
   The phenylthiazole ligand 1 was synthesized as described in example 1.
**Figure 2**: Drug pulldown experiment with immobilized phenylthiazole ligand 1 and Western blot detection of PI3K proteins.
   As biological material a cell lysate prepared from MOLT-4 cells was used. The drug pull down experiment was performed as described in Example 2 with lysate samples containing 50 mg of protein. Captured proteins were eluted with DMSO containing buffer (lane 1), 100 µM of free phenylthiazole ligand 1 or SDS sample buffer (lane 3). The eluted samples were separated on SDS-polyacrylamide gels and transferred to membranes. The blots were first incubated with specific antibodies directed against PI3K gamma (Figure 2A) and PI3K delta (Figure 2B). Secondary detection antibodies labeled with fluorescent dyes for detection were used with the Odyssey infrared imaging system. Lane 1: DMSO elution control; lane 2: elution with 100 µM free phenylthiazole ligand 1; lane 3: SDS elution.

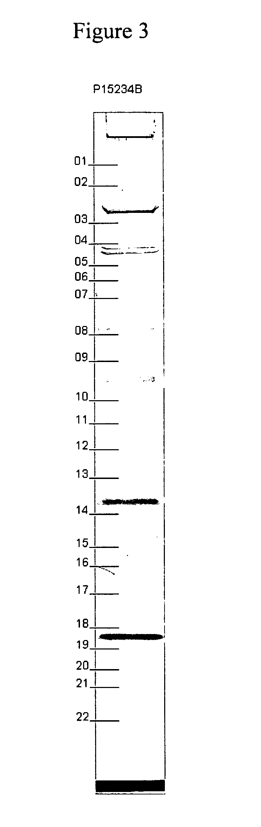

**Figure 3**: Drug pulldown experiment with immobilized phenylthiazole ligand 1 for mass spectrometry analysis of proteins.
   A protein gel after staining with Coomassie blue is shown. The indicated gel areas were cut out as gel slices and proteins were subjected to analysis by mass spectrometry.
   The drug pulldown experiment was performed as described in Example 2 with a MOLT-4 cell lysate sample containing 50 mg of protein. Proteins bound to immobilized phenylthiazole ligand 1 were eluted with SDS sample buffer and separated by SDS-polyacrylamide gel electrophoresis (SDS-PAGE).
**Figure 4**: Peptides identified of PI3K gamma.
   The peptides that were identified by mass spectrometry analysis of the human PI3K delta sequence are shown in bold type and underlined.
**Figure 5**: Peptides identified of PI3K delta.
   The peptides that were identified by mass spectrometry analysis of the human PI3K gamma sequence are shown in bold type and underlined.

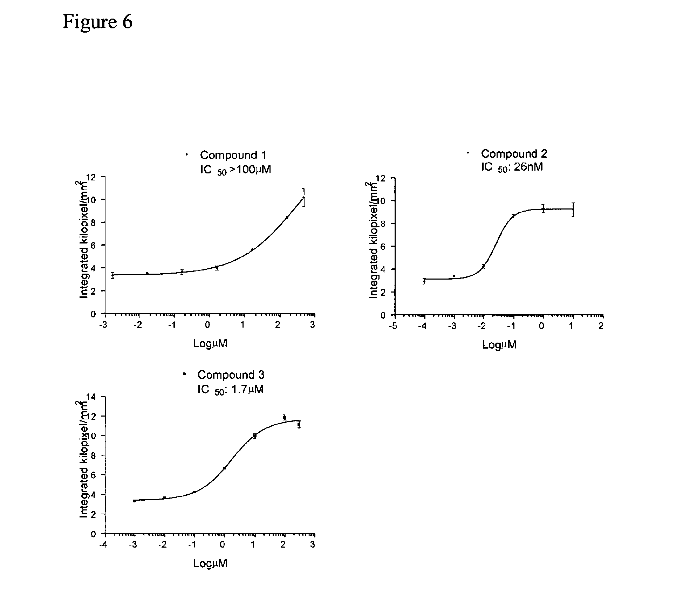

**Figure 6**: Elution assay for the identification of PI3K gamma interacting compounds.
   The experiment was performed as described in example 3. PI3K gamma protein was captured by immobilized phenylthiazole ligand 1 from MOLT-4 cell lysate and eluted by the compounds as indicated. Eluates were transferred to a nitrocellulose membrane and PI3K gamma was detected with the Odyssey Infrared Imaging system. First antibody: anti-PI3K gamma (Jena Bioscience ABD-026S; mouse antibody). Second antibody: anti-mouse IRDye800 (Rockland, 610-732-124). Integrated Intensity (integrated kilopixel/mm²) are shown.
   Compounds used for elution:
   Compound 1 (LY294002); IC₅₀ > 100 µM; compound 2 (AS-605240): IC₅₀=26 nM; compound 3 (AS-604850); IC₅₀=1.7 µM,

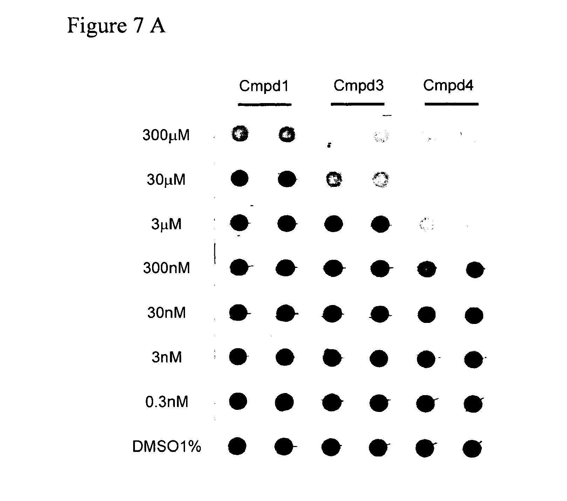

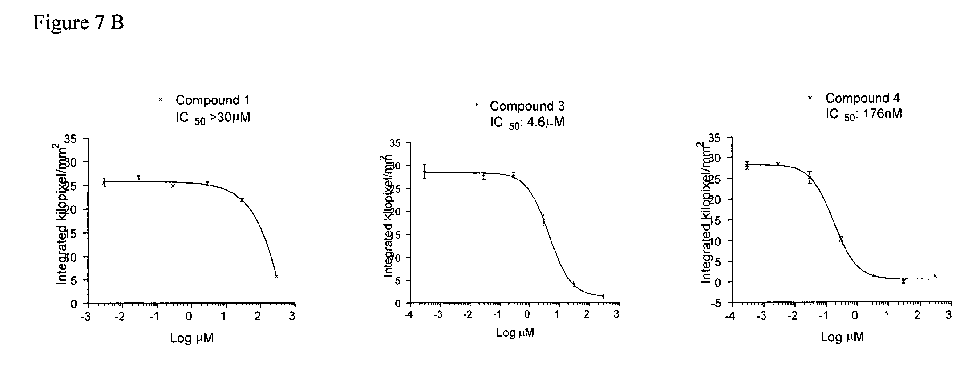

   **Figure 7**: Competitive binding assay for the identification of PI3K gamma interacting compounds.
   The experiment was performed as described in example 4. Test compounds at the indicated concentrations and the affinity matrix were added to MOLT-4 cell lysate and the PI3K gamma protein not interacting with test compounds was captured by the immobilized phenylthiazole ligand 1 on the affinity matrix. The affinity matrix was separated from the lysate, bound proteins were eluted with SDS sample buffer and the eluates were transferred to a nitrocellulose membrane. The amount of PI3K gamma was determined with the Odyssey Infrared Imaging system.
   7A: Dot blot probed with antibodies and signals detected with Odyssey infrared imaging system. First antibody: anti-PI3K gamma (Jena Bioscience ABD-026S; mouse antibody). Second antibody: anti-mouse IRDye800 (Rockland, 610-732-124).
   7B: Competion binding curves. Relative Odyssey units (Integrated Intensity; integrated kilopixel/mm²) are plotted against compound concentrations and half maximal binding competition (IC) values calculated. Compound 1 (LY294002): IC₅₀ > 30 µM; compound 2 (AS-605240): IC₅₀ = 4.6 µM; compound 3 (AS-604850): IC₅₀ = 176 nM.

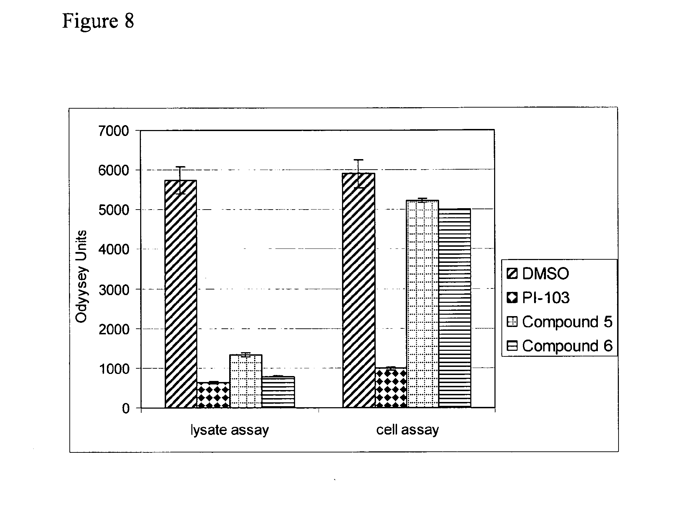

**Figure 8**: Compound profiling by adding compounds to cell lysates (lysate assay) or by incubating compound with living RAW264.7 cells (cell assay).
   The experiment was performed as described in example 5. Compounds were used at a concentration of 10 µM in both assays and the amount of PI3Kdelta was quantified with the Odyssey Infrared Imaging system.

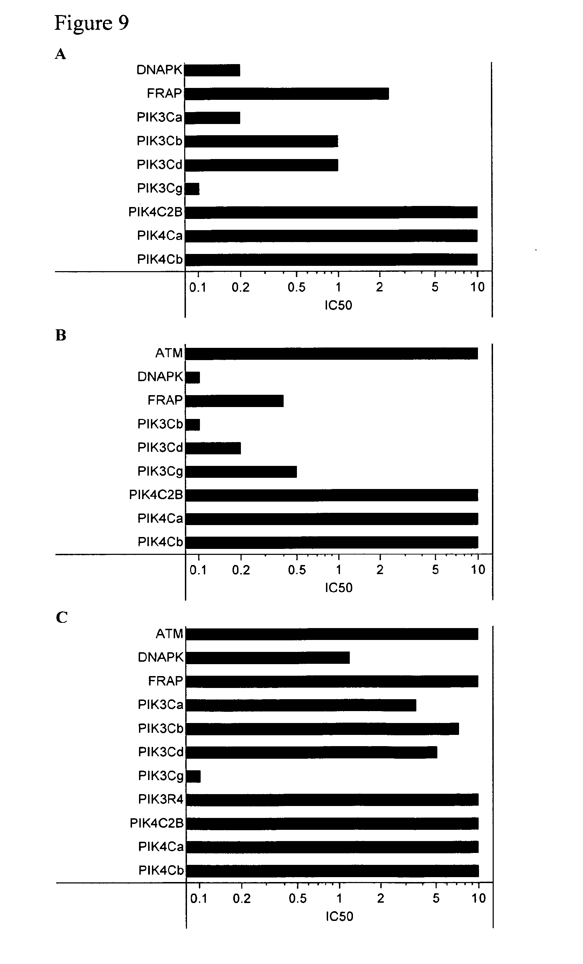

**Figure 9**: Selectivity profiling of PI3K inhibitors using mass spectrometry quantification. The experiment was performed as a Kinobeads competition binding assay in Ramos cell lysates as described in Example 6. Based on quantitative mass spectrometry profiling the IC₅₀ values (µM) are shown for individual targets.
   A: Compound CZC00015097
   B: Compound CZC00018052
   C: Compound CZC00019091
**Figure 10**: Dose response curves of compound CZC18052.
   The compound was tested in the competition binding assay with multiplexed immunodetection of kinases as described in Example 7. In a single assay, the binding affinity of the compound was measured for PI3Kalpha, PI3Kbeta, PI3Kgamma, PI3Kdelta and DNAPK. Briefly, a 1:1 mixture of Molt-4 and Jurkat cell lysates was incubated with the affinity matrix (1:1 mixture of beads with immobilized phenylthiazole ligand 1 and beads with the phenylmorpholin-chromen ligand) and compound CZC18052. The beads were washed and the bound kinases were eluted. Aliquots of the eluate were spotted on five different nitrocellulose membranes, each of which was incubated with the respective target antibody and subsequently a fluorescent secondary antibody. The fluorescent signal was quantified using an infrared scanner. The compound showed potent binding for a range of kinases:
   PI3Kalpha (IC₅₀ = 0.027 µM), PI3Kbeta (IC₅₀ = 0.034 µM), PI3Kgamma (IC₅₀ = 0.43 µM), PI3Kdelta (IC₅₀ = 0.14 µM) and DNAPK (IC₅₀ = 0.038 µM).

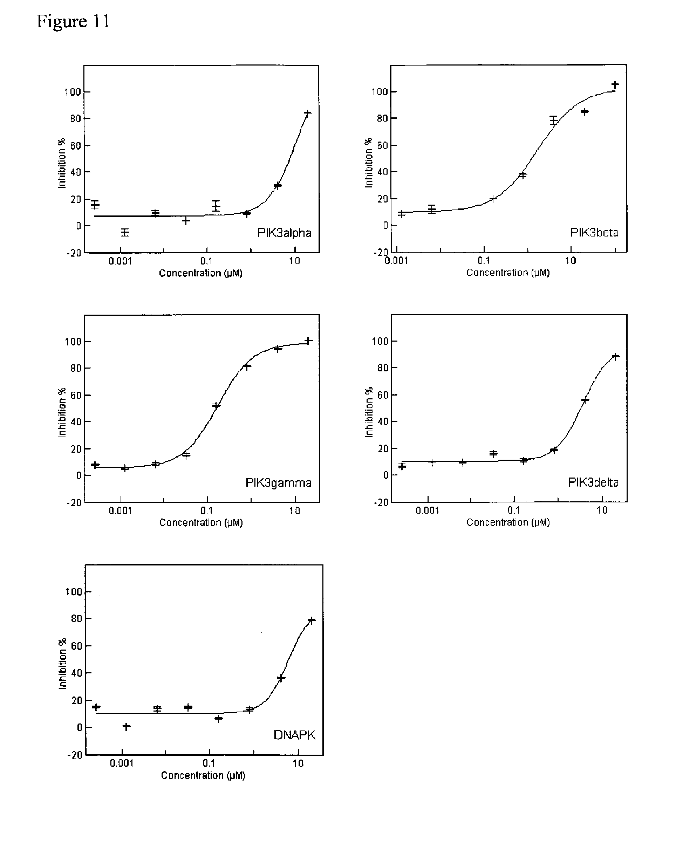

**Figure 11**: Dose response curves for compound CZC19950.
   The experiment was carried out as described in Example 7. The compound showed binding to the following kinases: PI3Kalpha (IC₅₀ > 7 µM), PI3Kbeta (IC₅₀ = 1.7 µM), PI3Kgamma (IC₅₀ = 0.17 µM), PI3Kdelta (IC₅₀ > 3 µM) and DNAPK (IC₅₀ > 6 µM). Compound CZC19950 showed potent binding only to PI3Kgamma (IC₅₀ = 0.17 µM).

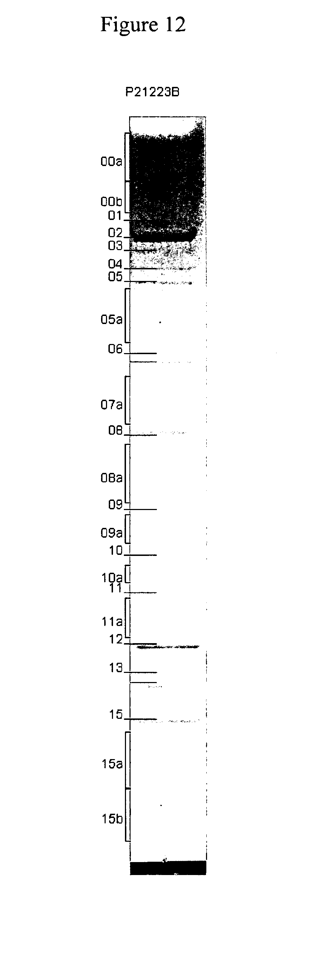

**Figure 12**: Drug pulldown experiment with immobilized phenylthiazole ligand 1 for mass spectrometry analysis of proteins.
   A protein gel after staining with Coomassie blue is shown. The indicated gel areas were cut out as gel slices and proteins were subjected to analysis by mass spectrometry. The drug pulldown experiment was performed as described in Example 2 with a 1:1 mixture of Jurkat and Ramos cell lysate sample containing 50 mg of protein. Proteins bound to immobilized phenylthiazole ligand 1 were eluted with SDS sample buffer and separated by SDS-polyacrylamide gel electrophoresis (SDS-PAGE). The following proteins were identified in this experiment: DNA-PK, ATM, and mTOR.

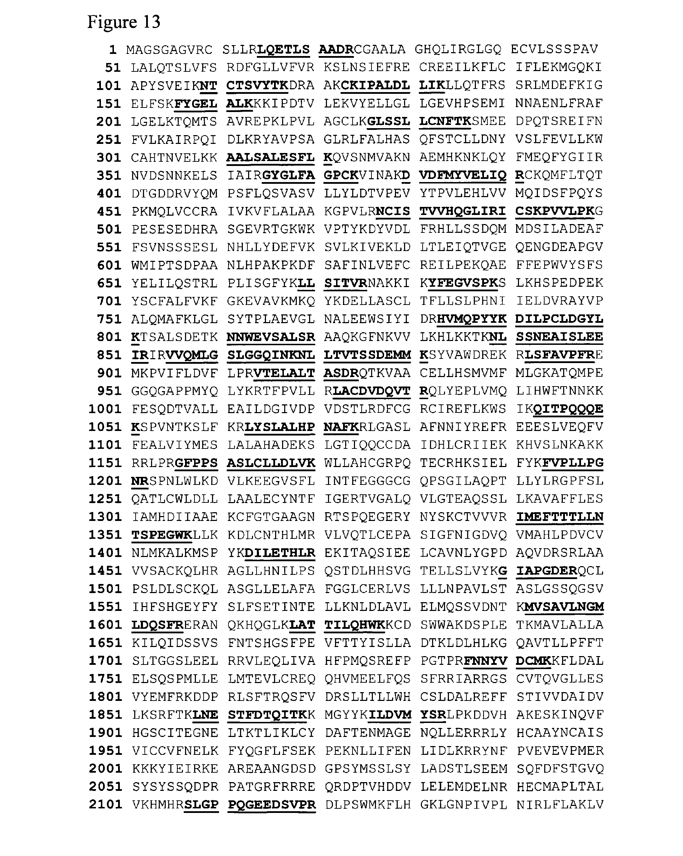

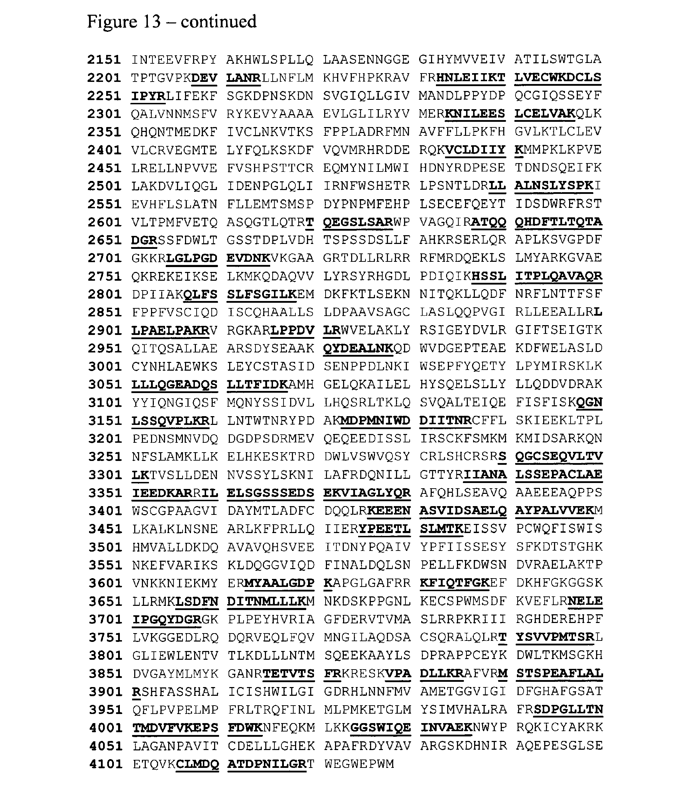

**Figure 13**: Peptides identified by mass spectrometry of DNA-PK after a drug pulldown with immobilized phenylthiazole ligand 1. The identified peptides are underlined.

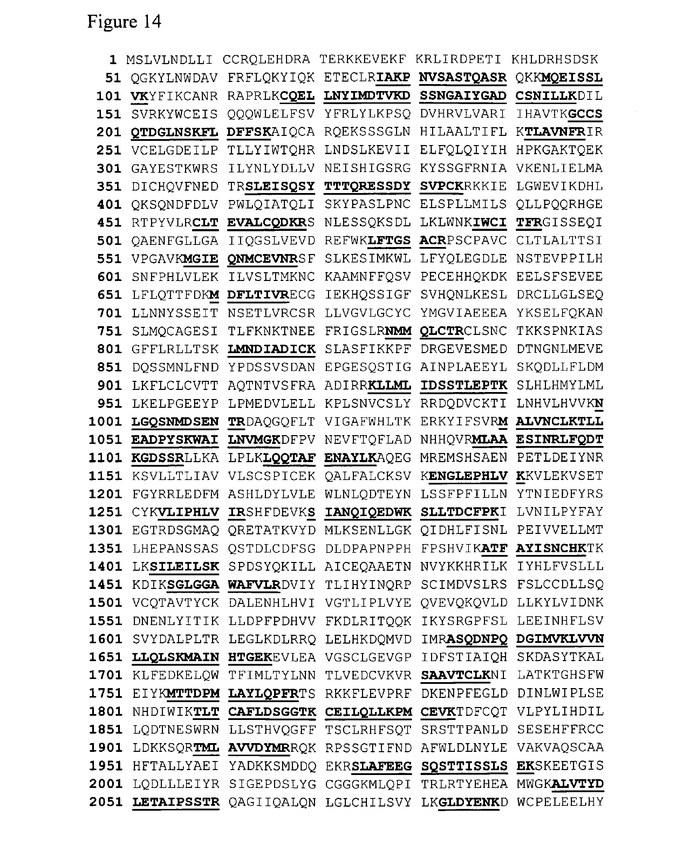

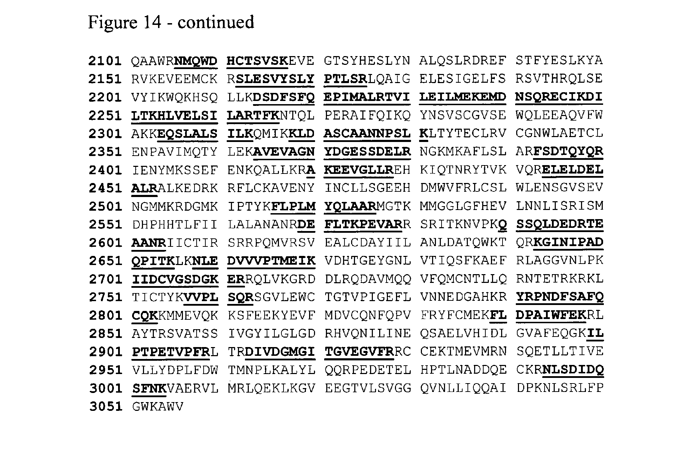

**Figure 14**: Peptides identified by mass spectrometry of ATM after a drug pulldown with immobilized phenylthiazole ligand 1.

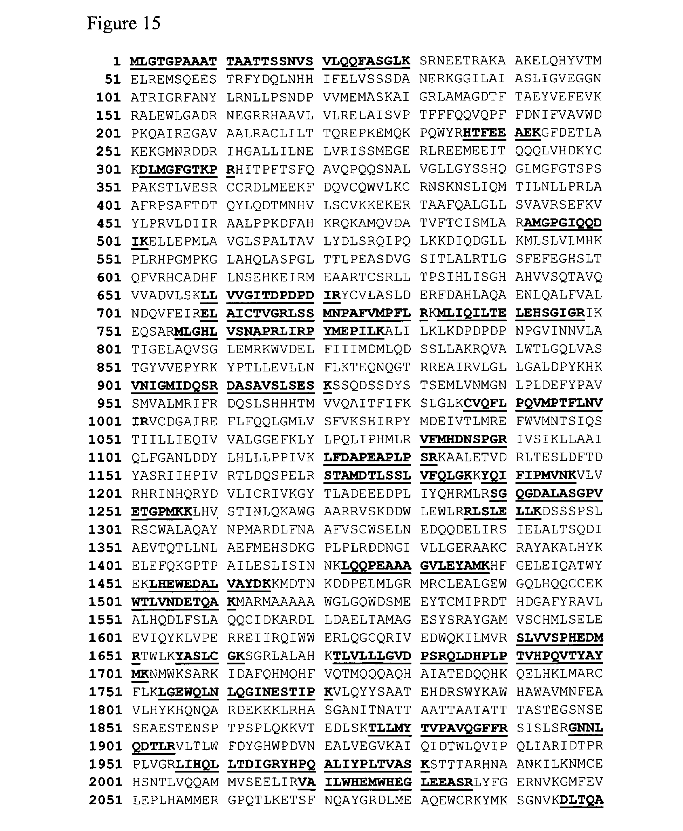

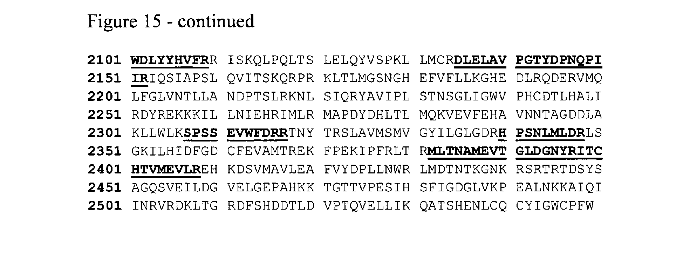

**Figure 15**: Peptides identified by mass spectrometry of mTOR after a drug pulldown with immobilized phenylthiazole ligand 1.
**Figure 16**: Synthesis and structure of the phenylmorpholin-chromen ligand (8-(4-aminomethyl-phenyl)-2-morpholin-4-yl-chromen-4-one). The ligand was synthesized as described in Example 8. The structure of the ligand is shown [G].

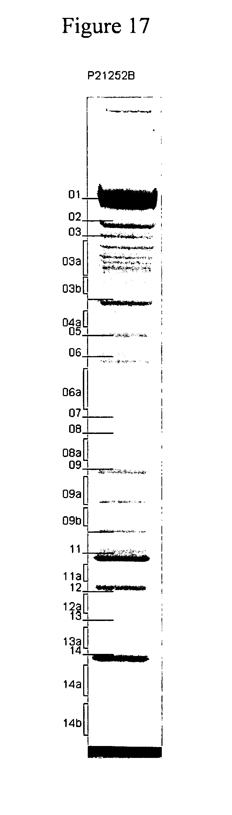

**Figure 17**: Drug pulldown experiment with the immobilized phenylmorpholin-chromen ligand for mass spectrometry analysis of proteins. A protein gel after staining with Coomassie blue is shown. The indicated gel areas were cut out as gel slices and proteins were subjected to analysis by mass spectrometry. The drug pulldown experiment was performed as described in Example 2 with a 1:1 mixture of HeLa and K-562 cell lysate sample containing 50 mg of protein. Proteins bound to the phenylmorpholin-chromen ligand were eluted with SDS sample buffer and separated by SDS-polyacrylamide gel electrophoresis (SDS-PAGE).

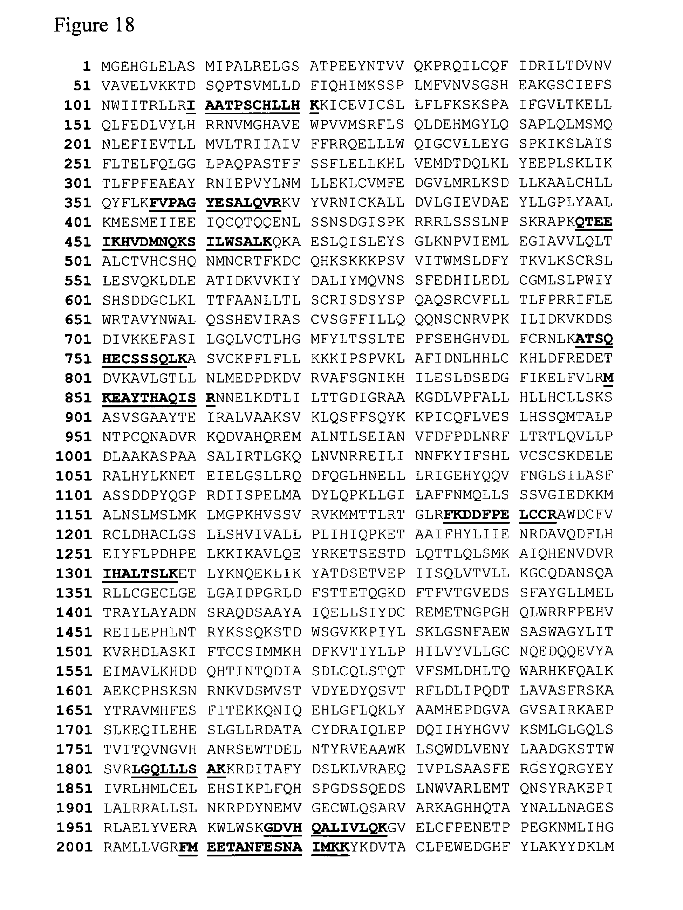

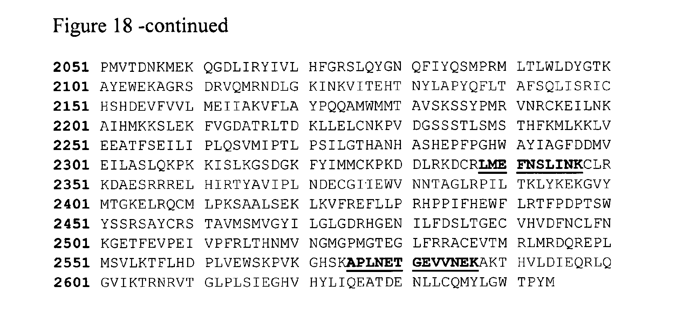

**Figure 18**: Peptides identified by mass spectrometry of human ATR after a drug pulldown with the immobilized phenylmorpholin-chromen ligand from HeLa- K562 lysate mix.

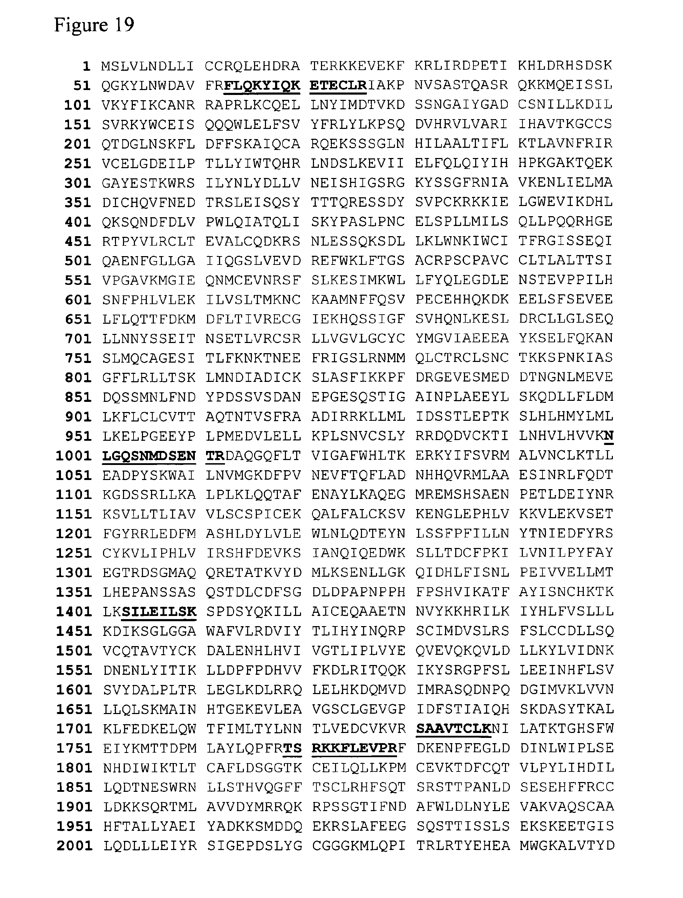

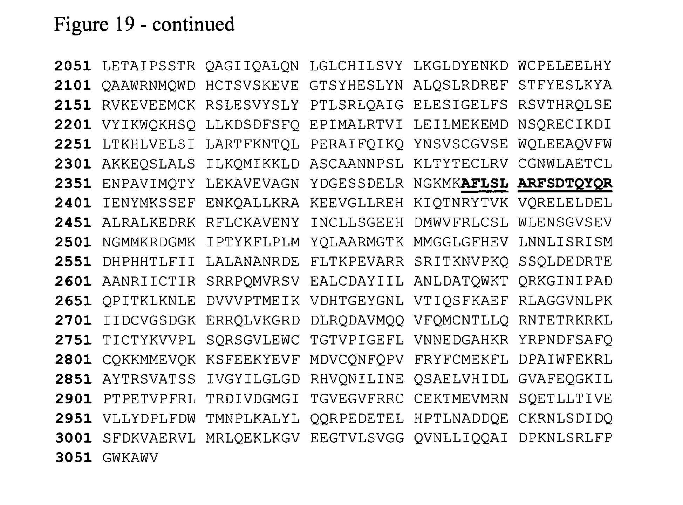

**Figure 19**: Peptides identified by mass spectrometry of human ATM after a drug pulldown with the immobilized phenylmorpholin-chromen ligand from HeLa-K562 lysate mix.

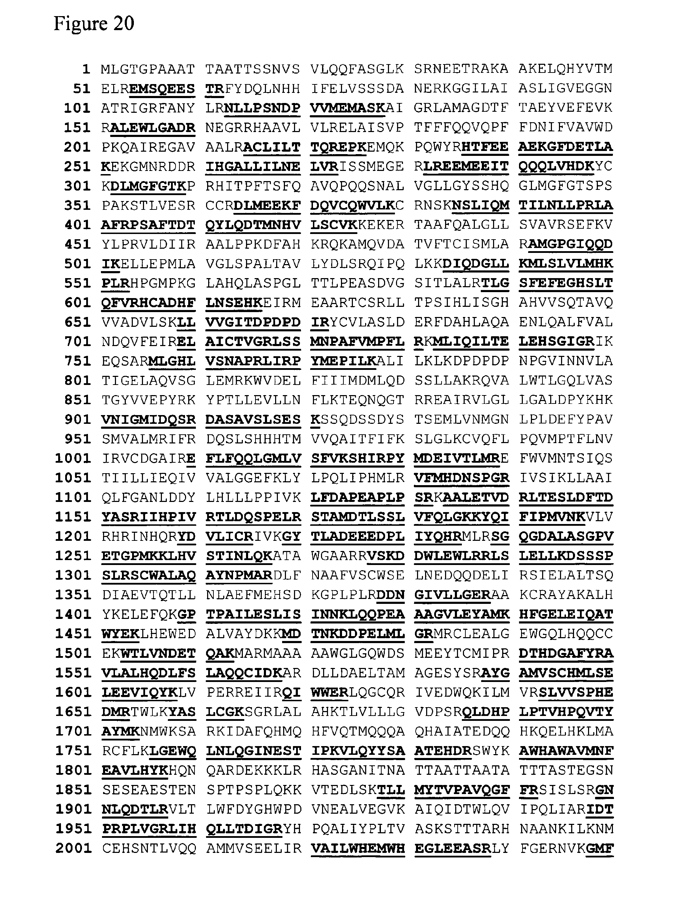

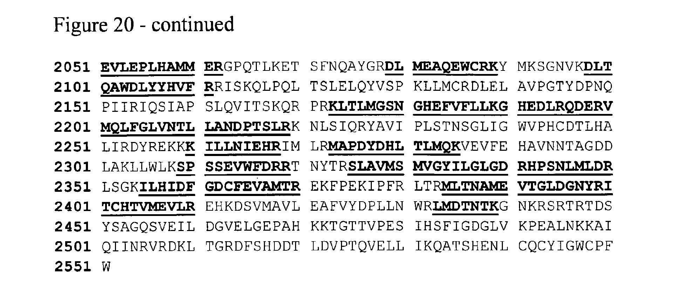

**Figure 20**: Peptides identified by mas spectrometry of human mTOR after a drug pulldown with the immobilized phenylmorpholin-chromen ligand from HeLa-K562 lysate mix.

### Example 1: Preparation of the affinity matrix

This example illustrates the preparation of the affinity matrix for affinity capture of PI3K kinases from cell lysates. The capturing ligand was covalently immobilized on a solid support through covalent linkage using an amino functional group. This affinity matrix was used in example 2, example 3 and example 4.

### Synthesis of phenylthiazole ligand 1 (3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide hydrochloride)

Steps 1-3: 1-bromo-1-(4-chloro-3-methanesulfonyl-phenyl)-propan-2-one was prepared following the procedure described in WO 2003/072557.

### Step 4:5-(4-chloro-3-methanesufonyl-phenyl)-4-methyl-thiazol-2-ylamine

1-bromo-1-(4-chloro-3-methanesulfonyl-phenyl)-propan-2-one (480mg 1.5mmol) and thiourea (114mg 1.5mmol) were combined in ethanol (12ml) and heated to 70°C for 2 hours. The reaction mixture was allowed to cool to room temperature and the solid product was collected by filtration and dried under vacuum to yield 5-(4-chloro-3-methanesufonyl-phenyl)-4-methyl-thiazol-2-ylamine as an off-white solid (375mg). ¹H NMR (400MHz DMSO-*d*₆) δ 9.4 (br s, 2H), 8.0 (d, 1H), 7.9 (d, 1H), 7.8 (dd, 1H), 3.4 (s, 3H), 2.3 (s, 3H).

### Step 5: (2-{2-[2-(2-{2[5-(4-chloro-3-methanesulfonyl-phenyl)-4-methyl-thiazol-2-ylcarbamoyl]-ethoxy}-ethoxy)-ethoxy]-ethoxy}-ethyl)-carbamic acid tert-butyl ester 3-(2-{2-[2-(2-tert-butoxycarbonylamino-ethoxy)-ethoxy]-ethoxy}-ethoxy)-propionic acid (690mg 1.9mmol), EDAC (403mg 2.1mmol), HOBT (284mg 2.1mmol), NMM (420uL 3.8mmol) and 5-(4-chloro-3-methanesufonyl-phenyl)-4-methyl-thiazol-2-ylamine (520mg 1.7mmol) were combined in dimethylformamide (16ml) and stirred over night at room temperature. The solvent was removed under reduced pressure and the residue dissolved in dichloromethane (150ml), washed with 1M HCl aqueous solution (50ml) and saturated aqueous sodium hydrogen carbonate (50ml),dried (Magnesium sulphate), filtered and evaporated. The residue was purified by flash chromatography using 50g IST silica flash cartridge eluting with 0-2% methanol/dichloromethane to yield (2-{2-[2-(2-{2[5-(4-chloro-3-methanesulfonyl-phenyl)-4-methyl-thiazol-2-ylcarbamoyl]-ethoxy}-ethoxy)-ethoxy]-ethoxy}-ethyl)-carbamic acid tert-butyl ester as an oil (1.1g residual solvent present) ¹H NMR (400MHz CDCl3) 10.3 (br s, 1H), 8.2 (s, 1H), 7.6 (m, 2H), 7.2 (br s, 1H), 3.9 (t, 2H) 3.8-3.5 (br m, 14H), 3.3 ( br m, 5H), 2.8 (t, 2H), 2.4 (s, 3H), 1.4 (s, 9H).

### Step 6:3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide hydrochloride

(2-{2-[2-(2-{2[5-(4-chloro-3-methanesulfonyl-phenyl)-4-methyl-thiazol-2-ylcarbamoyl]-ethoxy}-ethoxy)-ethoxy]-ethoxy}-ethyl)-carbamic acid tert-butyl ester (1.0g 1.5mmol) was dissolved in dichloromethane (10ml) and treated with HCl (4ml 4M solution in dioxane). The reaction was stirred at room temperature for 3 hours. The solvent was evaporated and the residue dried under vacuum to yield 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide hydrochloride as a yellow viscous oil ( 830mg residual solvent present) ¹H NMR (400MHz CDCl3) 8.4 (br s, 3H), 8.2 (s, 1H), 7.7 (br d, 1H), 7.6 (br d, 1H) 3.9 (br m, 4H), 3.8-3.6 (br m, 12H), 3.3 (s, 3H), 3.3 (br m, 2H), 3.1 (br m, 2H), 2.6 (s, 3H). NMR spectra were obtained on a Bruker dpx400.

**Table 1: Abbreviations used**

| | |
|---|---|
| br | broad |
| CDCl3 | deuterochloroform |
| d | doublet |
| dd | doublet of doublets |
| DMSO | dimethyl sulphoxide |
| EDAC | 1-ethyl-3-(3'-dimethylaminopropyl)carbodiimide |
| g | gram |
| HCl | Hydrochloric acid |
| HOBT | N-Hydroxybenzotriazole |
| m | multiplet |
| mg | milligram |
| ml | millilitre |
| mmol | millimole |
| M | molar |
| MHz | megahertz |
| NMM | N-methyl morpholine |
| NMR | nuclear magnetic resonance |
| q | quartet |
| s | singlet |
| t | triplet |

### Immobilization of phenylthiazole ligand 1 on beads (affinity matrix)

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in example 2, 3 and 4. Control beads (no ligand immobilized) were generated by blocking the NHS-groups by incubation with aminoethanol as described above.

### Example 2: Drug pulldown of PI3K using immobilized phenylthiazole ligand 1

This example demonstrates the use of the immobilized phenylthiazole ligand 1 for the identification of PI3K proteins from cell lysates of a human T cell line (MOLT-4 cells; ATCC number CRL-1582). To this end a lysate of MOLT-4 cells was contacted with the affinity matrix described in example 1. Proteins binding to the phenylthiazole ligand 1 were identified by Western blot and mass spectrometry (MS) analysis.

For Western blot analysis bound proteins were eluted from the affinity matrix and subsequently separated by SDS-Polyacrylamide gel elecrophoresis. PI3K gamma and PI3K delta were detected with specific antibodies (Figure 2). The results of the Western blot analysis show that immobilized phenylthiazole ligand 1 captures (pulls down) PI3K gamma and PI3K delta from the cell lysate.

For the identification of proteins by mass spectrometry analysis the proteins captured by the affinty matrix were eluted and subsequently separated by SDS-Polyacrylamide gel elecrophoresis (Figure 3). Suitable gel bands were cut out and subjected to in-gel proteolytic digestion with trypsin and analyzed by LC-MS/MS mass spectrometry.

The identification of members of the PI3K family is documented in Table 3. The peptide sequence coverage of PI3K gamma is shown in Figure 4 and for PI3K delta in Figure 5.

### 1. Cell culture

MOLT-4 cells (ATCC number 1582) were grown in 1 litre Spinner flasks (Integra Biosciences, #182101) in suspension in RPMI 1640 medium (Invitrogen, #21875-034) supplemented with 10% Fetal Bovine Serum (Invitrogen) at a density between 0.15 x 10⁶ and 1.2 x 10⁶ cells/ml. Cells were harvested by centrifugation, washed once with 1 x PBS buffer (Invitrogen, #14190-094) and cell pellets were frozen in liquid nitrogen and subsequently stored at -80°C.

### 2. Preparation of cell lysates

MOLT-4 cells were homogenized in a Potter S homogenizer in lysis buffer: 50 mM Tris-HCl, 0.8% NP40, 5% glycerol, 150 mM NaCl, 1.5 mM MgCl2, 25 mM NaF, 1 mM sodium vanadate, 1 mM DTT, pH 7.5. One complete EDTA-free tablet (protease inhibitor cocktail, Roche Diagnostics, 1 873 580) per 25 ml buffer was added. The material was dounced 10 times using a mechanized POTTER S, transferred to 50 ml falcon tubes, incubated for 30 minutes on ice and spun down for 10 min at 20,000 g at 4°C (10,000 rpm in Sorvall SLA600, precooled). The supernatant was transferred to an ultracentrifuge (UZ)-polycarbonate tube (Beckmann, 355654) and spun for 1 hour at 100.000 g at 4°C (33.500 rpm in Ti50.2, precooled). The supernatant was transferred again to a fresh 50 ml falcon tube, the protein concentration was determined by a Bradford assay (BioRad) and samples containing 50 mg of protein per aliquot were prepared. The samples were immediately used for experiments or frozen in liquid nitrogen and stored frozen at -80°C.

### 3. Compound pull-down experiment

Sepharose-beads with immobilized compound (100 µl beads per pull-down experiment) were equilibrated in lysis buffer and incubated with a cell lysate sample containing 50 mg of protein on an end-over-end shaker (Roto Shake Genie, Scientific Industries Inc.) for 2 hours at 4°C. Beads were collected, transfered to Mobicol-columns (MoBiTech 10055) and washed with 10 ml lysis buffer containing 0.5% NP40 detergent, followed by 5 ml lysis buffer with 0.25 % detergent. To elute the bound protein, 60 µl 2 x SDS sample buffer was added, the column was heated for 30 minutes at 50°C and the eluate was transferred to a microfuge tube by centrifugation. Proteins were then separated by SDS-Polyacrylamide electrophoresis (SDS-PAGE).

### 4. Protein detection by Western blot analysis

Western blots were performed according to standard procedures and the PI3K proteins were detected and quantified by using specific anti-PI3K antibodies (first antibody), a fluorescently labeled secondary antibody and the Odyssey Infrared Imaging system from LI-COR Biosciences (Lincoln, Nebraska, USA) according to instructions provided by the manufacturer (Schutz-Geschwendener et al., 2004. Quantitative, two-color Western blot detection with infrared fluorescence. Published May 2004 by LI-COR Biosciences, www.licor.com).

The mouse anti PI3K gamma antibody (Jena Bioscience, catalogue number ABD-026S) was used at a dilution of 1:200 and incubated with the blot over night at 4°C. The secondary anti-mouse IRDye™ 800 antibody (Rockland, ctalogue number 610-732-124) was used at a dilution of 1:15000. The rabbit anti PI3K delta antibody (Santa Cruz, catalogue number sc-7176 was diluted 1:600 and incubated over night at 4°C. As a secondary detection antibody the anti-rabbit IRDyeTM 800 antibody was diluted 1:20 000 (LICOR, catalogue number 926-32211).

### 5. Protein Identification by Mass Spectrometry

### 5.1 Protein digestion prior to mass spectrometric analysis

Gel-separated proteins were reduced, alkylated and digested in gel essentially following the procedure described by Shevchenko et al., 1996, Anal. Chem. 68:850-858. Briefly, gel-separated proteins were excised from the gel using a clean scalpel, reduced using 10 mM DTT (in 5mM ammonium bicarbonate, 54°C, 45 min) and subsequently alkylated with 55 mM iodoacetamid (in 5 mM ammonium bicarbonate) at room temperature in the dark (30 minutes). Reduced and alkylated proteins were digested in gel with porcine trypsin (Promega) at a protease concentration of 12.5 ng/µl in 5mM ammonium bicarbonate. Digestion was allowed to proceed for 4 hours at 37°C and the reaction was subsequently stopped using 5 µl 5% formic acid.

### 5.2 Sample preparation prior to analysis by mass spectrometry

Gel plugs were extracted twice with 20 µl 1% TFA and pooled with acidified digest supernatants. Samples were dried in a a vaccuum centrifuge and resuspended in 10 µl 0.1 % formic acid.

### 5.3. Mass spectrometric data acquisition

Peptide samples were injected into a nano LC system (CapLC, Waters or Ultimate, Dionex) which was directly coupled either to a quadrupole TOF (QTOF2, QTOF Ultima, QTOF Micro, Micromass) or ion trap (LCQ Deca XP) mass spectrometer. Peptides were separated on the LC system using a gradient of aqueous and organic solvents (see below). Solvent A was 5% acetonitrile in 0.5% formic acid and solvent B was 70% acetonitrile in 0.5% formic acid.

**Table 2: Peptides eluting off the LC system were partially sequenced within the mass spectrometer.**

| Time (min) | % solvent A | % solvent B |
|---|---|---|
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |

### 5.4. Protein identification

The peptide mass and fragmentation data generated in the LC-MS/MS experiments were used to query fasta formatted protein and nucleotide sequence databases maintained and updated regularly at the NCBI (for the NCBInr, dbEST and the human and mouse genomes) and European Bioinformatics Institute (EBI, for the human, mouse, D. melanogaster and C. elegans proteome databases). Proteins were identified by correlating the measured peptide mass and fragmentation data with the same data computed from the entries in the database using the software tool Mascot (Matrix Science; Perkins et al., 1999. Probability-based protein identification by searching sequence databases using mass spectrometry data. Electrophoresis 20, 3551-3567). Search criteria varied depending on which mass spectrometer was used for the analysis.

**Table 3: PI3K proteins identified by mass spectrometry (MOLT-4 cells; experiment P15234B; MS sample refers to the gel slice cut out from the polyacrylamide gel (Figure 3).**

| **MS sample** | **Protein accesion number (IPI)** | **Protein name** | **Number of peptides identified** |
|---|---|---|---|
| 4 | IPI00070943.3 | PIK4CA; phosphatidylinositol 4-kinase, catalytic, alpha polypeptide | 62 |
| 5 | IPI00024006.1 | PIK3R4; phosphoinositide-3-kinase, regulatory subunit 4, p150 | 11 |
| 6 | IPI00292690.1 | PIK3CG; phosphoinositide-3-kinase, catalytic, gamma polypeptide | 39 |
| 6 | IPI00298410.2 | PIK3CD; phosphoinositide-3-kinase, catalytic, delta polypeptide | 26 |
| 7 | IPI00298410.2 | PIK3CD; phosphoinositide-3-kinase, catalytic, delta polypeptide | 12 |
| 8 | IPI00002591.3 | PIK4CB; phosphatidylinositol 4-kinase, catalytic, beta polypeptide | 15 |
| 9 | IPI00021448.1 | PIK3R1; phosphoinositide-3-kinase, regulatory subunit 1 (p85 alpha) | 27 |
| 9 | IPI00011736.3 | PIK3R2; phosphoinositide-3-kinase, regulatory subunit 2 (p85 beta) | 8 |
| 14 | IPI00333040.3 | PIK3R1; phosphoinositide-3-kinase, regulatory subunit 1 (p85 alpha) | 7 |

### Example 3: Elution assay for the identification of PI3K gamma interacting compounds

The preparation of the phenylthiazole ligand 1 affinity matrix was done as described in example 1. To screen maximally 80 compounds in a 96 well plate the elution experiment is performed as described below.

### Elution assay

The affinity matrix (1200 µl of beads) was washed 2 x with 30 ml 1x DP-buffer. After each washing step the beads were collected by centrifugation for 2 minutes at 1200 rpm at 4°C in a Heraeus centrifuge. The supernatants were discarded. Finally, the beads were equilibrated in 15 ml binding buffer (1x DP buffer/0.4% NP40). After this incubation time the beads were harvested and mixed in a 50 ml falcon tube with MOLT-4 cell lysate at a protein concentration of 5 mg/ml with a total amount of 75mg protein. The preparation of the lysate was done as described in example 2. Beads and the lysate were incubated for 2 hours at 4°C. After the incubation with the lysate beads were collected by centrifugation as described and transferred to 2 ml columns (MoBiTec, #S10129) and washed with 10 ml 1x DP buffer/0.4% NP40 and 5ml 1x DP buffer/0.2% NP40 . Once the washing buffer had run through the column completely the volume of beads left in the column was calculated (approximately 1000 µl). The beads were resuspended in 4 fold excess of 1x DP-buffer/0.2% NP40 (4 ml) to generate a 20% slurry. For compound elution tests 50 µl of this suspension was added to each well of a 96 well plate (Millipore MultiScreenHTS , MSBVN1210, with lid and 1.2um hydrophilic low protein binding Durapore membrane). To remove residual buffer the 96 well plate was assembled with Assemble filter and collection plate and this sandwich assembly was spun down for 10 seconds at 800 rpm in a centrifuge. Then 40 µl of elution buffer (1x DP-buffer/0.2% NP40) supplemented with the test compound was added to the beads. Test compounds were prepared by diluting them in dilution buffer starting from 40 fold concentrated stock solution in DMSO. The plate was assembled on the collection plate, fixed on an Eppendorf incubator and incubated for 30 minutes at 4°C at 650 rpm shaking. To harvest the eluate the 96 well filter plate assembled on the 96 well collection plate was centrifuged for 1 minute at 800 rpm in a table top centrifuge at 4°C (Heraeus). The eluates were checked for the presence of PI3Kgamma and PI3Kdelta by using a dot blot procedure.

### Detection of eluted PI3K gamma

The eluted PI3K gamma protein was detected and quantified by a dot blot procedure using an antibody directed against PI3K gamma (Jena Bioscience, # ABD-026S), a fluorescently labeled secondary anti mouse IRDye™ 800 (Rockland, #610-732-124) and the Odyssey Infrared Imaging system from LI-COR Biosciences (Lincoln, Nebraska, USA) according to instructions provided by the manufacturer (Schutz-Geschwendener et al., 2004. Quantitative, two-color Western blot detection with infrared fluorescence. Published May 2004 by LI-COR Biosciences, www.licor.com).

Nitrocellulose membranes were treated with 20% ethanol and subsequently washed with 1 x PBS buffer. Eluates (as described above) were combined with 12 µl of 4 x SDS loading buffer (200 mM Tris-HCl pH6.8, 8% SDS, 40% glycerol, 0.04% Bromphenol blue) and applied to the Nitrocellulose membrane with a BioRad dot blot appartus (BioRad, # 170-6545).

For detection of PI3K gamma the membranes were first blocked by incubation with Odyssey blocking buffer for 1 hour. Blocked membranes were incubated for 16 hours at 4°C with the first antibody (mouse anti PI3K gamma from Jena Bioscience, ABD-026S) diluted 1:100 in Odyssey blocking buffer supplemented with 0.2% Tween 20. After washing the membrane four times for 5 minutes with 1 x PBS buffer containing 0.1% Tween 20 the membrane was incubated for 40 minutes with the detection antibody (anti-mouse IRDye™ 800 from Rockland, 610-732-124) , diluted 1:10 000 in Odyssey Blocking Buffer supplemented with 0.2% Tween 20. Afterwards the membrane was washed four times for 5 minutes with 1 x PBS buffer/0.1% Tween 20 and once for 5 minutes with 1 x PBS buffer. Afterwards the membrane was scanned with the Odyssey reader and data were analysed.

**Table 4: Preparation of 5x-DP buffer**

| **Substance:** | **Stock solution** | **Final conc. in 1 x lysis buffer** | **Add for 115 x lysis buffer** |
|---|---|---|---|
| **Tris/HCl pH 7.5** | 1 M | 50 mM | 250 ml |
| **Glycerol** | 87 % | 5% | 288 ml |
| **MgCl₂** | 1 M | 1.5 mM | 7.5 ml |
| **NaCl** | 5 M | 150 mM | 150 ml |
| **Na₃VO₄** | 100 mM | 1 mM | 50 ml |

The 5x-DP buffer was filtered through 0.22 µm filter and stored in 40 ml-aliquots at - 80°C. These solutions were obtained from the following suppliers: 1.0 M Tris/HCl pH 7.5 (Sigma, T-2663), 87% Glycerol (Merck, catalogue number 04091.2500); 1.0 M MgCl₂ (Sigma, M-1028); 5.0 M NaCl (Sigma, S-5150).

### Test compounds for elution

The test compounds listed below were used for elution experiments after dilution as described below. Typically all compounds were dissolved in 100 % DMSO (Fluka, cat.no 41647) at a concentration of 100 mM or 50 mM. Compounds are stored at -20°C. Dilution of test compound for elution experiments: Prepare 50 mM stock by diluting the 100 mM stock 1:1 with 100% DMSO. For elution experiments further dilute the compound with elution buffer (1x DP-buffer/0.2% NP40). Compounds used for elution:
Compound 1: PI3K inhibitor LY294002 (Tocris 1130; Vlahos et al., 1994, J. Biol. Chem. 269, 5241-5248).
Compound 2: PI3K gamma inhibitor (Calbiochem 528106; AS-605240; Camps et al., 2005, Nature Medicine 11, 936-943).
Compound 3: PI3K gamma inhibitor II (Calbiochem 528108; AS-604850; Camps et al., 2005, Nature Medicine 11, 936-943).

### Example 4: Competitive binding assay for the identification of PI3K gamma interacting compounds

This examples demonstrates a competitive binding assay in which test compunds are added directly into a cell lysate. Test compounds (at various concentrations) and the affinity matrix with the immobilzed phenylthiazole ligand 1 were added to lysate aliquots and allowed to bind to the proteins contained in the lysate sample. After the incubation time the beads with captured proteins were separated from the lysate. Bound proteins were then eluted and the presence of PI3K gamma was detected and quantified using a specific antibody in a dot blot procedure and the Odyssey infrared detection system (Figure 7A). Dose response curves for three compounds were generated (Figure 7B).

### Washing of affinity matrix

The affinity matrix as described in example 1 (1.1 ml of dry volume) was washed two times with 15 ml of 1x DP buffer containing 0.4% NP40 and then resupended in 5.5 ml of 1x DP buffer containing 0.4% NP40 (20% beads slurry).

### Preparation of test compounds

Stock solutions of test compounds were prepared in DMSO corresponding to a 100fold higher concentration compared to the final desired test concentraion (e.g. a 4 mM stock solution was prepared for a final test concentration of 4 µM). This dilution scheme resulted in a final DMSO concentration of 1%. For control experiments (no test compound) a buffer containing 1% DMSO was used so that all test samples contained 1% DMSO.
Compound 1: PI3K inhibitor LY294002 (Tocris 1130; Vlahos et al., 1994, J. Biol. Chem. 269, 5241-5248).
Compound 3: PI3K gamma inhibitor II (Calbiochem 528108; AS-604850; Camps et al., 2005, Nature Medicine 11, 936-943).
Compound 4 (CZC00015387).

### Dilution of cell lysate

Cell lysates were prepared as described in example 2. For a typical experiment 1 lysate aliquot containing 50 mg of protein was thawed in a 37°C water bath and then kept at 4°C. To the lysate one volume of 1xDP buffer was added so that a final NP40 concentration of 0.4% was achieved. Then, 1/50 of the final volume of a 50fold concentrated protease inhibitor solution was added (1 tablet of protease inhibitor dissolved in 0.5 ml of 1x DP buffer containing 0.4% NP40; EDTA-free tablet protease inhibitor cocktail from Roche Diagnostics, catalogue number 41647). The lysate was further dilute by adding 1x DP buffer containing 0.4% NP40 so that a final protein concentration of 5 mg/ml was achieved.

### Incubation of lysates with test compound and affinity matrix

A volume of 100 µl of diluted lysate was dispensed into each well of a 96 well filter plate. Then 1.5 µl of test compound diluted in DMSO was added. For control reactions 1.5 µl DMSO without test compound were used. Then 50 µl of affinity matrix (20% slurry) per well were added. The plate was incubated for 2 hours at 4°C on a shaker (750 rpm on a Thermomixer, Eppendorf).

The plate was washed using a vacuum manifold instrument (Millipore, MAVM 096 0R). Each well was washed 4 times with 400 µl of 1x DP buffer containing 0.4% NP-40 and 2 times with 400 µl with 1x DP buffer containing 0.2% NP-40.

For elution the filter plate was placed on a collection plate and 40 µl of 2x sample buffer (100 mM TrisHCl, pH6.8; 4% SDS; 20% glycerol; 0.02% Bromphenol blue) with DTT (50 mM final concentration) was added to each well. The plates were incubated for 30 minutes at room temperature on a shaker (750 rpm on a Thermomixer, Eppendorf). Subsequently the plates were centrifuged for 2 minutes at 1100 rpm (Heraeus centrifuge) and the eluate was collected in the wells of the collection plate.

### Detection and quantification of eluted PI3K gamma

The PI3K gamma protein in the eluates was detected and quantified by a dot blot procedure using a first antibody directed against PI3K gamma (anti PI3K gamma from Jena Bioscience, ABD-026S) and a fluorescently labeled secondary antibody (anti-mouse IRDye™ 800,from Rockland, 610-732-124). The Odyssey Infrared Imaging system from LI-COR Biosciences (Lincoln, Nebraska, USA) was operated according to instructions provided by the manufacturer (Schutz-Geschwendener et al., 2004. Quantitative, two-color Western blot detection with infrared fluorescence. Published May 2004 by LI-COR Biosciences, www.licor.com).

The dot blot apparatus was used according to the instructions of the supplier (Bio-Dot microfiltration apparatus, BioRad 170-65). Nitrocellulose membranes (BioTrace NT Nitrocellulose, PALL BTNT30R) were treated with 20% ethanol and subsequently washed with PBS buffer. Per dot 30 µl of eluate sample were applied and left for 30 min before a vacuum pump was applied.

For detection of PI3K gamma the membranes were first blocked by incubation with Odyssey blocking buffer (LICOR, 927-40000) for 1 hour at room temperature. Blocked membranes were then incubated for 16 hours at 4°C with the first antibody (anti PI3K gamma from Jena Bioscience, ABD-026S) which was diluted in Odyssey blocking buffer containing 0.2% Tween-20. After washing the membrane four times for 5 minutes with PBS buffer containing 0.1% Tween 20 the membrane was incubated for 40 minutes with the detection antibody (anti-mouse IRDye™ 800 from Rockland, 610-732-124) diluted in Odyssey blocking buffer containing 0.2% Tween-20. Afterwards the membrane was washed four times for 5 minutes each with 1 x PBS buffer/0.1% Tween 20 and once for 5 minutes with 1 x PBS buffer. The membrane was kept in PBS buffer at 4°C and then scanned with the Odyssey instrument and signals were recorded and analysed according to the instructions of the manufacturer.

### Example 5: Compound profiling of PI3Kdelta interacting compounds by adding compounds to cell lysates or living cells

This example demonstrates binding assays in which test compounds are added directly into a cell lysate or incubated with living cells (RAW264.7 macrophages).

For the cell lysate competitive binding assay compounds were added to lysate samples and allowed to bind to the proteins contained in the lysate sample. Then the affinity matrix containing the immobilized phenylthiazole ligand was added in order to capture proteins not bound to the test compound. After the incubation time the beads with captured proteins were separated from the lysate by centrifugation. Bead-bound proteins were then eluted and the presence of PI3Kdelta protein was detected and quantified using a specific antibody and the Odyssey infrared detection system.

For the in cell profiling experiment aliquots of life RAW264.7 macrophages were first incubated with compounds for 30 minutes in cell culture medium. During this incubation time the compounds can enter the cells and bind to protein targets within the cells. Then the cells were harvested, cell lysates were prepared and the affinity matrix was added in order to capture proteins not bound to the test compound. After 90 minutes of incubation of the cell lysate with the affinity matrix the beads with the captured proteins were separated from the lysate by centrifugation. Bound proteins were then eluted and the presence of PI3Kdelta was detected and quantified using a specific antibody and the Odyssey infrared detection system.

Both approaches yielded similar results for the cell-permeable reference compound PI-103 (Figure 8). The two other compounds (compound 5 and 6) interacted with PI3Kdelta in the lysate assay but not significantly in the cell assay. A possible reason for this difference is that the latter two compounds were not sufficiently cell-permeable.

### Cell culture

RAW264.7 macrophages (American Type Culture Collection, Rockville, MD) were cultured in Dulbecco's modified Eagle's medium (DMEM, 4mM L-glutamine, 4.5 g/L glucose; Gibco #41965) supplemented with 10 % heat-inactivated fetal bovine serum (Gibco #10270) and 1.5 g/L Sodium bicarbonate (Gibco #25080, 7.5% solution) at 37 °C in a humidified atmosphere in the presence of 5 % CO₂. Macrophages were sub-cultured by scraping the cells from the culture dish in DMEM culture medium using a cell scraper and replating them in fresh culture medium. RAW264.7 macrophages were used for experiments after reaching passage number 3. The cells were washed once with phosphate buffered saline (D-PBS, Gibco #14040), removed from the culture dish in DMEM culture medium and centrifuged at 1,000 rpm at room temperature for 3 minutes. The cell pellet was resuspended in DMEM culture medium and the cell number was determined. 25 x 10⁶ cells were plated onto one 10 cm-culture dish and incubated for 48 hours in fresh DMEM culture medium until they reached approximately 90 % confluence.

### A) Compound profiling in living cells

### Treatment of cells with test compound

The macrophages were washed with D-PBS buffer and fresh DMEM culture-medium was added. Cells were treated with DMEM culture medium containing 0.2 % DMSO (vehicle control) or DMEM culture medium with 10 µM PI-103 (Calbiochem, catalogue number 528100; Knight et al., 2006, Cell 125, 733-747), 10 µM compound 5 or 10 µM compound 6 over a period of 30 minutes. Test compounds were prepared as 20 mM stock solutions in DMSO and further diluted to reach the final concentration of 10 µM compound and 0.2% DMSO in the cell culture medium.

### Preparation of cell lysates

The culture medium was removed, cells were washed once with D-PBS buffer and 4 ml ice-cold D-PBS buffer was added. Macrophages were removed by gently scraping the cells and resuspending them in D-PBS buffer. The cell suspensions were transferred into 15 ml Falcon tubes and kept on ice. The macrophage suspensions were centrifuged at 1500 rpm 4 °C for 3 minutes in a Heraeus Multifuge. The supernatant was removed and the cell pellets were washed with cold D-PBS buffer. After an additional centrifugation step, the cell pellets were quickly frozen in liquid nitrogen. Cells were thawed on ice and lysed by adding 120 µl 1x lysis buffer (1 x DP buffer, 0.8% NP40). The lysates were transferred into 1.5 ml Eppendorf tubes and incubated for 30 minutes rotating at 4 °C and then centrifuged for 10 minutes at 13,200 rpm at 4°C. The supernatants was transferred into ultracentrifuge tubes and centrifuged in a TLA-120.2 rotor at 53,000 rpm (100,000 x g) for 1 hour at 4°C. An aliquot of the clarified supernatant was used for protein quantification performing Bradford assay (Biorad Protein Assay dye concentrate, catalogue number 500-0006). The remaining samples were quickly frozen in liquid nitrogen and stored at -80°C until use in the binding assay.

### Dilution of cell lysate

Cell lysates were prepared as described below from RAW264.7 macrophages. One lysate aliquot was thawed in a 37°C water bath and then kept at 4°C. To the lysate one volume of 1xDP buffer containing protease inhibitor (1 tablet of protease inhibitor dissolved in 25 ml of 1x DP buffer or 25 ml of 1x DP buffer containing 0.8% NP40; EDTA-free tablet protease inhibitor cocktail from Roche Diagnostics, catalogue number 41647) was added so that a final NP40 concentration of 0.8% was achieved. The lysate was further diluted by adding 1x DP buffer containing 0.8% NP40 and proteinase inhibitors so that a final protein concentration of 10 mg/ml was achieved.

### Washing of affinity matrix

The affinity matrix as described in example 1 (0.25 ml of dry bead volume) was washed two times with 10 ml of 1x DP buffer containing 0.2% NP40 and was finally resuspended in 5.0 ml of 1x DP buffer containing 0.2% NP40 (5% beads slurry).

### Incubation of cell lysate with the affinity matrix

A volume of 50 µl of diluted lysate (10 mg/ml protein) was dispensed into each well of a 96 well filter plate. Then 100 µl of affinity matrix (5% slurry) per well were added. The plate was incubated for two hours at 4°C on a shaker (750 rpm on a Thermomixer, Eppendorf). The plate was washed using a vacuum manifold instrument (Millipore, MAVM 096 0R). Each well was washed two times with 220 µl of 1x DP buffer containing 0.4% NP-40. For the elution of proteins the filter plate was placed on a collection plate and 20 µl of 2x sample buffer (100 mM TrisHCl, pH7.4; 4% SDS; 20% glycerol; 0.0002% Bromphenol blue) with DTT (50 mM final concentration) was added to each well. The plates were incubated for 30 minutes at room temperature on a shaker (750 rpm on a Thermomixer, Eppendorf). Subsequently the plates were centrifuged for four minutes at 1100 rpm (Heraeus centrifuge) and the eluate was collected in the wells of the collection plate.

### Detection and quantification of PI3Kdelta

The PI3Kdelta protein in the eluates was detected and quantified by spotting aliquots on a nitrocellulose membrane and detection with a first antibody directed against P13Kdelta and a fluorescently labeled secondary antibody. The nitrocellulose membranes (BioTrace NT Nitrocellulose, PALL BTNT30R) were pretreated with 20% ethanol and subsequently washed with PBS buffer.

For detection of P13Kdelta the membranes were first blocked by incubation with Odyssey blocking buffer (LICOR, 927-40000) for one hour at room temperature. Blocked membranes were then incubated for 16 hours at 4°C with the first antibody (anti P13Kdelta, rabbit polyclonal antibody from Santa Cruz, catalogue number sc-7176) which was diluted 1:800 in Odyssey blocking buffer containing 0.2% Tween-20. After washing the membrane four times for seven minutes with PBS buffer containing 0.1% Tween 20 the membrane was incubated for 60 minutes with the detection antibody (goat ant-rabbit IRDye™ 800CW from LICOR, catalogue number 926-32211) diluted 1:2500 in Odyssey blocking buffer containing 0.2% Tween-20 and 0.02% SDS. Afterwards the membrane was washed four times for 5 minutes each with 1 x PBS buffer/0.1% Tween 20 and once for five minutes with 1 x PBS buffer. The membrane was kept in PBS buffer at 4°C and then scanned with the Odyssey instrument and signals were recorded and analysed according to the instructions of the manufacturer. The Odyssey Infrared Imaging system from LI-COR Biosciences (Lincoln, Nebraska, USA) was operated according to instructions provided by the manufacturer (Schutz-Geschwendener et al., 2004. Quantitative, two-color Western blot detection with infrared fluorescence. Published May 2004 by LI-COR Biosciences, www.licor.com).

### B) Compound profiling in cell lysates

### Preparation of cell lysates

The culture medium was removed, cells were washed once with D-PBS buffer and 4 ml ice-cold D-PBS buffer was added. Macrophages were removed by gently scraping the cells and resuspending them in D-PBS buffer. The cell suspensions were transferred into 15 ml Falcon tubes and kept on ice. The macrophage suspensions were centrifuged at 1500 rpm 4 °C for 3 minutes in a Heraeus Multifuge. The supernatant was removed and the cell pellets were washed with cold D-PBS buffer. After an additional centrifugation step, the cell pellets were quickly frozen in liquid nitrogen. Cells were thawed on ice and lysed by adding 120 µl 1x lysis buffer (1 x DP buffer, 0.8% NP40). The lysates were transferred into 1.5 ml Eppendorf tubes and incubated for 30 minutes rotating at 4 °C and then centrifuged for 10 minutes at 13,200 rpm at 4°C. The supernatants was transferred into ultracentrifuge tubes and centrifuged in a TLA-120.2 rotor at 53,000 rpm (100,000 x g) for 1 hour at 4°C. An aliquot of the clarified supernatant was used for protein quantification performing Bradford assay (Biorad Protein Assay dye concentrate, catalogue number 500-0006). The remaining samples were quickly frozen in liquid nitrogen and stored at -80°C until use in the binding assay.

### Dilution of cell lysate

Cell lysates were prepared as described below from RAW264.7 macrophages. One lysate aliquot was thawed in a 37°C water bath and then kept at 4°C. To the lysate one volume of 1xDP buffer containing protease inhibitor (1 tablet of protease inhibitor dissolved in 25 ml of 1x DP buffer or 25 ml of 1x DP buffer containing 0.8% NP40; EDTA-free tablet protease inhibitor cocktail from Roche Diagnostics, catalogue number 41647) was added so that a final NP40 concentration of 0.8% was achieved. The lysate was further diluted by adding 1x DP buffer containing 0.8% NP40 and proteinase inhibitors so that a final protein concentration of 10 mg/ml was achieved.

### Washing of affinity matrix

The affinity matrix as described in example 1 (0.25 ml of dry bead volume) was washed two times with 10 ml of 1x DP buffer containing 0.2% NP40 and was finally resuspended in 5.0 ml of 1x DP buffer containing 0.2% NP40 (5% beads slurry).

### Preparation of test compounds

For in the lysate competition experiment stock solutions of test compounds were prepared in DMSO corresponding to a 50fold higher concentration compared to the final concentration in the assay (for example a 500 µM stock solution was prepared for a final test concentration of 10 µM). This dilution scheme resulted in a final DMSO concentration of 2% in the assay. For control experiments (no test compound) a buffer containing 2% DMSO was used so that all test samples contained 2% DMSO.

### Incubation of cell lysate with test compound and affinity matrix

A volume of 50 µl of diluted lysate (10 mg/ml protein) was dispensed into each well of a 96 well filter plate. Then 3.0 µl of test compound diluted in DMSO was added. For control reactions 3.0 µl DMSO without test compound were used. Then 100 µl of affinity matrix (5% slurry) per well were added. The plate was incubated for two hours at 4°C on a shaker (750 rpm on a Thermomixer, Eppendorf). The plate was washed using a vacuum manifold instrument (Millipore, MAVM 096 0R). Each well was washed two times with 220 µl of 1x DP buffer containing 0.4% NP-40. For the elution of proteins the filter plate was placed on a collection plate and 20 µl of 2x sample buffer (100 mM TrisHCl, pH7.4; 4% SDS; 20% glycerol; 0.0002% Bromphenol blue) with DTT (50 mM final concentration) was added to each well. The plates were incubated for 30 minutes at room temperature on a shaker (750 rpm on a Thermomixer, Eppendorf). Subsequently the plates were centrifuged for four minutes at 1100 rpm (Heraeus centrifuge) and the eluate was collected in the wells of the collection plate. The detection and quantification of PI3Kdelta was performed as described above.

### Example 6: Selectivity profiling of PI3K interacting compounds using quantitative mass spectrometry

This examples demonstrates a competitive binding assay in which test compounds are added directly into a cell lysate. Test compounds (at various concentrations) and the affinity matrix (1:1 mixture of beads with immobilzed phenylthiazole ligand 1 and beads with immobilized phenylmorpholin-chromen ligand) were added to cell lysate aliquots and allowed to bind to the proteins contained in the lysate sample. After the incutation time the beads with captured proteins were separated from the lysate. Bound proteins were then eluted and the presence of kinases was measured using quantitative mass spectrometry based on the ITRAQ method. The IC₅₀ values for the interaction of three compounds with several kinase were determined (Figure 9).

### Washing of affinity matrix

The affinity matrix (1:1 mixture of beads with immobilzed phenylthiazole ligand 1 and beads with immobilized phenylmorpholin-chromen ligand) was washed two times with 15 ml of 1x DP buffer containing 0.4% NP40 and then resupended in 5.5 ml of 1x DP buffer containing 0.4% NP40 (20% beads slurry).

### Preparation of test compounds

Stock solutions of test compounds were prepared in DMSO corresponding to a 100fold higher concentration compared to the final desired test concentraion (e.g. a 4 mM stock solution was prepared for a final test concentration of 4 µM). This dilution scheme resulted in a final DMSO concentration of 1%. For control experiments (no test compound) a buffer containing 1% DMSO was used so that all test samples contained 1% DMSO.
Compound CZC00018052: dual PI3K/mTOR kinase inhibitor PI-103 (Calbiochem catalogue number 528100; Knight et al., 2006, Cell 125, 733-747).
Compound CZC00015097: PI3K gamma inhibitor I (Calbiochem 528106; AS-605240; Camps et al., 2005, Nature Medicine 11, 936-943).

### Dilution of cell lysate

Cell lysates were prepared from Ramos cells (ATCC number CRL-1596) as described in example 2. For a typical experiment one lysate aliquot containing 50 mg of protein was thawed in a 37°C water bath and then kept at 4°C. To the lysate one volume of 1xDP buffer was added so that a final NP40 concentration of 0.4% was achieved. Then, 1/50 of the final volume of a 50fold concentrated protease inhibitor solution was added (1 tablet of protease inhibitor dissolved in 0.5 ml of 1x DP buffer containing 0.4% NP40; EDTA-free tablet protease inhibitor cocktail from Roche Diagnostics, catalogue number 41647). The lysate was further dilute by adding 1x DP buffer containing 0.4% NP40 so that a final protein concentration of 5 mg/ml was achieved.

### Incubation of lysate with test compound and affinity matrix

A volume of 100 µl of diluted lysate was dispensed into each well of a 96 well filter plate. Then 1.5 µl of test compound diluted in DMSO was added. For control reactions 1.5 µl DMSO without test compound were used. Then 50 µl of affinity matrix (20% slurry) per well were added. The plate was incubated for 2 hours at 4°C on a shaker (750 rpm on a Thermomixer, Eppendorf).

The plate was washed using a vacuum manifold instrument (Millipore, MAVM 096 0R). Each well was washed 4 times with 400 µl of 1x DP buffer containing 0.4% NP-40 and 2 times with 400 µl with 1x DP buffer containing 0.2% NP-40.

For elution the filter plate was placed on a collection plate and 40 µl of 2x sample buffer (100 mM TrisHCl, pH6.8; 4% SDS; 20% glycerol; 0.02% Bromphenol blue) with DTT (50 mM final concentration) was added to each well. The plates were incubated for 30 minutes at room temperature on a shaker (750 rpm on a Thermomixer, Eppendorf). Subsequently the plates were centrifuged for 2 minutes at 1100 rpm (Heraeus centrifuge) and the eluate was collected in the wells of the collection plate.

### Detection and quantification of kinases by mass spectrometry

The kinases in the eluate were detected by mass spectrometry as described in example 2 and quantitative analysis using the ITRAQ method was performed as described previously (WO 2006/134056; Bantscheff et al., 2007. Nature Biotechnology 25, 1035-1044) and IC₅₀ values were calculated for the interaction of individual compounds and kinases (Figure 9).

### Example 7: Selectivity profiling of PI3K interacting compounds using multiplex immunodetection

This examples demonstrates a competitive binding assay in which test compounds are added directly into a cell lysate. Test compounds (at various concentrations) and the affinity matrix (1:1 mixture of beads with immobilzed phenylthiazole ligand 1 and beads with immobilized phenylmorpholin-chromen ligand) were added to cell lysate aliquots and allowed to bind to the proteins contained in the lysate sample. After the incutation time the beads with captured proteins were separated from the lysate. Bound proteins were then eluted and the presence of kinases was detected and quantified using a multiplexed immunodetection format. Dose response curves for individual kinases were generated and IC₅₀ values calculated (Figures 10 and 11).

### Washing of affinity matrix

The affinity matrix (1:1 mixture of beads with immobilzed phenylthiazole ligand 1 and beads with immobilized phenylmorpholin-chromen ligand) was washed two times with 15 ml of 1x DP buffer containing 0.4% NP40 and then resupended in 5.5 ml of 1x DP buffer containing 0.4% NP40 (20% beads slurry).

### Preparation of test compounds

Stock solutions of test compounds were prepared in DMSO corresponding to a 100fold higher concentration compared to the final desired test concentraion (e.g. a 4 mM stock solution was prepared for a final test concentration of 4 µM). This dilution scheme resulted in a final DMSO concentration of 1%. For control experiments (no test compound) a buffer containing 1% DMSO was used so that all test samples contained 1% DMSO. Compound CZC00018052: dual PI3K/mTOR kinase inhibitor PI-103 (Calbiochem catalogue number 528100; Knight et al., 2006, Cell 125, 733-747).

### Dilution of cell lysate

Cell lysates were prepared as described in example 2. For this experiment a 1:1 mixture of Jurkat (ATCC catalogue number TIB-152 Jurkat, cloe E6-1) and Molt-4 (ATCC catalogue number CRL-1582) cell lysates was used. For a typical experiment one lysate aliquot containing 50 mg of protein was thawed in a 37°C water bath and then kept at 4°C. To the lysate one volume of 1xDP buffer was added so that a final NP40 concentration of 0.4% was achieved. Then, 1/50 of the final volume of a 50fold concentrated protease inhibitor solution was added (1 tablet of protease inhibitor dissolved in 0.5 ml of 1x DP buffer containing 0.4% NP40; EDTA-free tablet protease inhibitor cocktail from Roche Diagnostics, catalogue number 41647). The lysate was further dilute by adding 1x DP buffer containing 0.4% NP40 so that a final protein concentration of 5 mg/ml was achieved.

### Incubation of lysates with test compound and affinity matrix

To a 96 well filter plate (Multiscreen Solvinert Filter Plate, Millipore MSRL N04 10) were added: 100 µl affinity matrix (beads) per well, 3 µl of compound solution, and 50 µl of cell lysate. Plates were sealed and incubated for two hours in a cold room on a Thermoxer with shaking (750 rpm). Afterwards the plate was washed twice with 220 µl washing buffer. The beads were then eluted with 20 µl of sample buffer. The eluate was frozen qickly at -80°C and stored at -20°C.

### Detection and quantification of eluted kinases

The kinases in the eluates were detected and quantified by a spotting procedure on Nitrocellulose membranes using a first antibody directed against the kinase of interest and a fluorescently labeled secondary antibody (anti-mouse or anti-rabbit IRDye™ antibodies from Rockland). The Odyssey Infrared Imaging system from LI-COR Biosciences (Lincoln, Nebraska, USA) was operated according to instructions provided by the manufacturer (Schutz-Geschwendener et al., 2004. Quantitative, two-color Western blot detection with infrared fluorescence. Published May 2004 by LI-COR Biosciences, www.licor.com).

After spotting of the eluates the nitrocellulose membrane (BioTrace NT, Millipore #66485) was first blocked by incubation with Odyssey blocking buffer (LICOR, 927-40000) for one hour at room temperature. Blocked membranes were then incubated for 16 hours at 25°C with the first antibody which was diluted in Odyssey blocking buffer containing 0.2% Tween-20. Afterwards the membrane was washed four times for 7 minutes with PBS buffer containing 0.1 % Tween 20. Then the membrane was incubated for 60 minutes at room temperature with the detection antibody (IRDye™ labelled antibody from Rockland) diluted in Odyssey blocking buffer containing 0.2% Tween-20 and 0.02% SDS. Afterwards the membrane was washed four times for 7 minutes each with 1 x PBS buffer/0.1% Tween 20 and once for 5 minutes with 1 x PBS buffer. The membrane was kept in PBS buffer at 4°C and then scanned with the Odyssey instrument. Fluorescence signals were recorded and analysed according to the instructions of the manufacturer.

### Sources of antibodies:

Anti-PI3K gamma mouse (Jena Bioscience ABD-026); anti-PI3K delta (Santa Cruz #sc-7176); anti-PI3K alpha (Cell signaling #4255); anti-DNAPK (Calbiochem #NA57); Licor IRDye 800 mouse (926-32210); Licor IRDye 680 rabbit (926-32221); Licor IRDye 800 rabbit (926-32211); Licor IRDye 680 mouse (926-32220).

### Example 8: Preparation of the affinity matrix with the phenylmorpholin-chromen ligand

This example describes the synthesis of the phenylmorpholin-chromen ligand (8-(4-aminomethyl-phenyl)-2-morpholin-4-yl-chromen-4-one) (Figure 16). This capture ligand was immobilized on a solid support through covalent linkage using an amino functional group and used for the capturing of proteins from cell lysates (see for example Figure 17).

### Synthesis of 8-(4-aminomethyl-phenyl)-2-morpholin-4-yl-chromen-4-one

### Step 1

2,3-Dihydroxy-benzoic acid [A] (25g, 0.16mol) (Sigma-Aldrich, Cat no. 126209) was stirred in methanol (125ml) with concentrated sulphuric acid (1ml) and the reaction heated to gentle reflux over night. It was then concentrated and the residue partitioned between ethyl acetate and saturated aqueous sodium bicarbonate. The organic layer was washed with further saturated aqueous sodium bicarbonate, dried with magnesium sulphate, filtered and concentrated to afford 2,3-dihydroxy-benzoic acid methyl ester [B]. Yield 15.2g, 57%.

HPLC (Method B): (M-H⁺) 167; RT = 2.3min. ¹H NMR: (CDCl₃) δ 10.92 (s, 1H); 7.39 (dd, 1H); 7.13 (dd, 1H); 6.82 (dt, 1H); 5.70 (s, 1H); 3.98 (s, 3H).

### Step 2

2,3-Dihydroxy-benzoic acid methyl ester [B] (15.g, 89mmol) was dissolved in dichloromethane (100ml) with pyridine (3.6ml, 44.6mmol, 0.5eq) and DMAP (272mg, 2.2mmol, 0.025eq) and the reaction cooled in an ice/water bath. Trifluoromethanesulphonic anhydride (16.2ml, 98.2mmol, 1.1eq) was added, the reaction was allowed to warm to room temperature and stirred over night. The reaction mixture was diluted with dichloromethane, washed with 1M hydrochloric acid (150ml), dried with sodium sulphate, filtered and evaporated. The product was recrystallised from ethyl acetate to afford 2-hydroxy-3-trifluoromethanesulfonyloxy-benzoic acid methyl ester [C]. Yield crop 1, 6.5g, 24%. Further recrysatllisation afforded a second crop, 6.8g, 26%.
¹H NMR (CDCl₃): δ 11.11 (s, 1H); 7.80 (dd, 1H); 7.36 (dd, 1H); 6.86 (t, 1H); 3.93 (s, 3H).

### Step 3

A solution of *N*-acetylmorpholine (1.72g, 13.3mmol, 2eq) in 30ml dry tetrahydrofuran under nitrogen was cooled in an acetone/dry ice bath (-78°C) and treated with LDA (10ml, 2M solution in THF, 3eq). The reaction mixture was stirred for 60 minutes then 2-hydroxy-3-trifluoromethanesulfonyloxy-benzoic acid methyl ester [C] (2g, 6.6mmol, leq as a solution in 10ml dry THF) was added. The reaction mixture was allowed to warm from -78°C to room temperature and stirred over night. The reaction was diluted with water (4ml) followed by 2M hydrochloric acid (40ml), then extracted three times with dichloromethane. The extracts were combined, washed with brine, dried with magnesium sulphate, filtered and evaporated. The crude product was purified by flash chromatography eluting with ethyl acetate to afford trifluoro-methanesulfonic acid 2-hydroxy-3-(3-morpholin-4-yl-3-oxo-propionyl)-phenyl ester [D]. Yield 1.06g, 40%
¹H NMR (CDCl₃): δ 7.96 (dd, 1H); 7.49 (dd, 1H); 7.00 (t, 1H); 4.14 (s, 2H); 3.65-3.73 (m, 6H), 3.56 (t, 2H).

### Step 4

Trifluoro-methanesulfonic acid 2-hydroxy-3-(3-morpholin-4-yl-3-oxo-propionyl)-phenyl ester [D] (1.06g, 2.7mmol) in dichloromethane (30ml) was treated with trifluoromethanesulphonic anhydride and stirred over night at room temperature. The reaction mixture was then concentrated, re-dissolved in methanol and stirred for a further 2 hours. The solution was diluted with water and basified to pH8. It was then extracted three times with dichloromethane. The extracts were combined, washed with brine, dried with magnesium sulphate and evaporated to give the crude product as a brown oil. Trituration with ether gave trifluoro-methanesulfonic acid 2-morpholin-4-yl-4-oxo-4H-chromen-8-yl ester [E] as a brown solid. Yield 210mg, 20%.
HPLC (Method B): RT = 2.8min. ¹H NMR (CDCl₃): δ 8.16 (dd, 1H); 7.49 (dd, 1H); 7.40 (t, 1H); 5.62 (s, 1H); 3.85 (dd, 4H), 3.60 (dd, 4H).

### Step 5

Trifluoro-methanesulfonic acid 2-morpholin-4-yl-4-oxo-4H-chromen-8-yl ester [E] (380mg, 1.0mmol), 4-(*N*-Boc-aminomethyl)phenylboronic acid (280mg, 1.1mmol, 1.1eq), potassium carbonate (275mg, 2.0mmol, 2eq) and tetrakis triphenylphosphine palladium (0) (60mg, 0.05mmol 0.05eq) were stirred in dioxane (4ml) and heated to 80°C for 4 hours. The cooled reaction was then filtered and the filtrate concentrated *in vacuo.* The residue was purified by flash chromatography eluting with 0-3% methanol in dichloromethane to afford [4-(2-morpholin-4-yl-4-oxo-4H-chromen-8-yl)-benzyl]-carbamic acid tert-butyl ester [F]. Yield 238mg, 54%.
HPLC (Method A): (MH⁺) 437, (MNa⁺) 459; RT 3.0 min. ¹H NMR (CDCl₃) δ 8.17 (dd, 1H); 7.55 (dd, 1H); 7.49 (d, 2H); 7.37-7.42 (m, 3H); 5.51 (s, 1H), 5.00 (brs, 1H), 4.39 (d, 2H); 3.74 (dd, 4H); 3.35 (dd, 4H); 1.48 (s, 9H).

### Step 6

[4-(2-Morpholin-4-yl-4-oxo-4H-chromen-8-yl)-benzyl]-carbamic acid *tert*-butyl ester [F] (230mg, 0.53mmol), in dichloromethane (5ml) was treated with 4M hydrogen chloride in dioxane (2ml). The reaction was stirred at room temperature for 3 hours during which time a precipitate forms. The solvent was removed *in vacuo* and the residue triturated with ether. The resulting solid was collected by filtration and dried to give 8-(4-aminomethyl-phenyl)-2-morpholin-4-yl-chromen-4-one [G]. Yield 189mg, quantitative. HPLC (Method 18): (MH⁺) 337, (MNa⁺) 359; RT 1.32 min (broad). ¹H NMR (DMSO-d₆): δ 8.54 (brs, 2H); 7.99 (dd, 1H); 7.68-7.73 (m, 3H); 7.62 (d, 2H); 7.51 (t, 1H); 5.79 (s, 1H); 4.09 (q, 2H); 3.68 (t, 4H); 3.41 (t, 4H)

**Table 5: Abbreviations**

| | |
|---|---|
| DCM | Dichloromethane |
| DMAP | 4-(Dimethylamino)pyridine |
| LDA | Lithium diisopropylamide |
| MeOH | Methanol |
| THF | Tetrahydrofuran |

NMR spectra were obtained on a Bruker dpx400. LCMS was carried out on an Agilent 1100 using a ZORBAX^{®} SB-C18, 4.6 x 75 mm, 3.5 micron column. Column flow was 1ml/min and solvents used were water and acetonitrile (0.1% formic acid) with an injection volume of 10ul. Wavelengths were 254 and 210nm. Methods are described below.

**Table 6: Analytical methods**

| **Method** | **Easy Access Method Name** | **ChemStation Method Name** | **Flow Rate** | **Solvent** | **Run Time** |
|---|---|---|---|---|---|
| A | Short column ANL Positive Medium | SANL_PGM.M | 1ml/min | 0-1.5min 30-95% MeCN | 5 min |
| | | | | 1.5-4.5 min 95% MeCN | |
| B | Short column ANL Negative Medium | SANL_NGM.M | 1ml/min | 0-1.5min 30-95% MeCN | 5 min |
| | | | | 1.5-4.5 min 95% MeCN | |

### Immobilization of the phenylmorpholin-chromen ligand on beads (affinity matrix)

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in example 2, 3 and 4. Control beads (no ligand immobilized) were generated by blocking the NHS-groups by incubation with aminoethanol as described above.

The following pages 59 to 62 contain specific embodiments
1. A method for the identification of a PI3K interacting compound, comprising the steps of
   a) providing a protein preparation containing PI3K,
   b) contacting the protein preparation with phenylthiazole ligand 1 immobilized on a solid support under conditions allowing the formation of a phenylthiazole ligand 1 - PI3K complex,
   c) incubating the phenylthiazole ligand 1 - PI3K complex with a given compound,
   d) determining whether the compound is able to separate PI3K from the immobilized phenylthiazole ligand 1, and
   e) determining whether the compound is able to separate also ATM, ATR, DNAPK and/or mTOR from the immobilized phenylthiazole ligand 1.
2. The method of 1, wherein step d) includes the detection of separated PI3K or the determination of the amount of separated PI3K and/or wherein step e) includes the detection of separated ATM, ATR, DNAPK and/or mTOR or the determination of the amount of separated ATM, ATR, DNAPK and/or mTOR.
3. The method of 2, wherein separated PI3K, ATM, ATR, DNAPK and/or mTOR is detected or the amount of separated PI3K, ATM, ATR, DNAPK and/or mTOR is determined by mass spectrometry or immunodetection methods, preferably with an antibody directed against PI3K, ATM, ATR, DNAPK and/or mTOR.
4. A method for the identification of a PI3K interacting compound, comprising the steps of
   a) providing a protein preparation containing PI3K,
   b) contacting the protein preparation with phenylthiazole ligand 1 immobilized on a solid support and with a given compound under conditions allowing the formation of a phenylthiazole ligand 1 - PI3K complex,
   c) detecting the phenylthiazole ligand 1 - PI3K complex formed in step b), and
   d) detecting whether also a complex between phenylthiazole ligand 1 and ATM, ATR, DNAPK and or mTOR has been formed in step b).
5. The method of 4, wherein in step c) said detecting is performed by determining the amount of the phenylthiazole ligand 1 - PI3K complex and/or wherein in step d) the amount of a complex between phenylthiazole ligand 1 and ATM, ATR, DNAPK and or mTOR is determined.
6. The method of 4 or 5, wherein steps a) to c) are performed with several protein preparations in order to test different compounds.
7. A method for the identification of a PI3K interacting compound, comprising the steps of:
   a) providing two aliquots of a protein preparation containing PI3K,
   b) contacting one aliquot with the phenylthiazole ligand 1 immobilized on a solid support under conditions allowing the formation of a phenylthiazole ligand 1 - PI3K complex,
   c) contacting the other aliquot with the phenylthiazole ligand 1 immobilized on a solid support and with a given compound under conditions allowing the formation of a phenylthiazole ligand 1 - PI3K complex,
   d) determining the amount of the phenylthiazole ligand 1 - PI3K complex formed in steps b) and c), and
   e) determining whether also a complex between phenylthiazole ligand 1 and ATM, ATR, DNAPK and or mTOR has been formed in steps b) and c).
8. A method for the identification of a PI3K interacting compound, comprising the steps of:
   a) providing two aliquots comprising each at least one cell containing PI3K,
   b) incubating one aliquot with a given compound,
   c) harvesting the cells of each aliquot,
   d) lysing the cells in order to obtain protein preparations,
   e) contacting the protein preparations with the phenylthiazole ligand 1 immobilized on a solid support under conditions allowing the formation of a phenylthiazole ligand 1 - PI3K complex, and
   f) determining the amount of the phenylthiazole ligand 1 - PI3K complex formed in each aliquot in step e), and
   g) determining whether also a complex between phenylthiazole ligand 1 and ATM, ATR, DNAPK and or mTOR has been formed in step e).
9. The method of 7 or 8, wherein a reduced amount of the phenylthiazole ligand 1 - PI3K complex formed in the aliquot incubated with the compound in comparison to the aliquot not incubated with the compound indicates that PI3K is a target of the compound.
10. The method of 5 to 9, wherein the amount of the phenylthiazole ligand 1 - PI3K complex is determined by separating PI3K from the immobilized phenylthiazole ligand 1 and subsequent detection of separated PI3K or subsequent determination of the amount of separated PI3K.
11. The method of 5 to 10, wherein said determination whether also a complex between phenylthiazole ligand 1 and ATM, ATR, DNAPK and/or mTOR has been formed is performed by separating said protein from the immobilized phenylthiazole ligand 1 and subsequent detection of separated ATM, ATR, DNAPK and or mTOR or subsequent determination of the amount of separated ATM, ATR, DNAPK and or mTOR.
12. The method of 10 or 11, wherein said protein is detected or the amount of said protein is determined by mass spectrometry or immunodetection methods, preferably with an antibody directed against said protein.
13. The method of 1 to 12, performed as a medium or high throughput screening.
14. The method of 1 to 13, wherein said compound is selected from the group consisting of synthetic compounds, or organic synthetic drugs, more preferably small molecule organic drugs, and natural small molecule compounds.
15. The method of 1 to 14, wherein the PI3K interacting compound is a PI3K inhibitor.
16. The method of 1 to 15, wherein the solid support is selected from the group consisting of agarose, modified agarose, sepharose beads (e.g. NHS-activated sepharose), latex, cellulose, and ferro- or ferrimagnetic particles.
17. The method of 1 to 16, wherein the phenylthiazole ligand 1 is covalently coupled to the solid support.
18. A method for the preparation of a pharmaceutical composition comprising the steps of
   a) identifying a PI3K interacting compound according to 1 to 17, and
   b) formulating the interacting compound to a pharmaceutical composition.
19. The method of 1 to 18, wherein the PI3K is PI3K gamma and/or PI3K delta.
20. The method of 1 to 19, wherein the provision of a protein preparation includes the steps of harvesting at least one cell containing PI3K and lysing the cell.
21. The method of 1 to 20, wherein the steps of the formation of the phenylthiazole ligand 1 - PI3K complex are performed under essentially physiological conditions.

## Claims

1. A method for the identification of a PI3K interacting compound, comprising the steps of
a) providing a protein preparation containing PI3K,
b) contacting the protein preparation with phenylthiazole ligand 1 immobilized on a solid support under conditions allowing the formation of a phenylthiazole ligand 1 - PI3K complex,
c) incubating the phenylthiazole ligand 1 - PI3K complex with a given compound,
d) determining whether the compound is able to separate PI3K from the immobilized phenylthiazole ligand 1, and
e) determining whether the compound is able to separate also ATM, ATR, DNAPK and/or mTOR from the immobilized phenylthiazole ligand 1.

2. The method of claim 1, wherein step d) includes the detection of separated PI3K or the determination of the amount of separated PI3K and/or wherein step e) includes the detection of separated ATM, ATR, DNAPK and/or mTOR or the determination of the amount of separated ATM, ATR, DNAPK and/or mTOR.

3. The method of claim 2, wherein separated PI3K, ATM, ATR, DNAPK and/or mTOR is detected or the amount of separated PI3K, ATM, ATR, DNAPK and/or mTOR is determined by mass spectrometry or immunodetection methods, preferably with an antibody directed against PI3K, ATM, ATR, DNAPK and/or mTOR.

4. A method for the identification of a PI3K interacting compound, comprising the steps of
a) providing a protein preparation containing PI3K,
b) contacting the protein preparation with phenylthiazole ligand 1 immobilized on a solid support and with a given compound under conditions allowing the formation of a phenylthiazole ligand 1 - PI3K complex,
c) detecting the phenylthiazole ligand 1 - PI3K complex formed in step b), and
d) detecting whether also a complex between phenylthiazole ligand 1 and ATM, ATR, DNAPK and or mTOR has been formed in step b).

5. The method of claim 4, wherein in step c) said detecting is performed by determining the amount of the phenylthiazole ligand 1 - PI3K complex and/or wherein in step d) the amount of a complex between phenylthiazole ligand 1 and ATM, ATR, DNAPK and or mTOR is determined.

6. The method of any of claims 4 or 5, wherein steps a) to c) are performed with several protein preparations in order to test different compounds.

7. A method for the identification of a PI3K interacting compound, comprising the steps of:
a) providing two aliquots of a protein preparation containing PI3K,
b) contacting one aliquot with the phenylthiazole ligand 1 immobilized on a solid support under conditions allowing the formation of a phenylthiazole ligand 1 - PI3K complex,
c) contacting the other aliquot with the phenylthiazole ligand 1 immobilized on a solid support and with a given compound under conditions allowing the formation of a phenylthiazole ligand 1 - PI3K complex,
d) determining the amount of the phenylthiazole ligand 1 - PI3K complex formed in steps b) and c), and
e) determining whether also a complex between phenylthiazole ligand 1 and ATM, ATR, DNAPK and or mTOR has been formed in steps b) and c).

8. A method for the identification of a PI3K interacting compound, comprising the steps of:
a) providing two aliquots comprising each at least one cell containing PI3K,
b) incubating one aliquot with a given compound,
c) harvesting the cells of each aliquot,
d) lysing the cells in order to obtain protein preparations,
e) contacting the protein preparations with the phenylthiazole ligand 1 immobilized on a solid support under conditions allowing the formation of a phenylthiazole ligand 1 - PI3K complex, and
f) determining the amount of the phenylthiazole ligand 1 - PI3K complex formed in each aliquot in step e), and
g) determining whether also a complex between phenylthiazole ligand 1 and ATM, ATR, DNAPK and or mTOR has been formed in step e).

9. The method of any of claims 7 or 8, wherein a reduced amount of the phenylthiazole ligand 1 - PI3K complex formed in the aliquot incubated with the compound in comparison to the aliquot not incubated with the compound indicates that PI3K is a target of the compound.

10. The method of any of claims 5 to 9, wherein the amount of the phenylthiazole ligand 1 - PI3K complex is determined by separating PI3K from the immobilized phenylthiazole ligand 1 and subsequent detection of separated PI3K or subsequent determination of the amount of separated PI3K.

11. The method of any of claims 5 to 10, wherein said determination whether also a complex between phenylthiazole ligand 1 and ATM, ATR, DNAPK and/or mTOR has been formed is performed by separating said protein from the immobilized phenylthiazole ligand 1 and subsequent detection of separated ATM, ATR, DNAPK and or mTOR or subsequent determination of the amount of separated ATM, ATR, DNAPK and or mTOR.

12. The method of any of claim 10 or 11, wherein said protein is detected or the amount of said protein is determined by mass spectrometry or immunodetection methods, preferably with an antibody directed against said protein.

13. The method of any of claims 1 to 12, performed as a medium or high throughput screening.

14. The method of any of claims 1 to 13, wherein said compound is selected from the group consisting of synthetic compounds, or organic synthetic drugs, more preferably small molecule organic drugs, and natural small molecule compounds.

15. The method of any of claims 1 to 14, wherein the PI3K interacting compound is a PI3K inhibitor.

16. The method of any of claims 1 to 15, wherein the solid support is selected from the group consisting of agarose, modified agarose, sepharose beads (e.g. NHS-activated sepharose), latex, cellulose, and ferro- or ferrimagnetic particles.

17. The method of any of claims 1 to 16, wherein the phenylthiazole ligand 1 is covalently coupled to the solid support.

18. A method for the preparation of a pharmaceutical composition comprising the steps of
a) identifying a PI3K interacting compound according to any of claims 1 to 17, and
b) formulating the interacting compound to a pharmaceutical composition.

19. The method of any of claims 1 to 18, wherein the PI3K is PI3K gamma and/or PI3K delta.

20. The method of any of claims 1 to 19, wherein the provision of a protein preparation includes the steps of harvesting at least one cell containing PI3K and lysing the cell.

21. The method of any of claims 1 to 20, wherein the steps of the formation of the phenylthiazole ligand 1 - PI3K complex are performed under essentially physiological conditions.

22. Use of phenylthiazole ligand 1 for the identification of ATM, ATR, DNAPK and / or mTOR interacting compounds.

23. The use of claim 22, wherein a compound is identified which interacts with PI3K and with at least one enzyme selected from the group consisting of ATM, ATR, DNAPK and mTOR.

24. The method of any of claims 1 to 21 or the use of any of claims 22 or 23, wherein the phenylthiazole ligand 1 is 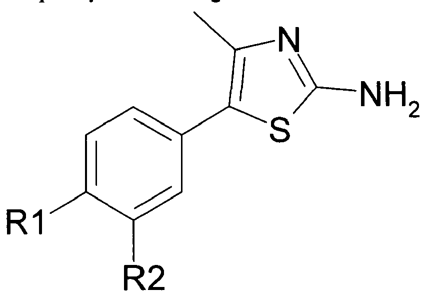 linked to a solid support,
wherein R) is a halogen, preferably chloride or bromide, and
R₂ is selected from the group consisting of hydroxyl, carboxyl, C1-C8 alkyl group, optionally substituted by halogen, and SO₂, preferably wherein R₂ is SO₂.

25. The method or use of claim 24, wherein the phenylthiazole ligand 1 is linked to the solid support via the amino group.

26. The method or use of any of claims 1 to 25, wherein the linker is a C1-C8 alkylcarbonyl or a C1-C8 alkylaminocarbonyl, either of which being optionally substituted by halogen, hydroxy, amino, C1-C8-alkylamino, C1-C8-alkoxycarbonyl, C1-C8-alkoxy optionally substituted by hydroxyl or C1-C8-alkyl optionally substituted by hydroxyl or halogen.

27. The method or use of any of claims 1 to 26, wherein the phenylthiazole ligand 1 is selected from the group consisting of 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide hydrochloride, and 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide.


SEQUENCE LISTING

<110>  Cellzome AG

<120>  Selectivity profiling of PI3K interacting molecules against
       multiple targets

<130>  CEL65406PC

<160>  8

<170>  PatentIn version 3.5

<210>  1
<211>  1102
<212>  PRT
<213>  Homo sapiens

<400>  1

Met Glu Leu Glu Asn Tyr Lys Gln Pro Val Val Leu Arg Glu Asp Asn
1               5                   10                  15

Cys Arg Arg Arg Arg Arg Met Lys Pro Arg Ser Ala Ala Ala Ser Leu
            20                  25                  30

Ser Ser Met Glu Leu Ile Pro Ile Glu Phe Val Leu Pro Thr Ser Gln
        35                  40                  45

Arg Lys Cys Lys Ser Pro Glu Thr Ala Leu Leu His Val Ala Gly His
    50                  55                  60

Gly Asn Val Glu Gln Met Lys Ala Gln Val Trp Leu Arg Ala Leu Glu
65                  70                  75                  80

Thr Ser Val Ala Ala Asp Phe Tyr His Arg Leu Gly Pro His His Phe
                85                  90                  95

Leu Leu Leu Tyr Gln Lys Lys Gly Gln Trp Tyr Glu Ile Tyr Asp Lys
            100                 105                 110

Tyr Gln Val Val Gln Thr Leu Asp Cys Leu Arg Tyr Trp Lys Ala Thr
        115                 120                 125

His Arg Ser Pro Gly Gln Ile His Leu Val Gln Arg His Pro Pro Ser
    130                 135                 140

Glu Glu Ser Gln Ala Phe Gln Arg Gln Leu Thr Ala Leu Ile Gly Tyr
145                 150                 155                 160

Asp Val Thr Asp Val Ser Asn Val His Asp Asp Glu Leu Glu Phe Thr
                165                 170                 175

Arg Arg Gly Leu Val Thr Pro Arg Met Ala Glu Val Ala Ser Arg Asp
            180                 185                 190

Pro Lys Leu Tyr Ala Met His Pro Trp Val Thr Ser Lys Pro Leu Pro

        195                 200                 205

Glu Tyr Leu Trp Lys Lys Ile Ala Asn Asn Cys Ile Phe Ile Val Ile
    210                 215                 220

His Arg Ser Thr Thr Ser Gln Thr Ile Lys Val Ser Pro Asp Asp Thr
225                 230                 235                 240

Pro Gly Ala Ile Leu Gln Ser Phe Phe Thr Lys Met Ala Lys Lys Lys
                245                 250                 255

Ser Leu Met Asp Ile Pro Glu Ser Gln Ser Glu Gln Asp Phe Val Leu
            260                 265                 270

Arg Val Cys Gly Arg Asp Glu Tyr Leu Val Gly Glu Thr Pro Ile Lys
        275                 280                 285

Asn Phe Gln Trp Val Arg His Cys Leu Lys Asn Gly Glu Glu Ile His
    290                 295                 300

Val Val Leu Asp Thr Pro Pro Asp Pro Ala Leu Asp Glu Val Arg Lys
305                 310                 315                 320

Glu Glu Trp Pro Leu Val Asp Asp Cys Thr Gly Val Thr Gly Tyr His
                325                 330                 335

Glu Gln Leu Thr Ile His Gly Lys Asp His Glu Ser Val Phe Thr Val
            340                 345                 350

Ser Leu Trp Asp Cys Asp Arg Lys Phe Arg Val Lys Ile Arg Gly Ile
        355                 360                 365

Asp Ile Pro Val Leu Pro Arg Asn Thr Asp Leu Thr Val Phe Val Glu
    370                 375                 380

Ala Asn Ile Gln His Gly Gln Gln Val Leu Cys Gln Arg Arg Thr Ser
385                 390                 395                 400

Pro Lys Pro Phe Thr Glu Glu Val Leu Trp Asn Val Trp Leu Glu Phe
                405                 410                 415

Ser Ile Lys Ile Lys Asp Leu Pro Lys Gly Ala Leu Leu Asn Leu Gln
            420                 425                 430

Ile Tyr Cys Gly Lys Ala Pro Ala Leu Ser Ser Lys Ala Ser Ala Glu
        435                 440                 445

Ser Pro Ser Ser Glu Ser Lys Gly Lys Val Gln Leu Leu Tyr Tyr Val
    450                 455                 460

Asn Leu Leu Leu Ile Asp His Arg Phe Leu Leu Arg Arg Gly Glu Tyr
465                 470                 475                 480

Val Leu His Met Trp Gln Ile Ser Gly Lys Gly Glu Asp Gln Gly Ser
                485                 490                 495

Phe Asn Ala Asp Lys Leu Thr Ser Ala Thr Asn Pro Asp Lys Glu Asn
            500                 505                 510

Ser Met Ser Ile Ser Ile Leu Leu Asp Asn Tyr Cys His Pro Ile Ala
        515                 520                 525

Leu Pro Lys His Gln Pro Thr Pro Asp Pro Glu Gly Asp Arg Val Arg
    530                 535                 540

Ala Glu Met Pro Asn Gln Leu Arg Lys Gln Leu Glu Ala Ile Ile Ala
545                 550                 555                 560

Thr Asp Pro Leu Asn Pro Leu Thr Ala Glu Asp Lys Glu Leu Leu Trp
                565                 570                 575

His Phe Arg Tyr Glu Ser Leu Lys His Pro Lys Ala Tyr Pro Lys Leu
            580                 585                 590

Phe Ser Ser Val Lys Trp Gly Gln Gln Glu Ile Val Ala Lys Thr Tyr
        595                 600                 605

Gln Leu Leu Ala Arg Arg Glu Val Trp Asp Gln Ser Ala Leu Asp Val
    610                 615                 620

Gly Leu Thr Met Gln Leu Leu Asp Cys Asn Phe Ser Asp Glu Asn Val
625                 630                 635                 640

Arg Ala Ile Ala Val Gln Lys Leu Glu Ser Leu Glu Asp Asp Asp Val
                645                 650                 655

Leu His Tyr Leu Leu Gln Leu Val Gln Ala Val Lys Phe Glu Pro Tyr
            660                 665                 670

His Asp Ser Ala Leu Ala Arg Phe Leu Leu Lys Arg Gly Leu Arg Asn
        675                 680                 685

Lys Arg Ile Gly His Phe Leu Phe Trp Phe Leu Arg Ser Glu Ile Ala
    690                 695                 700

Gln Ser Arg His Tyr Gln Gln Arg Phe Ala Val Ile Leu Glu Ala Tyr
705                 710                 715                 720

Leu Arg Gly Cys Gly Thr Ala Met Leu His Asp Phe Thr Gln Gln Val
                725                 730                 735

Gln Val Ile Glu Met Leu Gln Lys Val Thr Leu Asp Ile Lys Ser Leu
            740                 745                 750

Ser Ala Glu Lys Tyr Asp Val Ser Ser Gln Val Ile Ser Gln Leu Lys
        755                 760                 765

Gln Lys Leu Glu Asn Leu Gln Asn Ser Gln Leu Pro Glu Ser Phe Arg
    770                 775                 780

Val Pro Tyr Asp Pro Gly Leu Lys Ala Gly Ala Leu Ala Ile Glu Lys
785                 790                 795                 800

Cys Lys Val Met Ala Ser Lys Lys Lys Pro Leu Trp Leu Glu Phe Lys
                805                 810                 815

Cys Ala Asp Pro Thr Ala Leu Ser Asn Glu Thr Ile Gly Ile Ile Phe
            820                 825                 830

Lys His Gly Asp Asp Leu Arg Gln Asp Met Leu Ile Leu Gln Ile Leu
        835                 840                 845

Arg Ile Met Glu Ser Ile Trp Glu Thr Glu Ser Leu Asp Leu Cys Leu
    850                 855                 860

Leu Pro Tyr Gly Cys Ile Ser Thr Gly Asp Lys Ile Gly Met Ile Glu
865                 870                 875                 880

Ile Val Lys Asp Ala Thr Thr Ile Ala Lys Ile Gln Gln Ser Thr Val
                885                 890                 895

Gly Asn Thr Gly Ala Phe Lys Asp Glu Val Leu Asn His Trp Leu Lys
            900                 905                 910

Glu Lys Ser Pro Thr Glu Glu Lys Phe Gln Ala Ala Val Glu Arg Phe
        915                 920                 925

Val Tyr Ser Cys Ala Gly Tyr Cys Val Ala Thr Phe Val Leu Gly Ile
    930                 935                 940

Gly Asp Arg His Asn Asp Asn Ile Met Ile Thr Glu Thr Gly Asn Leu
945                 950                 955                 960

Phe His Ile Asp Phe Gly His Ile Leu Gly Asn Tyr Lys Ser Phe Leu
                965                 970                 975

Gly Ile Asn Lys Glu Arg Val Pro Phe Val Leu Thr Pro Asp Phe Leu
            980                 985                 990

Phe Val Met Gly Thr Ser Gly Lys  Lys Thr Ser Pro His  Phe Gln Lys
        995                 1000                 1005

Phe Gln  Asp Ile Cys Val Lys  Ala Tyr Leu Ala Leu  Arg His His
    1010                 1015                 1020

Thr Asn  Leu Leu Ile Ile Leu  Phe Ser Met Met Leu  Met Thr Gly
    1025                    1030                    1035

Met Pro  Gln Leu Thr Ser Lys  Glu Asp Ile Glu Tyr  Ile Arg Asp
    1040                    1045                    1050

Ala Leu  Thr Val Gly Lys Asn  Glu Glu Asp Ala Lys  Lys Tyr Phe
    1055                    1060                    1065

Leu Asp  Gln Ile Glu Val Cys  Arg Asp Lys Gly Trp  Thr Val Gln
    1070                    1075                    1080

Phe Asn  Trp Phe Leu His Leu  Val Leu Gly Ile Lys  Gln Gly Glu
    1085                    1090                    1095

Lys His  Ser Ala
    1100

<210>  2
<211>  1044
<212>  PRT
<213>  Homo sapiens

<400>  2

Met Pro Pro Gly Val Asp Cys Pro Met Glu Phe Trp Thr Lys Glu Glu
1               5                   10                  15

Asn Gln Ser Val Val Val Asp Phe Leu Leu Pro Thr Gly Val Tyr Leu
            20                  25                  30

Asn Phe Pro Val Ser Arg Asn Ala Asn Leu Ser Thr Ile Lys Gln Leu
        35                  40                  45

Leu Trp His Arg Ala Gln Tyr Glu Pro Leu Phe His Met Leu Ser Gly
    50                  55                  60

Pro Glu Ala Tyr Val Phe Thr Cys Ile Asn Gln Thr Ala Glu Gln Gln
65                  70                  75                  80

Glu Leu Glu Asp Glu Gln Arg Arg Leu Cys Asp Val Gln Pro Phe Leu
                85                  90                  95

Pro Val Leu Arg Leu Val Ala Arg Glu Gly Asp Arg Val Lys Lys Leu
            100                 105                 110

Ile Asn Ser Gln Ile Ser Leu Leu Ile Gly Lys Gly Leu His Glu Phe
        115                 120                 125

Asp Ser Leu Cys Asp Pro Glu Val Asn Asp Phe Arg Ala Lys Met Cys
    130                 135                 140

Gln Phe Cys Glu Glu Ala Ala Ala Arg Arg Gln Gln Leu Gly Trp Glu
145                 150                 155                 160

Ala Trp Leu Gln Tyr Ser Phe Pro Leu Gln Leu Glu Pro Ser Ala Gln
                165                 170                 175

Thr Trp Gly Pro Gly Thr Leu Arg Leu Pro Asn Arg Ala Leu Leu Val
            180                 185                 190

Asn Val Lys Phe Glu Gly Ser Glu Glu Ser Phe Thr Phe Gln Val Ser
        195                 200                 205

Thr Lys Asp Val Pro Leu Ala Leu Met Ala Cys Ala Leu Arg Lys Lys
    210                 215                 220

Ala Thr Val Phe Arg Gln Pro Leu Val Glu Gln Pro Glu Asp Tyr Thr
225                 230                 235                 240

Leu Gln Val Asn Gly Arg His Glu Tyr Leu Tyr Gly Ser Tyr Pro Leu
                245                 250                 255

Cys Gln Phe Gln Tyr Ile Cys Ser Cys Leu His Ser Gly Leu Thr Pro
            260                 265                 270

His Leu Thr Met Val His Ser Ser Ser Ile Leu Ala Met Arg Asp Glu
        275                 280                 285

Gln Ser Asn Pro Ala Pro Gln Val Gln Lys Pro Arg Ala Lys Pro Pro
    290                 295                 300

Pro Ile Pro Ala Lys Lys Pro Ser Ser Val Ser Leu Trp Ser Leu Glu
305                 310                 315                 320

Gln Pro Phe Arg Ile Glu Leu Ile Gln Gly Ser Lys Val Asn Ala Asp
                325                 330                 335

Glu Arg Met Lys Leu Val Val Gln Ala Gly Leu Phe His Gly Asn Glu
            340                 345                 350

Met Leu Cys Lys Thr Val Ser Ser Ser Glu Val Ser Val Cys Ser Glu
        355                 360                 365

Pro Val Trp Lys Gln Arg Leu Glu Phe Asp Ile Asn Ile Cys Asp Leu
    370                 375                 380

Pro Arg Met Ala Arg Leu Cys Phe Ala Leu Tyr Ala Val Ile Glu Lys
385                 390                 395                 400

Ala Lys Lys Ala Arg Ser Thr Lys Lys Lys Ser Lys Lys Ala Asp Cys
                405                 410                 415

Pro Ile Ala Trp Ala Asn Leu Met Leu Phe Asp Tyr Lys Asp Gln Leu
            420                 425                 430

Lys Thr Gly Glu Arg Cys Leu Tyr Met Trp Pro Ser Val Pro Asp Glu
        435                 440                 445

Lys Gly Glu Leu Leu Asn Pro Thr Gly Thr Val Arg Ser Asn Pro Asn
    450                 455                 460

Thr Asp Ser Ala Ala Ala Leu Leu Ile Cys Leu Pro Glu Val Ala Pro
465                 470                 475                 480

His Pro Val Tyr Tyr Pro Ala Leu Glu Lys Ile Leu Glu Leu Gly Arg
                485                 490                 495

His Ser Glu Cys Val His Val Thr Glu Glu Glu Gln Leu Gln Leu Arg
            500                 505                 510

Glu Ile Leu Glu Arg Arg Gly Ser Gly Glu Leu Tyr Glu His Glu Lys
        515                 520                 525

Asp Leu Val Trp Lys Leu Arg His Glu Val Gln Glu His Phe Pro Glu
    530                 535                 540

Ala Leu Ala Arg Leu Leu Leu Val Thr Lys Trp Asn Lys His Glu Asp
545                 550                 555                 560

Val Ala Gln Met Leu Tyr Leu Leu Cys Ser Trp Pro Glu Leu Pro Val
                565                 570                 575

Leu Ser Ala Leu Glu Leu Leu Asp Phe Ser Phe Pro Asp Cys His Val
            580                 585                 590

Gly Ser Phe Ala Ile Lys Ser Leu Arg Lys Leu Thr Asp Asp Glu Leu
        595                 600                 605

Phe Gln Tyr Leu Leu Gln Leu Val Gln Val Leu Lys Tyr Glu Ser Tyr
    610                 615                 620

Leu Asp Cys Glu Leu Thr Lys Phe Leu Leu Asp Arg Ala Leu Ala Asn
625                 630                 635                 640

Arg Lys Ile Gly His Phe Leu Phe Trp His Leu Arg Ser Glu Met His
                645                 650                 655

Val Pro Ser Val Ala Leu Arg Phe Gly Leu Ile Leu Glu Ala Tyr Cys
            660                 665                 670

Arg Gly Arg Thr His His Met Lys Val Leu Met Lys Gln Gly Glu Ala
        675                 680                 685

Leu Ser Lys Leu Lys Ala Leu Asn Asp Phe Val Lys Leu Ser Ser Gln
    690                 695                 700

Lys Thr Pro Lys Pro Gln Thr Lys Glu Leu Met His Leu Cys Met Arg
705                 710                 715                 720

Gln Glu Ala Tyr Leu Glu Ala Leu Ser His Leu Gln Ser Pro Leu Asp
                725                 730                 735

Pro Ser Thr Leu Leu Ala Glu Val Cys Val Glu Gln Cys Thr Phe Met
            740                 745                 750

Asp Ser Lys Met Lys Pro Leu Trp Ile Met Tyr Ser Asn Glu Glu Ala
        755                 760                 765

Gly Ser Gly Gly Ser Val Gly Ile Ile Phe Lys Asn Gly Asp Asp Leu
    770                 775                 780

Arg Gln Asp Met Leu Thr Leu Gln Met Ile Gln Leu Met Asp Val Leu
785                 790                 795                 800

Trp Lys Gln Glu Gly Leu Asp Leu Arg Met Thr Pro Tyr Gly Cys Leu
                805                 810                 815

Pro Thr Gly Asp Arg Thr Gly Leu Ile Glu Val Val Leu Arg Ser Asp
            820                 825                 830

Thr Ile Ala Asn Ile Gln Leu Asn Lys Ser Asn Met Ala Ala Thr Ala
        835                 840                 845

Ala Phe Asn Lys Asp Ala Leu Leu Asn Trp Leu Lys Ser Lys Asn Pro
    850                 855                 860

Gly Glu Ala Leu Asp Arg Ala Ile Glu Glu Phe Thr Leu Ser Cys Ala
865                 870                 875                 880

Gly Tyr Cys Val Ala Thr Tyr Val Leu Gly Ile Gly Asp Arg His Ser
                885                 890                 895

Asp Asn Ile Met Ile Arg Glu Ser Gly Gln Leu Phe His Ile Asp Phe
            900                 905                 910

Gly His Phe Leu Gly Asn Phe Lys Thr Lys Phe Gly Ile Asn Arg Glu
        915                 920                 925

Arg Val Pro Phe Ile Leu Thr Tyr Asp Phe Val His Val Ile Gln Gln
    930                 935                 940

Gly Lys Thr Asn Asn Ser Glu Lys Phe Glu Arg Phe Arg Gly Tyr Cys
945                 950                 955                 960

Glu Arg Ala Tyr Thr Ile Leu Arg Arg His Gly Leu Leu Phe Leu His
                965                 970                 975

Leu Phe Ala Leu Met Arg Ala Ala Gly Leu Pro Glu Leu Ser Cys Ser

980                 985                 990

Lys Asp Ile Gln Tyr Leu Lys Asp  Ser Leu Ala Leu Gly  Lys Thr Glu
        995                 1000                1005

Glu Glu  Ala Leu Lys His Phe  Arg Val Lys Phe Asn  Glu Ala Leu
    1010                1015                1020

Arg Glu  Ser Trp Lys Thr Lys  Val Asn Trp Leu Ala  His Asn Val
    1025                1030                1035

Ser Lys  Asp Asn Arg Gln
    1040

<210>  3
<211>  4128
<212>  PRT
<213>  Homo sapiens

<400>  3

<210> 4
<211> 3056
<212> PRT
<213> Homo sapiens

<400> 4

Met Ser Leu Val Leu Asn Asp Leu Leu Ile Cys Cys Arg Gln Leu Glu
1               5                   10                  15

His Asp Arg Ala Thr Glu Arg Lys Lys Glu Val Glu Lys Phe Lys Arg
            20                  25                  30

Leu Ile Arg Asp Pro Glu Thr Ile Lys His Leu Asp Arg His Ser Asp
        35                  40                  45

Ser Lys Gln Gly Lys Tyr Leu Asn Trp Asp Ala Val Phe Arg Phe Leu
    50                  55                  60

Gln Lys Tyr Ile Gln Lys Glu Thr Glu Cys Leu Arg Ile Ala Lys Pro
65                  70                  75                  80

Asn Val Ser Ala Ser Thr Gln Ala Ser Arg Gln Lys Lys Met Gln Glu
                85                  90                  95

Ile Ser Ser Leu Val Lys Tyr Phe Ile Lys Cys Ala Asn Arg Arg Ala
            100                 105                 110

Pro Arg Leu Lys Cys Gln Glu Leu Leu Asn Tyr Ile Met Asp Thr Val
        115                 120                 125

Lys Asp Ser Ser Asn Gly Ala Ile Tyr Gly Ala Asp Cys Ser Asn Ile
    130                 135                 140

Leu Leu Lys Asp Ile Leu Ser Val Arg Lys Tyr Trp Cys Glu Ile Ser
145                 150                 155                 160

Gln Gln Gln Trp Leu Glu Leu Phe Ser Val Tyr Phe Arg Leu Tyr Leu
                165                 170                 175

Lys Pro Ser Gln Asp Val His Arg Val Leu Val Ala Arg Ile Ile His
            180                 185                 190

Ala Val Thr Lys Gly Cys Cys Ser Gln Thr Asp Gly Leu Asn Ser Lys
        195                 200                 205

Phe Leu Asp Phe Phe Ser Lys Ala Ile Gln Cys Ala Arg Gln Glu Lys
    210                 215                 220

Ser Ser Ser Gly Leu Asn His Ile Leu Ala Ala Leu Thr Ile Phe Leu
225                 230                 235                 240

Lys Thr Leu Ala Val Asn Phe Arg Ile Arg Val Cys Glu Leu Gly Asp
                245                 250                 255

Glu Ile Leu Pro Thr Leu Leu Tyr Ile Trp Thr Gln His Arg Leu Asn
            260                 265                 270

Asp Ser Leu Lys Glu Val Ile Ile Glu Leu Phe Gln Leu Gln Ile Tyr
        275                 280                 285

Ile His His Pro Lys Gly Ala Lys Thr Gln Glu Lys Gly Ala Tyr Glu
    290                 295                 300

Ser Thr Lys Trp Arg Ser Ile Leu Tyr Asn Leu Tyr Asp Leu Leu Val
305                 310                 315                 320

Asn Glu Ile Ser His Ile Gly Ser Arg Gly Lys Tyr Ser Ser Gly Phe
                325                 330                 335

Arg Asn Ile Ala Val Lys Glu Asn Leu Ile Glu Leu Met Ala Asp Ile
            340                 345                 350

Cys His Gln Val Phe Asn Glu Asp Thr Arg Ser Leu Glu Ile Ser Gln
        355                 360                 365

Ser Tyr Thr Thr Thr Gln Arg Glu Ser Ser Asp Tyr Ser Val Pro Cys
    370                 375                 380

Lys Arg Lys Lys Ile Glu Leu Gly Trp Glu Val Ile Lys Asp His Leu
385                 390                 395                 400

Gln Lys Ser Gln Asn Asp Phe Asp Leu Val Pro Trp Leu Gln Ile Ala
                405                 410                 415

Thr Gln Leu Ile Ser Lys Tyr Pro Ala Ser Leu Pro Asn Cys Glu Leu
            420                 425                 430

Ser Pro Leu Leu Met Ile Leu Ser Gln Leu Leu Pro Gln Gln Arg His
        435                 440                 445

Gly Glu Arg Thr Pro Tyr Val Leu Arg Cys Leu Thr Glu Val Ala Leu
    450                 455                 460

Cys Gln Asp Lys Arg Ser Asn Leu Glu Ser Ser Gln Lys Ser Asp Leu
465                 470                 475                 480

Leu Lys Leu Trp Asn Lys Ile Trp Cys Ile Thr Phe Arg Gly Ile Ser
                485                 490                 495

Ser Glu Gln Ile Gln Ala Glu Asn Phe Gly Leu Leu Gly Ala Ile Ile

            500                 505                 510

Gln Gly Ser Leu Val Glu Val Asp Arg Glu Phe Trp Lys Leu Phe Thr
        515                 520                 525

Gly Ser Ala Cys Arg Pro Ser Cys Pro Ala Val Cys Cys Leu Thr Leu
    530                 535                 540

Ala Leu Thr Thr Ser Ile Val Pro Gly Ala Val Lys Met Gly Ile Glu
545                 550                 555                 560

Gln Asn Met Cys Glu Val Asn Arg Ser Phe Ser Leu Lys Glu Ser Ile
                565                 570                 575

Met Lys Trp Leu Leu Phe Tyr Gln Leu Glu Gly Asp Leu Glu Asn Ser
            580                 585                 590

Thr Glu Val Pro Pro Ile Leu His Ser Asn Phe Pro His Leu Val Leu
        595                 600                 605

Glu Lys Ile Leu Val Ser Leu Thr Met Lys Asn Cys Lys Ala Ala Met
    610                 615                 620

Asn Phe Phe Gln Ser Val Pro Glu Cys Glu His His Gln Lys Asp Lys
625                 630                 635                 640

Glu Glu Leu Ser Phe Ser Glu Val Glu Glu Leu Phe Leu Gln Thr Thr
                645                 650                 655

Phe Asp Lys Met Asp Phe Leu Thr Ile Val Arg Glu Cys Gly Ile Glu
            660                 665                 670

Lys His Gln Ser Ser Ile Gly Phe Ser Val His Gln Asn Leu Lys Glu
        675                 680                 685

Ser Leu Asp Arg Cys Leu Leu Gly Leu Ser Glu Gln Leu Leu Asn Asn
    690                 695                 700

Tyr Ser Ser Glu Ile Thr Asn Ser Glu Thr Leu Val Arg Cys Ser Arg
705                 710                 715                 720

Leu Leu Val Gly Val Leu Gly Cys Tyr Cys Tyr Met Gly Val Ile Ala
                725                 730                 735

Glu Glu Glu Ala Tyr Lys Ser Glu Leu Phe Gln Lys Ala Asn Ser Leu
            740                 745                 750

Met Gln Cys Ala Gly Glu Ser Ile Thr Leu Phe Lys Asn Lys Thr Asn
        755                 760                 765

Glu Glu Phe Arg Ile Gly Ser Leu Arg Asn Met Met Gln Leu Cys Thr
    770                 775                 780

Arg Cys Leu Ser Asn Cys Thr Lys Lys Ser Pro Asn Lys Ile Ala Ser
785                 790                 795                 800

Gly Phe Phe Leu Arg Leu Leu Thr Ser Lys Leu Met Asn Asp Ile Ala
                805                 810                 815

Asp Ile Cys Lys Ser Leu Ala Ser Phe Ile Lys Lys Pro Phe Asp Arg
            820                 825                 830

Gly Glu Val Glu Ser Met Glu Asp Asp Thr Asn Gly Asn Leu Met Glu
        835                 840                 845

Val Glu Asp Gln Ser Ser Met Asn Leu Phe Asn Asp Tyr Pro Asp Ser
    850                 855                 860

Ser Val Ser Asp Ala Asn Glu Pro Gly Glu Ser Gln Ser Thr Ile Gly
865                 870                 875                 880

Ala Ile Asn Pro Leu Ala Glu Glu Tyr Leu Ser Lys Gln Asp Leu Leu
                885                 890                 895

Phe Leu Asp Met Leu Lys Phe Leu Cys Leu Cys Val Thr Thr Ala Gln
            900                 905                 910

Thr Asn Thr Val Ser Phe Arg Ala Ala Asp Ile Arg Arg Lys Leu Leu
        915                 920                 925

Met Leu Ile Asp Ser Ser Thr Leu Glu Pro Thr Lys Ser Leu His Leu
    930                 935                 940

His Met Tyr Leu Met Leu Leu Lys Glu Leu Pro Gly Glu Glu Tyr Pro
945                 950                 955                 960

Leu Pro Met Glu Asp Val Leu Glu Leu Leu Lys Pro Leu Ser Asn Val
                965                 970                 975

Cys Ser Leu Tyr Arg Arg Asp Gln Asp Val Cys Lys Thr Ile Leu Asn
            980                 985                 990

His Val Leu His Val Val Lys Asn  Leu Gly Gln Ser Asn  Met Asp Ser
        995                 1000                1005

Glu Asn  Thr Arg Asp Ala Gln  Gly Gln Phe Leu Thr  Val Ile Gly
    1010                1015                1020

Ala Phe  Trp His Leu Thr Lys  Glu Arg Lys Tyr Ile  Phe Ser Val
    1025                1030                1035

Arg Met  Ala Leu Val Asn Cys  Leu Lys Thr Leu Leu  Glu Ala Asp
    1040                1045                1050

Pro Tyr Ser Lys Trp Ala Ile Leu Asn Val Met Gly Lys Asp Phe
    1055                1060                1065

Pro Val Asn Glu Val Phe Thr Gln Phe Leu Ala Asp Asn His His
    1070                1075                1080

Gln Val Arg Met Leu Ala Ala Glu Ser Ile Asn Arg Leu Phe Gln
    1085                1090                1095

Asp Thr Lys Gly Asp Ser Ser Arg Leu Leu Lys Ala Leu Pro Leu
    1100                1105                1110

Lys Leu Gln Gln Thr Ala Phe Glu Asn Ala Tyr Leu Lys Ala Gln
    1115                1120                1125

Glu Gly Met Arg Glu Met Ser His Ser Ala Glu Asn Pro Glu Thr
    1130                1135                1140

Leu Asp Glu Ile Tyr Asn Arg Lys Ser Val Leu Leu Thr Leu Ile
    1145                1150                1155

Ala Val Val Leu Ser Cys Ser Pro Ile Cys Glu Lys Gln Ala Leu
    1160                1165                1170

Phe Ala Leu Cys Lys Ser Val Lys Glu Asn Gly Leu Glu Pro His
    1175                1180                1185

Leu Val Lys Lys Val Leu Glu Lys Val Ser Glu Thr Phe Gly Tyr
    1190                1195                1200

Arg Arg Leu Glu Asp Phe Met Ala Ser His Leu Asp Tyr Leu Val
    1205                1210                1215

Leu Glu Trp Leu Asn Leu Gln Asp Thr Glu Tyr Asn Leu Ser Ser
    1220                1225                1230

Phe Pro Phe Ile Leu Leu Asn Tyr Thr Asn Ile Glu Asp Phe Tyr
    1235                1240                1245

Arg Ser Cys Tyr Lys Val Leu Ile Pro His Leu Val Ile Arg Ser
    1250                1255                1260

His Phe Asp Glu Val Lys Ser Ile Ala Asn Gln Ile Gln Glu Asp
    1265                1270                1275

Trp Lys Ser Leu Leu Thr Asp Cys Phe Pro Lys Ile Leu Val Asn
    1280                1285                1290

Ile Leu Pro Tyr Phe Ala Tyr Glu Gly Thr Arg Asp Ser Gly Met
    1295                1300                1305

Ala Gln Gln Arg Glu Thr Ala Thr Lys Val Tyr Asp Met Leu Lys
    1310                1315                1320

Ser Glu Asn Leu Leu Gly Lys Gln Ile Asp His Leu Phe Ile Ser
    1325                1330                1335

Asn Leu Pro Glu Ile Val Val Glu Leu Leu Met Thr Leu His Glu
    1340                1345                1350

Pro Ala Asn Ser Ser Ala Ser Gln Ser Thr Asp Leu Cys Asp Phe
    1355                1360                1365

Ser Gly Asp Leu Asp Pro Ala Pro Asn Pro Pro His Phe Pro Ser
    1370                1375                1380

His Val Ile Lys Ala Thr Phe Ala Tyr Ile Ser Asn Cys His Lys
    1385                1390                1395

Thr Lys Leu Lys Ser Ile Leu Glu Ile Leu Ser Lys Ser Pro Asp
    1400                1405                1410

Ser Tyr Gln Lys Ile Leu Leu Ala Ile Cys Glu Gln Ala Ala Glu
    1415                1420                1425

Thr Asn Asn Val Tyr Lys Lys His Arg Ile Leu Lys Ile Tyr His
    1430                1435                1440

Leu Phe Val Ser Leu Leu Leu Lys Asp Ile Lys Ser Gly Leu Gly
    1445                1450                1455

Gly Ala Trp Ala Phe Val Leu Arg Asp Val Ile Tyr Thr Leu Ile
    1460                1465                1470

His Tyr Ile Asn Gln Arg Pro Ser Cys Ile Met Asp Val Ser Leu
    1475                1480                1485

Arg Ser Phe Ser Leu Cys Cys Asp Leu Leu Ser Gln Val Cys Gln
    1490                1495                1500

Thr Ala Val Thr Tyr Cys Lys Asp Ala Leu Glu Asn His Leu His
    1505                1510                1515

Val Ile Val Gly Thr Leu Ile Pro Leu Val Tyr Glu Gln Val Glu
    1520                1525                1530

Val Gln Lys Gln Val Leu Asp Leu Leu Lys Tyr Leu Val Ile Asp
    1535                1540                1545

Asn Lys Asp Asn Glu Asn Leu Tyr Ile Thr Ile Lys Leu Leu Asp
    1550                1555                1560

Pro Phe Pro Asp His Val Val Phe Lys Asp Leu Arg Ile Thr Gln

1565                    1570                    1575

Gln Lys  Ile Lys Tyr Ser Arg  Gly Pro Phe Ser Leu  Leu Glu Glu
    1580                    1585                    1590

Ile Asn  His Phe Leu Ser Val  Ser Val Tyr Asp Ala  Leu Pro Leu
    1595                    1600                    1605

Thr Arg  Leu Glu Gly Leu Lys  Asp Leu Arg Arg Gln  Leu Glu Leu
    1610                    1615                    1620

His Lys  Asp Gln Met Val Asp  Ile Met Arg Ala Ser  Gln Asp Asn
    1625                    1630                    1635

Pro Gln  Asp Gly Ile Met Val  Lys Leu Val Val Asn  Leu Leu Gln
    1640                    1645                    1650

Leu Ser  Lys Met Ala Ile Asn  His Thr Gly Glu Lys  Glu Val Leu
    1655                    1660                    1665

Glu Ala  Val Gly Ser Cys Leu  Gly Glu Val Gly Pro  Ile Asp Phe
    1670                    1675                    1680

Ser Thr  Ile Ala Ile Gln His  Ser Lys Asp Ala Ser  Tyr Thr Lys
    1685                    1690                    1695

Ala Leu  Lys Leu Phe Glu Asp  Lys Glu Leu Gln Trp  Thr Phe Ile
    1700                    1705                    1710

Met Leu  Thr Tyr Leu Asn Asn  Thr Leu Val Glu Asp  Cys Val Lys
    1715                    1720                    1725

Val Arg  Ser Ala Ala Val Thr  Cys Leu Lys Asn Ile  Leu Ala Thr
    1730                    1735                    1740

Lys Thr  Gly His Ser Phe Trp  Glu Ile Tyr Lys Met  Thr Thr Asp
    1745                    1750                    1755

Pro Met  Leu Ala Tyr Leu Gln  Pro Phe Arg Thr Ser  Arg Lys Lys
    1760                    1765                    1770

Phe Leu  Glu Val Pro Arg Phe  Asp Lys Glu Asn Pro  Phe Glu Gly
    1775                    1780                    1785

Leu Asp  Asp Ile Asn Leu Trp  Ile Pro Leu Ser Glu  Asn His Asp
    1790                    1795                    1800

Ile Trp  Ile Lys Thr Leu Thr  Cys Ala Phe Leu Asp  Ser Gly Gly
    1805                    1810                    1815

Thr Lys  Cys Glu Ile Leu Gln  Leu Leu Lys Pro Met  Cys Glu Val
    1820                    1825                    1830

Lys Thr Asp Phe Cys Gln Thr Val Leu Pro Tyr Leu Ile His Asp
    1835                1840                1845

Ile Leu Leu Gln Asp Thr Asn Glu Ser Trp Arg Asn Leu Leu Ser
    1850                1855                1860

Thr His Val Gln Gly Phe Phe Thr Ser Cys Leu Arg His Phe Ser
    1865                1870                1875

Gln Thr Ser Arg Ser Thr Thr Pro Ala Asn Leu Asp Ser Glu Ser
    1880                1885                1890

Glu His Phe Phe Arg Cys Cys Leu Asp Lys Lys Ser Gln Arg Thr
    1895                1900                1905

Met Leu Ala Val Val Asp Tyr Met Arg Arg Gln Lys Arg Pro Ser
    1910                1915                1920

Ser Gly Thr Ile Phe Asn Asp Ala Phe Trp Leu Asp Leu Asn Tyr
    1925                1930                1935

Leu Glu Val Ala Lys Val Ala Gln Ser Cys Ala Ala His Phe Thr
    1940                1945                1950

Ala Leu Leu Tyr Ala Glu Ile Tyr Ala Asp Lys Lys Ser Met Asp
    1955                1960                1965

Asp Gln Glu Lys Arg Ser Leu Ala Phe Glu Glu Gly Ser Gln Ser
    1970                1975                1980

Thr Thr Ile Ser Ser Leu Ser Glu Lys Ser Lys Glu Glu Thr Gly
    1985                1990                1995

Ile Ser Leu Gln Asp Leu Leu Leu Glu Ile Tyr Arg Ser Ile Gly
    2000                2005                2010

Glu Pro Asp Ser Leu Tyr Gly Cys Gly Gly Gly Lys Met Leu Gln
    2015                2020                2025

Pro Ile Thr Arg Leu Arg Thr Tyr Glu His Glu Ala Met Trp Gly
    2030                2035                2040

Lys Ala Leu Val Thr Tyr Asp Leu Glu Thr Ala Ile Pro Ser Ser
    2045                2050                2055

Thr Arg Gln Ala Gly Ile Ile Gln Ala Leu Gln Asn Leu Gly Leu
    2060                2065                2070

Cys His Ile Leu Ser Val Tyr Leu Lys Gly Leu Asp Tyr Glu Asn
    2075                2080                2085

Lys Asp Trp Cys Pro Glu Leu Glu Glu Leu His Tyr Gln Ala Ala
    2090                2095                2100

Trp Arg Asn Met Gln Trp Asp His Cys Thr Ser Val Ser Lys Glu
    2105                2110                2115

Val Glu Gly Thr Ser Tyr His Glu Ser Leu Tyr Asn Ala Leu Gln
    2120                2125                2130

Ser Leu Arg Asp Arg Glu Phe Ser Thr Phe Tyr Glu Ser Leu Lys
    2135                2140                2145

Tyr Ala Arg Val Lys Glu Val Glu Glu Met Cys Lys Arg Ser Leu
    2150                2155                2160

Glu Ser Val Tyr Ser Leu Tyr Pro Thr Leu Ser Arg Leu Gln Ala
    2165                2170                2175

Ile Gly Glu Leu Glu Ser Ile Gly Glu Leu Phe Ser Arg Ser Val
    2180                2185                2190

Thr His Arg Gln Leu Ser Glu Val Tyr Ile Lys Trp Gln Lys His
    2195                2200                2205

Ser Gln Leu Leu Lys Asp Ser Asp Phe Ser Phe Gln Glu Pro Ile
    2210                2215                2220

Met Ala Leu Arg Thr Val Ile Leu Glu Ile Leu Met Glu Lys Glu
    2225                2230                2235

Met Asp Asn Ser Gln Arg Glu Cys Ile Lys Asp Ile Leu Thr Lys
    2240                2245                2250

His Leu Val Glu Leu Ser Ile Leu Ala Arg Thr Phe Lys Asn Thr
    2255                2260                2265

Gln Leu Pro Glu Arg Ala Ile Phe Gln Ile Lys Gln Tyr Asn Ser
    2270                2275                2280

Val Ser Cys Gly Val Ser Glu Trp Gln Leu Glu Glu Ala Gln Val
    2285                2290                2295

Phe Trp Ala Lys Lys Glu Gln Ser Leu Ala Leu Ser Ile Leu Lys
    2300                2305                2310

Gln Met Ile Lys Lys Leu Asp Ala Ser Cys Ala Ala Asn Asn Pro
    2315                2320                2325

Ser Leu Lys Leu Thr Tyr Thr Glu Cys Leu Arg Val Cys Gly Asn
    2330                2335                2340

Trp Leu Ala Glu Thr Cys Leu Glu Asn Pro Ala Val Ile Met Gln
    2345                2350                2355

Thr Tyr Leu Glu Lys Ala Val Glu Val Ala Gly Asn Tyr Asp Gly
    2360                2365                2370

Glu Ser Ser Asp Glu Leu Arg Asn Gly Lys Met Lys Ala Phe Leu
    2375                2380                2385

Ser Leu Ala Arg Phe Ser Asp Thr Gln Tyr Gln Arg Ile Glu Asn
    2390                2395                2400

Tyr Met Lys Ser Ser Glu Phe Glu Asn Lys Gln Ala Leu Leu Lys
    2405                2410                2415

Arg Ala Lys Glu Glu Val Gly Leu Leu Arg Glu His Lys Ile Gln
    2420                2425                2430

Thr Asn Arg Tyr Thr Val Lys Val Gln Arg Glu Leu Glu Leu Asp
    2435                2440                2445

Glu Leu Ala Leu Arg Ala Leu Lys Glu Asp Arg Lys Arg Phe Leu
    2450                2455                2460

Cys Lys Ala Val Glu Asn Tyr Ile Asn Cys Leu Leu Ser Gly Glu
    2465                2470                2475

Glu His Asp Met Trp Val Phe Arg Leu Cys Ser Leu Trp Leu Glu
    2480                2485                2490

Asn Ser Gly Val Ser Glu Val Asn Gly Met Met Lys Arg Asp Gly
    2495                2500                2505

Met Lys Ile Pro Thr Tyr Lys Phe Leu Pro Leu Met Tyr Gln Leu
    2510                2515                2520

Ala Ala Arg Met Gly Thr Lys Met Met Gly Gly Leu Gly Phe His
    2525                2530                2535

Glu Val Leu Asn Asn Leu Ile Ser Arg Ile Ser Met Asp His Pro
    2540                2545                2550

His His Thr Leu Phe Ile Ile Leu Ala Leu Ala Asn Ala Asn Arg
    2555                2560                2565

Asp Glu Phe Leu Thr Lys Pro Glu Val Ala Arg Arg Ser Arg Ile
    2570                2575                2580

Thr Lys Asn Val Pro Lys Gln Ser Ser Gln Leu Asp Glu Asp Arg
    2585                2590                2595

Thr Glu Ala Ala Asn Arg Ile Ile Cys Thr Ile Arg Ser Arg Arg

2600                    2605                    2610

Pro Gln Met Val Arg Ser Val Glu Ala Leu Cys Asp Ala Tyr Ile
    2615                    2620                    2625

Ile Leu Ala Asn Leu Asp Ala Thr Gln Trp Lys Thr Gln Arg Lys
    2630                    2635                    2640

Gly Ile Asn Ile Pro Ala Asp Gln Pro Ile Thr Lys Leu Lys Asn
    2645                    2650                    2655

Leu Glu Asp Val Val Val Pro Thr Met Glu Ile Lys Val Asp His
    2660                    2665                    2670

Thr Gly Glu Tyr Gly Asn Leu Val Thr Ile Gln Ser Phe Lys Ala
    2675                    2680                    2685

Glu Phe Arg Leu Ala Gly Gly Val Asn Leu Pro Lys Ile Ile Asp
    2690                    2695                    2700

Cys Val Gly Ser Asp Gly Lys Glu Arg Arg Gln Leu Val Lys Gly
    2705                    2710                    2715

Arg Asp Asp Leu Arg Gln Asp Ala Val Met Gln Gln Val Phe Gln
    2720                    2725                    2730

Met Cys Asn Thr Leu Leu Gln Arg Asn Thr Glu Thr Arg Lys Arg
    2735                    2740                    2745

Lys Leu Thr Ile Cys Thr Tyr Lys Val Val Pro Leu Ser Gln Arg
    2750                    2755                    2760

Ser Gly Val Leu Glu Trp Cys Thr Gly Thr Val Pro Ile Gly Glu
    2765                    2770                    2775

Phe Leu Val Asn Asn Glu Asp Gly Ala His Lys Arg Tyr Arg Pro
    2780                    2785                    2790

Asn Asp Phe Ser Ala Phe Gln Cys Gln Lys Lys Met Met Glu Val
    2795                    2800                    2805

Gln Lys Lys Ser Phe Glu Glu Lys Tyr Glu Val Phe Met Asp Val
    2810                    2815                    2820

Cys Gln Asn Phe Gln Pro Val Phe Arg Tyr Phe Cys Met Glu Lys
    2825                    2830                    2835

Phe Leu Asp Pro Ala Ile Trp Phe Glu Lys Arg Leu Ala Tyr Thr
    2840                    2845                    2850

Arg Ser Val Ala Thr Ser Ser Ile Val Gly Tyr Ile Leu Gly Leu
    2855                    2860                    2865

Gly Asp Arg His Val Gln Asn Ile Leu Ile Asn Glu Gln Ser Ala
    2870                2875                2880

Glu Leu Val His Ile Asp Leu Gly Val Ala Phe Glu Gln Gly Lys
    2885                2890                2895

Ile Leu Pro Thr Pro Glu Thr Val Pro Phe Arg Leu Thr Arg Asp
    2900                2905                2910

Ile Val Asp Gly Met Gly Ile Thr Gly Val Glu Gly Val Phe Arg
    2915                2920                2925

Arg Cys Cys Glu Lys Thr Met Glu Val Met Arg Asn Ser Gln Glu
    2930                2935                2940

Thr Leu Leu Thr Ile Val Glu Val Leu Leu Tyr Asp Pro Leu Phe
    2945                2950                2955

Asp Trp Thr Met Asn Pro Leu Lys Ala Leu Tyr Leu Gln Gln Arg
    2960                2965                2970

Pro Glu Asp Glu Thr Glu Leu His Pro Thr Leu Asn Ala Asp Asp
    2975                2980                2985

Gln Glu Cys Lys Arg Asn Leu Ser Asp Ile Asp Gln Ser Phe Asn
    2990                2995                3000

Lys Val Ala Glu Arg Val Leu Met Arg Leu Gln Glu Lys Leu Lys
    3005                3010                3015

Gly Val Glu Glu Gly Thr Val Leu Ser Val Gly Gly Gln Val Asn
    3020                3025                3030

Leu Leu Ile Gln Gln Ala Ile Asp Pro Lys Asn Leu Ser Arg Leu
    3035                3040                3045

Phe Pro Gly Trp Lys Ala Trp Val
    3050                3055

<210>  5
<211>  2549
<212>  PRT
<213>  Homo sapiens

<400>  5

Met Leu Gly Thr Gly Pro Ala Ala Ala Thr Thr Ala Ala Thr Thr Ser
1               5                   10                  15

Ser Asn Val Ser Val Leu Gln Gln Phe Ala Ser Gly Leu Lys Ser Arg
            20                  25                  30

Asn Glu Glu Thr Arg Ala Lys Ala Ala Lys Glu Leu Gln His Tyr Val
        35                  40                  45

Thr Met Glu Leu Arg Glu Met Ser Gln Glu Glu Ser Thr Arg Phe Tyr
    50                  55                  60

Asp Gln Leu Asn His His Ile Phe Glu Leu Val Ser Ser Ser Asp Ala
65                  70                  75                  80

Asn Glu Arg Lys Gly Gly Ile Leu Ala Ile Ala Ser Leu Ile Gly Val
                85                  90                  95

Glu Gly Gly Asn Ala Thr Arg Ile Gly Arg Phe Ala Asn Tyr Leu Arg
            100                 105                 110

Asn Leu Leu Pro Ser Asn Asp Pro Val Val Met Glu Met Ala Ser Lys
        115                 120                 125

Ala Ile Gly Arg Leu Ala Met Ala Gly Asp Thr Phe Thr Ala Glu Tyr
    130                 135                 140

Val Glu Phe Glu Val Lys Arg Ala Leu Glu Trp Leu Gly Ala Asp Arg
145                 150                 155                 160

Asn Glu Gly Arg Arg His Ala Ala Val Leu Val Leu Arg Glu Leu Ala
                165                 170                 175

Ile Ser Val Pro Thr Phe Phe Phe Gln Gln Val Gln Pro Phe Phe Asp
            180                 185                 190

Asn Ile Phe Val Ala Val Trp Asp Pro Lys Gln Ala Ile Arg Glu Gly
        195                 200                 205

Ala Val Ala Ala Leu Arg Ala Cys Leu Ile Leu Thr Thr Gln Arg Glu
    210                 215                 220

Pro Lys Glu Met Gln Lys Pro Gln Trp Tyr Arg His Thr Phe Glu Glu
225                 230                 235                 240

Ala Glu Lys Gly Phe Asp Glu Thr Leu Ala Lys Glu Lys Gly Met Asn
                245                 250                 255

Arg Asp Asp Arg Ile His Gly Ala Leu Leu Ile Leu Asn Glu Leu Val
            260                 265                 270

Arg Ile Ser Ser Met Glu Gly Glu Arg Leu Arg Glu Glu Met Glu Glu
        275                 280                 285

Ile Thr Gln Gln Gln Leu Val His Asp Lys Tyr Cys Lys Asp Leu Met
    290                 295                 300

Gly Phe Gly Thr Lys Pro Arg His Ile Thr Pro Phe Thr Ser Phe Gln

305                 310                 315                 320

Ala Val Gln Pro Gln Gln Ser Asn Ala Leu Val Gly Leu Leu Gly Tyr
                325                 330                 335

Ser Ser His Gln Gly Leu Met Gly Phe Gly Thr Ser Pro Ser Pro Ala
            340                 345                 350

Lys Ser Thr Leu Val Glu Ser Arg Cys Cys Arg Asp Leu Met Glu Glu
        355                 360                 365

Lys Phe Asp Gln Val Cys Gln Trp Val Leu Lys Cys Arg Asn Ser Lys
    370                 375                 380

Asn Ser Leu Ile Gln Met Thr Ile Leu Asn Leu Leu Pro Arg Leu Ala
385                 390                 395                 400

Ala Phe Arg Pro Ser Ala Phe Thr Asp Thr Gln Tyr Leu Gln Asp Thr
                405                 410                 415

Met Asn His Val Leu Ser Cys Val Lys Lys Glu Lys Glu Arg Thr Ala
            420                 425                 430

Ala Phe Gln Ala Leu Gly Leu Leu Ser Val Ala Val Arg Ser Glu Phe
        435                 440                 445

Lys Val Tyr Leu Pro Arg Val Leu Asp Ile Ile Arg Ala Ala Leu Pro
    450                 455                 460

Pro Lys Asp Phe Ala His Lys Arg Gln Lys Ala Met Gln Val Asp Ala
465                 470                 475                 480

Thr Val Phe Thr Cys Ile Ser Met Leu Ala Arg Ala Met Gly Pro Gly
                485                 490                 495

Ile Gln Gln Asp Ile Lys Glu Leu Leu Glu Pro Met Leu Ala Val Gly
            500                 505                 510

Leu Ser Pro Ala Leu Thr Ala Val Leu Tyr Asp Leu Ser Arg Gln Ile
        515                 520                 525

Pro Gln Leu Lys Lys Asp Ile Gln Asp Gly Leu Leu Lys Met Leu Ser
    530                 535                 540

Leu Val Leu Met His Lys Pro Leu Arg His Pro Gly Met Pro Lys Gly
545                 550                 555                 560

Leu Ala His Gln Leu Ala Ser Pro Gly Leu Thr Thr Leu Pro Glu Ala
                565                 570                 575

Ser Asp Val Gly Ser Ile Thr Leu Ala Leu Arg Thr Leu Gly Ser Phe
            580                 585                 590

Glu Phe Glu Gly His Ser Leu Thr Gln Phe Val Arg His Cys Ala Asp
        595                 600                 605

His Phe Leu Asn Ser Glu His Lys Glu Ile Arg Met Glu Ala Ala Arg
    610                 615                 620

Thr Cys Ser Arg Leu Leu Thr Pro Ser Ile His Leu Ile Ser Gly His
625                 630                 635                 640

Ala His Val Val Ser Gln Thr Ala Val Gln Val Val Ala Asp Val Leu
                645                 650                 655

Ser Lys Leu Leu Val Val Gly Ile Thr Asp Pro Asp Pro Asp Ile Arg
            660                 665                 670

Tyr Cys Val Leu Ala Ser Leu Asp Glu Arg Phe Asp Ala His Leu Ala
        675                 680                 685

Gln Ala Glu Asn Leu Gln Ala Leu Phe Val Ala Leu Asn Asp Gln Val
    690                 695                 700

Phe Glu Ile Arg Glu Leu Ala Ile Cys Thr Val Gly Arg Leu Ser Ser
705                 710                 715                 720

Met Asn Pro Ala Phe Val Met Pro Phe Leu Arg Lys Met Leu Ile Gln
                725                 730                 735

Ile Leu Thr Glu Leu Glu His Ser Gly Ile Gly Arg Ile Lys Glu Gln
            740                 745                 750

Ser Ala Arg Met Leu Gly His Leu Val Ser Asn Ala Pro Arg Leu Ile
        755                 760                 765

Arg Pro Tyr Met Glu Pro Ile Leu Lys Ala Leu Ile Leu Lys Leu Lys
    770                 775                 780

Asp Pro Asp Pro Asp Pro Asn Pro Gly Val Ile Asn Asn Val Leu Ala
785                 790                 795                 800

Thr Ile Gly Glu Leu Ala Gln Val Ser Gly Leu Glu Met Arg Lys Trp
                805                 810                 815

Val Asp Glu Leu Phe Ile Ile Ile Met Asp Met Leu Gln Asp Ser Ser
            820                 825                 830

Leu Leu Ala Lys Arg Gln Val Ala Leu Trp Thr Leu Gly Gln Leu Val
        835                 840                 845

Ala Ser Thr Gly Tyr Val Val Glu Pro Tyr Arg Lys Tyr Pro Thr Leu
    850                 855                 860

Leu Glu Val Leu Leu Asn Phe Leu Lys Thr Glu Gln Asn Gln Gly Thr
865                 870                 875                 880

Arg Arg Glu Ala Ile Arg Val Leu Gly Leu Leu Gly Ala Leu Asp Pro
                885                 890                 895

Tyr Lys His Lys Val Asn Ile Gly Met Ile Asp Gln Ser Arg Asp Ala
            900                 905                 910

Ser Ala Val Ser Leu Ser Glu Ser Lys Ser Ser Gln Asp Ser Ser Asp
        915                 920                 925

Tyr Ser Thr Ser Glu Met Leu Val Asn Met Gly Asn Leu Pro Leu Asp
    930                 935                 940

Glu Phe Tyr Pro Ala Val Ser Met Val Ala Leu Met Arg Ile Phe Arg
945                 950                 955                 960

Asp Gln Ser Leu Ser His His His Thr Met Val Val Gln Ala Ile Thr
                965                 970                 975

Phe Ile Phe Lys Ser Leu Gly Leu Lys Cys Val Gln Phe Leu Pro Gln
            980                 985                 990

Val Met Pro Thr Phe Leu Asn Val  Ile Arg Val Cys Asp  Gly Ala Ile
        995                 1000                 1005

Arg Glu  Phe Leu Phe Gln Gln  Leu Gly Met Leu Val  Ser Phe Val
    1010                 1015                 1020

Lys Ser  His Ile Arg Pro Tyr  Met Asp Glu Ile Val  Thr Leu Met
    1025                 1030                 1035

Arg Glu  Phe Trp Val Met Asn  Thr Ser Ile Gln Ser  Thr Ile Ile
    1040                 1045                 1050

Leu Leu  Ile Glu Gln Ile Val  Val Ala Leu Gly Gly  Glu Phe Lys
    1055                 1060                 1065

Leu Tyr  Leu Pro Gln Leu Ile  Pro His Met Leu Arg  Val Phe Met
    1070                 1075                 1080

His Asp  Asn Ser Pro Gly Arg  Ile Val Ser Ile Lys  Leu Leu Ala
    1085                 1090                 1095

Ala Ile  Gln Leu Phe Gly Ala  Asn Leu Asp Asp Tyr  Leu His Leu
    1100                 1105                 1110

Leu Leu  Pro Pro Ile Val Lys  Leu Phe Asp Ala Pro  Glu Ala Pro
    1115                 1120                 1125

Leu Pro Ser Arg Lys Ala Ala Leu Glu Thr Val Asp Arg Leu Thr
    1130                1135                1140

Glu Ser Leu Asp Phe Thr Asp Tyr Ala Ser Arg Ile Ile His Pro
    1145                1150                1155

Ile Val Arg Thr Leu Asp Gln Ser Pro Glu Leu Arg Ser Thr Ala
    1160                1165                1170

Met Asp Thr Leu Ser Ser Leu Val Phe Gln Leu Gly Lys Lys Tyr
    1175                1180                1185

Gln Ile Phe Ile Pro Met Val Asn Lys Val Leu Val Arg His Arg
    1190                1195                1200

Ile Asn His Gln Arg Tyr Asp Val Leu Ile Cys Arg Ile Val Lys
    1205                1210                1215

Gly Tyr Thr Leu Ala Asp Glu Glu Glu Asp Pro Leu Ile Tyr Gln
    1220                1225                1230

His Arg Met Leu Arg Ser Gly Gln Gly Asp Ala Leu Ala Ser Gly
    1235                1240                1245

Pro Val Glu Thr Gly Pro Met Lys Lys Leu His Val Ser Thr Ile
    1250                1255                1260

Asn Leu Gln Lys Ala Trp Gly Ala Ala Arg Arg Val Ser Lys Asp
    1265                1270                1275

Asp Trp Leu Glu Trp Leu Arg Arg Leu Ser Leu Glu Leu Leu Lys
    1280                1285                1290

Asp Ser Ser Ser Pro Ser Leu Arg Ser Cys Trp Ala Leu Ala Gln
    1295                1300                1305

Ala Tyr Asn Pro Met Ala Arg Asp Leu Phe Asn Ala Ala Phe Val
    1310                1315                1320

Ser Cys Trp Ser Glu Leu Asn Glu Asp Gln Gln Asp Glu Leu Ile
    1325                1330                1335

Arg Ser Ile Glu Leu Ala Leu Thr Ser Gln Asp Ile Ala Glu Val
    1340                1345                1350

Thr Gln Thr Leu Leu Asn Leu Ala Glu Phe Met Glu His Ser Asp
    1355                1360                1365

Lys Gly Pro Leu Pro Leu Arg Asp Asp Asn Gly Ile Val Leu Leu
    1370                1375                1380

Gly Glu Arg Ala Ala Lys Cys Arg Ala Tyr Ala Lys Ala Leu His

1385                    1390                    1395

Tyr Lys Glu Leu Glu Phe Gln Lys Gly Pro Thr Pro Ala Ile Leu
    1400                    1405                    1410

Glu Ser Leu Ile Ser Ile Asn Asn Lys Leu Gln Gln Pro Glu Ala
    1415                    1420                    1425

Ala Ala Gly Val Leu Glu Tyr Ala Met Lys His Phe Gly Glu Leu
    1430                    1435                    1440

Glu Ile Gln Ala Thr Trp Tyr Glu Lys Leu His Glu Trp Glu Asp
    1445                    1450                    1455

Ala Leu Val Ala Tyr Asp Lys Lys Met Asp Thr Asn Lys Asp Asp
    1460                    1465                    1470

Pro Glu Leu Met Leu Gly Arg Met Arg Cys Leu Glu Ala Leu Gly
    1475                    1480                    1485

Glu Trp Gly Gln Leu His Gln Gln Cys Cys Glu Lys Trp Thr Leu
    1490                    1495                    1500

Val Asn Asp Glu Thr Gln Ala Lys Met Ala Arg Met Ala Ala Ala
    1505                    1510                    1515

Ala Ala Trp Gly Leu Gly Gln Trp Asp Ser Met Glu Glu Tyr Thr
    1520                    1525                    1530

Cys Met Ile Pro Arg Asp Thr His Asp Gly Ala Phe Tyr Arg Ala
    1535                    1540                    1545

Val Leu Ala Leu His Gln Asp Leu Phe Ser Leu Ala Gln Gln Cys
    1550                    1555                    1560

Ile Asp Lys Ala Arg Asp Leu Leu Asp Ala Glu Leu Thr Ala Met
    1565                    1570                    1575

Ala Gly Glu Ser Tyr Ser Arg Ala Tyr Gly Ala Met Val Ser Cys
    1580                    1585                    1590

His Met Leu Ser Glu Leu Glu Glu Val Ile Gln Tyr Lys Leu Val
    1595                    1600                    1605

Pro Glu Arg Arg Glu Ile Ile Arg Gln Ile Trp Trp Glu Arg Leu
    1610                    1615                    1620

Gln Gly Cys Gln Arg Ile Val Glu Asp Trp Gln Lys Ile Leu Met
    1625                    1630                    1635

Val Arg Ser Leu Val Val Ser Pro His Glu Asp Met Arg Thr Trp
    1640                    1645                    1650

Leu Lys Tyr Ala Ser Leu Cys Gly Lys Ser Gly Arg Leu Ala Leu
    1655                1660                1665

Ala His Lys Thr Leu Val Leu Leu Leu Gly Val Asp Pro Ser Arg
    1670                1675                1680

Gln Leu Asp His Pro Leu Pro Thr Val His Pro Gln Val Thr Tyr
    1685                1690                1695

Ala Tyr Met Lys Asn Met Trp Lys Ser Ala Arg Lys Ile Asp Ala
    1700                1705                1710

Phe Gln His Met Gln His Phe Val Gln Thr Met Gln Gln Gln Ala
    1715                1720                1725

Gln His Ala Ile Ala Thr Glu Asp Gln Gln His Lys Gln Glu Leu
    1730                1735                1740

His Lys Leu Met Ala Arg Cys Phe Leu Lys Leu Gly Glu Trp Gln
    1745                1750                1755

Leu Asn Leu Gln Gly Ile Asn Glu Ser Thr Ile Pro Lys Val Leu
    1760                1765                1770

Gln Tyr Tyr Ser Ala Ala Thr Glu His Asp Arg Ser Trp Tyr Lys
    1775                1780                1785

Ala Trp His Ala Trp Ala Val Met Asn Phe Glu Ala Val Leu His
    1790                1795                1800

Tyr Lys His Gln Asn Gln Ala Arg Asp Glu Lys Lys Lys Leu Arg
    1805                1810                1815

His Ala Ser Gly Ala Asn Ile Thr Asn Ala Thr Thr Ala Ala Thr
    1820                1825                1830

Thr Ala Ala Thr Ala Thr Thr Thr Ala Ser Thr Glu Gly Ser Asn
    1835                1840                1845

Ser Glu Ser Glu Ala Glu Ser Thr Glu Asn Ser Pro Thr Pro Ser
    1850                1855                1860

Pro Leu Gln Lys Lys Val Thr Glu Asp Leu Ser Lys Thr Leu Leu
    1865                1870                1875

Met Tyr Thr Val Pro Ala Val Gln Gly Phe Phe Arg Ser Ile Ser
    1880                1885                1890

Leu Ser Arg Gly Asn Asn Leu Gln Asp Thr Leu Arg Val Leu Thr
    1895                1900                1905

Leu Trp Phe Asp Tyr Gly His Trp Pro Asp Val Asn Glu Ala Leu
    1910                1915                1920

Val Glu Gly Val Lys Ala Ile Gln Ile Asp Thr Trp Leu Gln Val
    1925                1930                1935

Ile Pro Gln Leu Ile Ala Arg Ile Asp Thr Pro Arg Pro Leu Val
    1940                1945                1950

Gly Arg Leu Ile His Gln Leu Leu Thr Asp Ile Gly Arg Tyr His
    1955                1960                1965

Pro Gln Ala Leu Ile Tyr Pro Leu Thr Val Ala Ser Lys Ser Thr
    1970                1975                1980

Thr Thr Ala Arg His Asn Ala Ala Asn Lys Ile Leu Lys Asn Met
    1985                1990                1995

Cys Glu His Ser Asn Thr Leu Val Gln Gln Ala Met Met Val Ser
    2000                2005                2010

Glu Glu Leu Ile Arg Val Ala Ile Leu Trp His Glu Met Trp His
    2015                2020                2025

Glu Gly Leu Glu Glu Ala Ser Arg Leu Tyr Phe Gly Glu Arg Asn
    2030                2035                2040

Val Lys Gly Met Phe Glu Val Leu Glu Pro Leu His Ala Met Met
    2045                2050                2055

Glu Arg Gly Pro Gln Thr Leu Lys Glu Thr Ser Phe Asn Gln Ala
    2060                2065                2070

Tyr Gly Arg Asp Leu Met Glu Ala Gln Glu Trp Cys Arg Lys Tyr
    2075                2080                2085

Met Lys Ser Gly Asn Val Lys Asp Leu Thr Gln Ala Trp Asp Leu
    2090                2095                2100

Tyr Tyr His Val Phe Arg Arg Ile Ser Lys Gln Leu Pro Gln Leu
    2105                2110                2115

Thr Ser Leu Glu Leu Gln Tyr Val Ser Pro Lys Leu Leu Met Cys
    2120                2125                2130

Arg Asp Leu Glu Leu Ala Val Pro Gly Thr Tyr Asp Pro Asn Gln
    2135                2140                2145

Pro Ile Ile Arg Ile Gln Ser Ile Ala Pro Ser Leu Gln Val Ile
    2150                2155                2160

Thr Ser Lys Gln Arg Pro Arg Lys Leu Thr Leu Met Gly Ser Asn
    2165                2170                2175

Gly His Glu Phe Val Phe Leu Leu Lys Gly His Glu Asp Leu Arg
    2180                2185                2190

Gln Asp Glu Arg Val Met Gln Leu Phe Gly Leu Val Asn Thr Leu
    2195                2200                2205

Leu Ala Asn Asp Pro Thr Ser Leu Arg Lys Asn Leu Ser Ile Gln
    2210                2215                2220

Arg Tyr Ala Val Ile Pro Leu Ser Thr Asn Ser Gly Leu Ile Gly
    2225                2230                2235

Trp Val Pro His Cys Asp Thr Leu His Ala Leu Ile Arg Asp Tyr
    2240                2245                2250

Arg Glu Lys Lys Lys Ile Leu Leu Asn Ile Glu His Arg Ile Met
    2255                2260                2265

Leu Arg Met Ala Pro Asp Tyr Asp His Leu Thr Leu Met Gln Lys
    2270                2275                2280

Val Glu Val Phe Glu His Ala Val Asn Asn Thr Ala Gly Asp Asp
    2285                2290                2295

Leu Ala Lys Leu Leu Trp Leu Lys Ser Pro Ser Ser Glu Val Trp
    2300                2305                2310

Phe Asp Arg Arg Thr Asn Tyr Thr Arg Ser Leu Ala Val Met Ser
    2315                2320                2325

Met Val Gly Tyr Ile Leu Gly Leu Gly Asp Arg His Pro Ser Asn
    2330                2335                2340

Leu Met Leu Asp Arg Leu Ser Gly Lys Ile Leu His Ile Asp Phe
    2345                2350                2355

Gly Asp Cys Phe Glu Val Ala Met Thr Arg Glu Lys Phe Pro Glu
    2360                2365                2370

Lys Ile Pro Phe Arg Leu Thr Arg Met Leu Thr Asn Ala Met Glu
    2375                2380                2385

Val Thr Gly Leu Asp Gly Asn Tyr Arg Ile Thr Cys His Thr Val
    2390                2395                2400

Met Glu Val Leu Arg Glu His Lys Asp Ser Val Met Ala Val Leu
    2405                2410                2415

Glu Ala Phe Val Tyr Asp Pro Leu Leu Asn Trp Arg Leu Met Asp

2420                    2425                    2430

Thr Asn Thr Lys Gly Asn Lys Arg Ser Arg Thr Arg Thr Asp Ser
    2435                    2440                    2445

Tyr Ser Ala Gly Gln Ser Val Glu Ile Leu Asp Gly Val Glu Leu
    2450                    2455                    2460

Gly Glu Pro Ala His Lys Lys Thr Gly Thr Thr Val Pro Glu Ser
    2465                    2470                    2475

Ile His Ser Phe Ile Gly Asp Gly Leu Val Lys Pro Glu Ala Leu
    2480                    2485                    2490

Asn Lys Lys Ala Ile Gln Ile Ile Asn Arg Val Arg Asp Lys Leu
    2495                    2500                    2505

Thr Gly Arg Asp Phe Ser His Asp Asp Thr Leu Asp Val Pro Thr
    2510                    2515                    2520

Gln Val Glu Leu Leu Ile Lys Gln Ala Thr Ser His Glu Asn Leu
    2525                    2530                    2535

Cys Gln Cys Tyr Ile Gly Trp Cys Pro Phe Trp
    2540                    2545

<210>  6
<211>  2644
<212>  PRT
<213>  Homo sapiens

<400>  6
   Met Gly Glu His Gly Leu Glu Leu Ala Ser Met Ile Pro Ala Leu Arg
   1               5                   10                  15
   Glu Leu Gly Ser Ala Thr Pro Glu Glu Tyr Asn Thr Val Val Gln Lys
               20                  25                  30
   Pro Arg Gln Ile Leu Cys Gln Phe Ile Asp Arg Ile Leu Thr Asp Val
           35                  40                  45
   Asn Val Val Ala Val Glu Leu Val Lys Lys Thr Asp Ser Gln Pro Thr
       50                  55                  60
   Ser Val Met Leu Leu Asp Phe Ile Gln His Ile Met Lys Ser Ser Pro
   65                  70                  75                  80
   Leu Met Phe Val Asn Val Ser Gly Ser His Glu Ala Lys Gly Ser Cys
                   85                  90                  95
   Ile Glu Phe Ser Asn Trp Ile Ile Thr Arg Leu Leu Arg Ile Ala Ala
               100                 105                 110
   Thr Pro Ser Cys His Leu Leu His Lys Lys Ile Cys Glu Val Ile Cys
           115                 120                 125
   Ser Leu Leu Phe Leu Phe Lys Ser Lys Ser Pro Ala Ile Phe Gly Val
       130                 135                 140
   Leu Thr Lys Glu Leu Leu Gln Leu Phe Glu Asp Leu Val Tyr Leu His
   145                 150                 155                 160
   Arg Arg Asn Val Met Gly His Ala Val Glu Trp Pro Val Val Met Ser
                   165                 170                 175
   Arg Phe Leu Ser Gln Leu Asp Glu His Met Gly Tyr Leu Gln Ser Ala
               180                 185                 190
   Pro Leu Gln Leu Met Ser Met Gln Asn Leu Glu Phe Ile Glu Val Thr
           195                 200                 205
   Leu Leu Met Val Leu Thr Arg Ile Ile Ala Ile Val Phe Phe Arg Arg
       210                 215                 220
   Gln Glu Leu Leu Leu Trp Gln Ile Gly Cys Val Leu Leu Glu Tyr Gly
   225                 230                 235                 240
   Ser Pro Lys Ile Lys Ser Leu Ala Ile Ser Phe Leu Thr Glu Leu Phe
                   245                 250                 255
   Gln Leu Gly Gly Leu Pro Ala Gln Pro Ala Ser Thr Phe Phe Ser Ser
               260                 265                 270
   Phe Leu Glu Leu Leu Lys His Leu Val Glu Met Asp Thr Asp Gln Leu
           275                 280                 285
   Lys Leu Tyr Glu Glu Pro Leu Ser Lys Leu Ile Lys Thr Leu Phe Pro
       290                 295                 300
   Phe Glu Ala Glu Ala Tyr Arg Asn Ile Glu Pro Val Tyr Leu Asn Met
   305                 310                 315                 320
   Leu Leu Glu Lys Leu Cys Val Met Phe Glu Asp Gly Val Leu Met Arg
                   325                 330                 335
   Leu Lys Ser Asp Leu Leu Lys Ala Ala Leu Cys His Leu Leu Gln Tyr
               340                 345                 350
   Phe Leu Lys Phe Val Pro Ala Gly Tyr Glu Ser Ala Leu Gln Val Arg
           355                 360                 365
   Lys Val Tyr Val Arg Asn Ile Cys Lys Ala Leu Leu Asp Val Leu Gly
       370                 375                 380
   Ile Glu Val Asp Ala Glu Tyr Leu Leu Gly Pro Leu Tyr Ala Ala Leu
   385                 390                 395                 400
   Lys Met Glu Ser Met Glu Ile Ile Glu Glu Ile Gln Cys Gln Thr Gln
                   405                 410                 415
   Gln Glu Asn Leu Ser Ser Asn Ser Asp Gly Ile Ser Pro Lys Arg Arg
               420                 425                 430
   Arg Leu Ser Ser Ser Leu Asn Pro Ser Lys Arg Ala Pro Lys Gln Thr
           435                 440                 445
   Glu Glu Ile Lys His Val Asp Met Asn Gln Lys Ser Ile Leu Trp Ser
       450                 455                 460
   Ala Leu Lys Gln Lys Ala Glu Ser Leu Gln Ile Ser Leu Glu Tyr Ser
   465                 470                 475                 480
   Gly Leu Lys Asn Pro Val Ile Glu Met Leu Glu Gly Ile Ala Val Val
                   485                 490                 495
   Leu Gln Leu Thr Ala Leu Cys Thr Val His Cys Ser His Gln Asn Met
               500                 505                 510
   Asn Cys Arg Thr Phe Lys Asp Cys Gln His Lys Ser Lys Lys Lys Pro
           515                 520                 525
   Ser Val Val Ile Thr Trp Met Ser Leu Asp Phe Tyr Thr Lys Val Leu
       530                 535                 540
   Lys Ser Cys Arg Ser Leu Leu Glu Ser Val Gln Lys Leu Asp Leu Glu
   545                 550                 555                 560
   Ala Thr Ile Asp Lys Val Val Lys Ile Tyr Asp Ala Leu Ile Tyr Met
                   565                 570                 575
   Gln Val Asn Ser Ser Phe Glu Asp His Ile Leu Glu Asp Leu Cys Gly
               580                 585                 590
   Met Leu Ser Leu Pro Trp Ile Tyr Ser His Ser Asp Asp Gly Cys Leu
           595                 600                 605
   Lys Leu Thr Thr Phe Ala Ala Asn Leu Leu Thr Leu Ser Cys Arg Ile
       610                 615                 620
   Ser Asp Ser Tyr Ser Pro Gln Ala Gln Ser Arg Cys Val Phe Leu Leu
   625                 630                 635                 640
   Thr Leu Phe Pro Arg Arg Ile Phe Leu Glu Trp Arg Thr Ala Val Tyr
                   645                 650                 655
   Asn Trp Ala Leu Gln Ser Ser His Glu Val Ile Arg Ala Ser Cys Val
   660 665 670
   Ser Gly Phe Phe Ile Leu Leu Gln Gln Gln Asn Ser Cys Asn Arg Val
   675 680 685
   Pro Lys Ile Leu Ile Asp Lys Val Lys Asp Asp Ser Asp Ile Val Lys
   690 695 700
   Lys Glu Phe Ala Ser Ile Leu Gly Gln Leu Val Cys Thr Leu His Gly
   705 710 715 720
   Met Phe Tyr Leu Thr Ser Ser Leu Thr Glu Pro Phe Ser Glu His Gly
   725 730 735
   His Val Asp Leu Phe Cys Arg Asn Leu Lys Ala Thr Ser Gln His Glu
   740 745 750
   Cys Ser Ser Ser Gln Leu Lys Ala Ser Val Cys Lys Pro Phe Leu Phe
   755 760 765
   Leu Leu Lys Lys Lys Ile Pro Ser Pro Val Lys Leu Ala Phe Ile Asp
   770 775 780
   Asn Leu His His Leu Cys Lys His Leu Asp Phe Arg Glu Asp Glu Thr
   785 790 795 800
   Asp Val Lys Ala Val Leu Gly Thr Leu Leu Asn Leu Met Glu Asp Pro
   805 810 815
   Asp Lys Asp Val Arg Val Ala Phe Ser Gly Asn Ile Lys His Ile Leu
   820 825 830
   Glu Ser Leu Asp Ser Glu Asp Gly Phe Ile Lys Glu Leu Phe Val Leu
   835 840 845
   Arg Met Lys Glu Ala Tyr Thr His Ala Gln Ile Ser Arg Asn Asn Glu
   850 855 860
   Leu Lys Asp Thr Leu Ile Leu Thr Thr Gly Asp Ile Gly Arg Ala Ala
   865 870 875 880
   Lys Gly Asp Leu Val Pro Phe Ala Leu Leu His Leu Leu His Cys Leu
   885 890 895
   Leu Ser Lys Ser Ala Ser Val Ser Gly Ala Ala Tyr Thr Glu Ile Arg
   900 905 910
   Ala Leu Val Ala Ala Lys Ser Val Lys Leu Gln Ser Phe Phe Ser Gln
   915 920 925
   Tyr Lys Lys Pro Ile Cys Gln Phe Leu Val Glu Ser Leu His Ser Ser
   930 935 940
   Gln Met Thr Ala Leu Pro Asn Thr Pro Cys Gln Asn Ala Asp Val Arg
   945                 950                 955                 960
   Lys Gln Asp Val Ala His Gln Arg Glu Met Ala Leu Asn Thr Leu Ser
                   965                 970                 975
   Glu Ile Ala Asn Val Phe Asp Phe Pro Asp Leu Asn Arg Phe Leu Thr
               980                 985                 990
   Arg Thr Leu Gln Val Leu Leu Pro  Asp Leu Ala Ala Lys  Ala Ser Pro
           995                 1000                1005
   Ala Ala  Ser Ala Leu Ile Arg  Thr Leu Gly Lys Gln  Leu Asn Val
       1010                 1015                1020
   Asn Arg  Arg Glu Ile Leu Ile  Asn Asn Phe Lys Tyr  Ile Phe Ser
       1025                 1030                1035
   His Leu  Val Cys Ser Cys Ser  Lys Asp Glu Leu Glu  Arg Ala Leu
       1040                 1045                1050
   His Tyr  Leu Lys Asn Glu Thr  Glu Ile Glu Leu Gly  Ser Leu Leu
       1055                 1060                1065
   Arg Gln  Asp Phe Gln Gly Leu  His Asn Glu Leu Leu  Leu Arg Ile
       1070                 1075                1080
   Gly Glu  His Tyr Gln Gln Val  Phe Asn Gly Leu Ser  Ile Leu Ala
       1085                 1090                1095
   Ser Phe  Ala Ser Ser Asp Asp  Pro Tyr Gln Gly Pro  Arg Asp Ile
       1100                 1105                1110
   Ile Ser  Pro Glu Leu Met Ala  Asp Tyr Leu Gln Pro  Lys Leu Leu
       1115                 1120                1125
   Gly Ile  Leu Ala Phe Phe Asn  Met Gln Leu Leu Ser  Ser Ser Val
       1130                 1135                1140
   Gly Ile  Glu Asp Lys Lys Met  Ala Leu Asn Ser Leu  Met Ser Leu
       1145                 1150                1155
   Met Lys  Leu Met Gly Pro Lys  His Val Ser Ser Val  Arg Val Lys
       1160                 1165                1170
   Met Met  Thr Thr Leu Arg Thr  Gly Leu Arg Phe Lys  Asp Asp Phe
       1175                 1180                1185
   Pro Glu  Leu Cys Cys Arg Ala  Trp Asp Cys Phe Val  Arg Cys Leu
       1190                 1195                1200
   Asp His Ala Cys Leu Gly Ser Leu Leu Ser His Val Ile Val Ala
       1205                1210                1215
   Leu Leu Pro Leu Ile His Ile Gln Pro Lys Glu Thr Ala Ala Ile
       1220                1225                1230
   Phe His Tyr Leu Ile Ile Glu Asn Arg Asp Ala Val Gln Asp Phe
       1235                1240                1245
   Leu His Glu Ile Tyr Phe Leu Pro Asp His Pro Glu Leu Lys Lys
       1250                1255                1260
   Ile Lys Ala Val Leu Gln Glu Tyr Arg Lys Glu Thr Ser Glu Ser
       1265                1270                1275
   Thr Asp Leu Gln Thr Thr Leu Gln Leu Ser Met Lys Ala Ile Gln
       1280                1285                1290
   His Glu Asn Val Asp Val Arg Ile His Ala Leu Thr Ser Leu Lys
       1295                1300                1305
   Glu Thr Leu Tyr Lys Asn Gln Glu Lys Leu Ile Lys Tyr Ala Thr
       1310                1315                1320
   Asp Ser Glu Thr Val Glu Pro Ile Ile Ser Gln Leu Val Thr Val
       1325                1330                1335
   Leu Leu Lys Gly Cys Gln Asp Ala Asn Ser Gln Ala Arg Leu Leu
       1340                1345                1350
   Cys Gly Glu Cys Leu Gly Glu Leu Gly Ala Ile Asp Pro Gly Arg
       1355                1360                1365
   Leu Asp Phe Ser Thr Thr Glu Thr Gln Gly Lys Asp Phe Thr Phe
       1370                1375                1380
   Val Thr Gly Val Glu Asp Ser Ser Phe Ala Tyr Gly Leu Leu Met
       1385                1390                1395
   Glu Leu Thr Arg Ala Tyr Leu Ala Tyr Ala Asp Asn Ser Arg Ala
       1400                1405                1410
   Gln Asp Ser Ala Ala Tyr Ala Ile Gln Glu Leu Leu Ser Ile Tyr
       1415                1420                1425
   Asp Cys Arg Glu Met Glu Thr Asn Gly Pro Gly His Gln Leu Trp
       1430                1435                1440
   Arg Arg Phe Pro Glu His Val Arg Glu Ile Leu Glu Pro His Leu
       1445                1450                1455
   Asn Thr Arg Tyr Lys Ser Ser Gln Lys Ser Thr Asp Trp Ser Gly
   1460 1465 1470
   Val Lys Lys Pro Ile Tyr Leu Ser Lys Leu Gly Ser Asn Phe Ala
   1475 1480 1485
   Glu Trp Ser Ala Ser Trp Ala Gly Tyr Leu Ile Thr Lys Val Arg
   1490 1495 1500
   His Asp Leu Ala Ser Lys Ile Phe Thr Cys Cys Ser Ile Met Met
   1505 1510 1515
   Lys His Asp Phe Lys Val Thr Ile Tyr Leu Leu Pro His Ile Leu
   1520 1525 1530
   Val Tyr Val Leu Leu Gly Cys Asn Gln Glu Asp Gln Gln Glu Val
   1535 1540 1545
   Tyr Ala Glu Ile Met Ala Val Leu Lys His Asp Asp Gln His Thr
   1550 1555 1560
   Ile Asn Thr Gln Asp Ile Ala Ser Asp Leu Cys Gln Leu Ser Thr
   1565 1570 1575
   Gln Thr Val Phe Ser Met Leu Asp His Leu Thr Gln Trp Ala Arg
   1580 1585 1590
   His Lys Phe Gln Ala Leu Lys Ala Glu Lys Cys Pro His Ser Lys
   1595 1600 1605
   Ser Asn Arg Asn Lys Val Asp Ser Met Val Ser Thr Val Asp Tyr
   1610 1615 1620
   Glu Asp Tyr Gln Ser Val Thr Arg Phe Leu Asp Leu Ile Pro Gln
   1625 1630 1635
   Asp Thr Leu Ala Val Ala Ser Phe Arg Ser Lys Ala Tyr Thr Arg
   1640 1645 1650
   Ala Val Met His Phe Glu Ser Phe Ile Thr Glu Lys Lys Gln Asn
   1655 1660 1665
   Ile Gln Glu His Leu Gly Phe Leu Gln Lys Leu Tyr Ala Ala Met
   1670 1675 1680
   His Glu Pro Asp Gly Val Ala Gly Val Ser Ala Ile Arg Lys Ala
   1685 1690 1695
   Glu Pro Ser Leu Lys Glu Gln Ile Leu Glu His Glu Ser Leu Gly
   1700 1705 1710
   Leu Leu Arg Asp Ala Thr Ala Cys Tyr Asp Arg Ala Ile Gln Leu
   1715                    1720                    1725
   Glu Pro Asp Gln Ile Ile His Tyr His Gly Val Val Lys Ser Met
       1730                    1735                    1740
   Leu Gly Leu Gly Gln Leu Ser Thr Val Ile Thr Gln Val Asn Gly
       1745                    1750                    1755
   Val His Ala Asn Arg Ser Glu Trp Thr Asp Glu Leu Asn Thr Tyr
       1760                    1765                    1770
   Arg Val Glu Ala Ala Trp Lys Leu Ser Gln Trp Asp Leu Val Glu
       1775                    1780                    1785
   Asn Tyr Leu Ala Ala Asp Gly Lys Ser Thr Thr Trp Ser Val Arg
       1790                    1795                    1800
   Leu Gly Gln Leu Leu Leu Ser Ala Lys Lys Arg Asp Ile Thr Ala
       1805                    1810                    1815
   Phe Tyr Asp Ser Leu Lys Leu Val Arg Ala Glu Gln Ile Val Pro
       1820                    1825                    1830
   Leu Ser Ala Ala Ser Phe Glu Arg Gly Ser Tyr Gln Arg Gly Tyr
       1835                    1840                    1845
   Glu Tyr Ile Val Arg Leu His Met Leu Cys Glu Leu Glu His Ser
       1850                    1855                    1860
   Ile Lys Pro Leu Phe Gln His Ser Pro Gly Asp Ser Ser Gln Glu
       1865                    1870                    1875
   Asp Ser Leu Asn Trp Val Ala Arg Leu Glu Met Thr Gln Asn Ser
       1880                    1885                    1890
   Tyr Arg Ala Lys Glu Pro Ile Leu Ala Leu Arg Arg Ala Leu Leu
       1895                    1900                    1905
   Ser Leu Asn Lys Arg Pro Asp Tyr Asn Glu Met Val Gly Glu Cys
       1910                    1915                    1920
   Trp Leu Gln Ser Ala Arg Val Ala Arg Lys Ala Gly His His Gln
       1925                    1930                    1935
   Thr Ala Tyr Asn Ala Leu Leu Asn Ala Gly Glu Ser Arg Leu Ala
       1940                    1945                    1950
   Glu Leu Tyr Val Glu Arg Ala Lys Trp Leu Trp Ser Lys Gly Asp
       1955                    1960                    1965
   Val His Gln Ala Leu Ile Val Leu Gln Lys Gly Val Glu Leu Cys
       1970                    1975                    1980
   Phe Pro Glu Asn Glu Thr Pro Pro Glu Gly Lys Asn Met Leu Ile
       1985                1990                1995
   His Gly Arg Ala Met Leu Leu Val Gly Arg Phe Met Glu Glu Thr
       2000                2005                2010
   Ala Asn Phe Glu Ser Asn Ala Ile Met Lys Lys Tyr Lys Asp Val
       2015                2020                2025
   Thr Ala Cys Leu Pro Glu Trp Glu Asp Gly His Phe Tyr Leu Ala
       2030                2035                2040
   Lys Tyr Tyr Asp Lys Leu Met Pro Met Val Thr Asp Asn Lys Met
       2045                2050                2055
   Glu Lys Gln Gly Asp Leu Ile Arg Tyr Ile Val Leu His Phe Gly
       2060                2065                2070
   Arg Ser Leu Gln Tyr Gly Asn Gln Phe Ile Tyr Gln Ser Met Pro
       2075                2080                2085
   Arg Met Leu Thr Leu Trp Leu Asp Tyr Gly Thr Lys Ala Tyr Glu
       2090                2095                2100
   Trp Glu Lys Ala Gly Arg Ser Asp Arg Val Gln Met Arg Asn Asp
       2105                2110                2115
   Leu Gly Lys Ile Asn Lys Val Ile Thr Glu His Thr Asn Tyr Leu
       2120                2125                2130
   Ala Pro Tyr Gln Phe Leu Thr Ala Phe Ser Gln Leu Ile Ser Arg
       2135                2140                2145
   Ile Cys His Ser His Asp Glu Val Phe Val Val Leu Met Glu Ile
       2150                2155                2160
   Ile Ala Lys Val Phe Leu Ala Tyr Pro Gln Gln Ala Met Trp Met
       2165                2170                2175
   Met Thr Ala Val Ser Lys Ser Ser Tyr Pro Met Arg Val Asn Arg
       2180                2185                2190
   Cys Lys Glu Ile Leu Asn Lys Ala Ile His Met Lys Lys Ser Leu
       2195                2200                2205
   Glu Lys Phe Val Gly Asp Ala Thr Arg Leu Thr Asp Lys Leu Leu
       2210                2215                2220
   Glu Leu Cys Asn Lys Pro Val Asp Gly Ser Ser Ser Thr Leu Ser
       2225                2230                2235
   Met Ser Thr His Phe Lys Met Leu Lys Lys Leu Val Glu Glu Ala
       2240                2245                2250
   Thr Phe Ser Glu Ile Leu Ile Pro Leu Gln Ser Val Met Ile Pro
       2255                2260                2265
   Thr Leu Pro Ser Ile Leu Gly Thr His Ala Asn His Ala Ser His
       2270                2275                2280
   Glu Pro Phe Pro Gly His Trp Ala Tyr Ile Ala Gly Phe Asp Asp
       2285                2290                2295
   Met Val Glu Ile Leu Ala Ser Leu Gln Lys Pro Lys Lys Ile Ser
       2300                2305                2310
   Leu Lys Gly Ser Asp Gly Lys Phe Tyr Ile Met Met Cys Lys Pro
       2315                2320                2325
   Lys Asp Asp Leu Arg Lys Asp Cys Arg Leu Met Glu Phe Asn Ser
       2330                2335                2340
   Leu Ile Asn Lys Cys Leu Arg Lys Asp Ala Glu Ser Arg Arg Arg
       2345                2350                2355
   Glu Leu His Ile Arg Thr Tyr Ala Val Ile Pro Leu Asn Asp Glu
       2360                2365                2370
   Cys Gly Ile Ile Glu Trp Val Asn Asn Thr Ala Gly Leu Arg Pro
       2375                2380                2385
   Ile Leu Thr Lys Leu Tyr Lys Glu Lys Gly Val Tyr Met Thr Gly
       2390                2395                2400
   Lys Glu Leu Arg Gln Cys Met Leu Pro Lys Ser Ala Ala Leu Ser
       2405                2410                2415
   Glu Lys Leu Lys Val Phe Arg Glu Phe Leu Leu Pro Arg His Pro
       2420                2425                2430
   Pro Ile Phe His Glu Trp Phe Leu Arg Thr Phe Pro Asp Pro Thr
       2435                2440                2445
   Ser Trp Tyr Ser Ser Arg Ser Ala Tyr Cys Arg Ser Thr Ala Val
       2450                2455                2460
   Met Ser Met Val Gly Tyr Ile Leu Gly Leu Gly Asp Arg His Gly
       2465                2470                2475
   Glu Asn Ile Leu Phe Asp Ser Leu Thr Gly Glu Cys Val His Val
       2480                2485                2490
   Asp Phe Asn Cys Leu Phe Asn Lys Gly Glu Thr Phe Glu Val Pro
       2495                    2500                    2505
   Glu Ile Val Pro Phe Arg Leu Thr His Asn Met Val Asn Gly Met
       2510                    2515                    2520
   Gly Pro Met Gly Thr Glu Gly Leu Phe Arg Arg Ala Cys Glu Val
       2525                    2530                    2535
   Thr Met Arg Leu Met Arg Asp Gln Arg Glu Pro Leu Met Ser Val
       2540                    2545                    2550
   Leu Lys Thr Phe Leu His Asp Pro Leu Val Glu Trp Ser Lys Pro
       2555                    2560                    2565
   Val Lys Gly His Ser Lys Ala Pro Leu Asn Glu Thr Gly Glu Val
       2570                    2575                    2580
   Val Asn Glu Lys Ala Lys Thr His Val Leu Asp Ile Glu Gln Arg
       2585                    2590                    2595
   Leu Gln Gly Val Ile Lys Thr Arg Asn Arg Val Thr Gly Leu Pro
       2600                    2605                    2610
   Leu Ser Ile Glu Gly His Val His Tyr Leu Ile Gln Glu Ala Thr
       2615                    2620                    2625
   Asp Glu Asn Leu Leu Cys Gln Met Tyr Leu Gly Trp Thr Pro Tyr
       2630                    2635                    2640
   Met

<210>  7
<211>  3056
<212>  PRT
<213>  Homo sapiens

<400>  7

<210>  8
<211>  2551
<212>  PRT
<213>  Homo sapiens

<400>  8

Met Leu Gly Thr Gly Pro Ala Ala Ala Thr Thr Ala Ala Thr Thr Ser
1               5                   10                  15

Ser Asn Val Ser Val Leu Gln Gln Phe Ala Ser Gly Leu Lys Ser Arg
            20                  25                  30

Asn Glu Glu Thr Arg Ala Lys Ala Ala Lys Glu Leu Gln His Tyr Val
        35                  40                  45

Thr Met Glu Leu Arg Glu Met Ser Gln Glu Glu Ser Thr Arg Phe Tyr
    50                  55                  60

Asp Gln Leu Asn His His Ile Phe Glu Leu Val Ser Ser Ser Asp Ala
65                  70                  75                  80

Asn Glu Arg Lys Gly Gly Ile Leu Ala Ile Ala Ser Leu Ile Gly Val
                85                  90                  95

Glu Gly Gly Asn Ala Thr Arg Ile Gly Arg Phe Ala Asn Tyr Leu Arg
            100                 105                 110

Asn Leu Leu Pro Ser Asn Asp Pro Val Val Met Glu Met Ala Ser Lys
        115                 120                 125

Ala Ile Gly Arg Leu Ala Met Ala Gly Asp Thr Phe Thr Ala Glu Tyr
    130                 135                 140

Val Glu Phe Glu Val Lys Arg Ala Leu Glu Trp Leu Gly Ala Asp Arg
145                 150                 155                 160

Asn Glu Gly Arg Arg His Ala Ala Val Leu Val Leu Arg Glu Leu Ala
                165                 170                 175

Ile Ser Val Pro Thr Phe Phe Phe Gln Gln Val Gln Pro Phe Phe Asp
            180                 185                 190

Asn Ile Phe Val Ala Val Trp Asp Pro Lys Gln Ala Ile Arg Glu Gly
        195                 200                 205

Ala Val Ala Ala Leu Arg Ala Cys Leu Ile Leu Thr Thr Gln Arg Glu
    210                 215                 220

Pro Lys Glu Met Gln Lys Pro Gln Trp Tyr Arg His Thr Phe Glu Glu
225                 230                 235                 240

Ala Glu Lys Gly Phe Asp Glu Thr Leu Ala Lys Glu Lys Gly Met Asn
                245                 250                 255

Arg Asp Asp Arg Ile His Gly Ala Leu Leu Ile Leu Asn Glu Leu Val
            260                 265                 270

Arg Ile Ser Ser Met Glu Gly Glu Arg Leu Arg Glu Glu Met Glu Glu
        275                 280                 285

Ile Thr Gln Gln Gln Leu Val His Asp Lys Tyr Cys Lys Asp Leu Met
    290                 295                 300

Gly Phe Gly Thr Lys Pro Arg His Ile Thr Pro Phe Thr Ser Phe Gln
305                 310                 315                 320

Ala Val Gln Pro Gln Gln Ser Asn Ala Leu Val Gly Leu Leu Gly Tyr
                325                 330                 335

Ser Ser His Gln Gly Leu Met Gly Phe Gly Thr Ser Pro Ser Pro Ala
            340                 345                 350

Lys Ser Thr Leu Val Glu Ser Arg Cys Cys Arg Asp Leu Met Glu Glu
        355                 360                 365

Lys Phe Asp Gln Val Cys Gln Trp Val Leu Lys Cys Arg Asn Ser Lys
    370                 375                 380

Asn Ser Leu Ile Gln Met Thr Ile Leu Asn Leu Leu Pro Arg Leu Ala
385                 390                 395                 400

Ala Phe Arg Pro Ser Ala Phe Thr Asp Thr Gln Tyr Leu Gln Asp Thr
                405                 410                 415

Met Asn His Val Leu Ser Cys Val Lys Lys Glu Lys Glu Arg Thr Ala
            420                 425                 430

Ala Phe Gln Ala Leu Gly Leu Leu Ser Val Ala Val Arg Ser Glu Phe
        435                 440                 445

Lys Val Tyr Leu Pro Arg Val Leu Asp Ile Ile Arg Ala Ala Leu Pro
    450                 455                 460

Pro Lys Asp Phe Ala His Lys Arg Gln Lys Ala Met Gln Val Asp Ala
465                 470                 475                 480

Thr Val Phe Thr Cys Ile Ser Met Leu Ala Arg Ala Met Gly Pro Gly
                485                 490                 495

Ile Gln Gln Asp Ile Lys Glu Leu Leu Glu Pro Met Leu Ala Val Gly
            500                 505                 510

Leu Ser Pro Ala Leu Thr Ala Val Leu Tyr Asp Leu Ser Arg Gln Ile
        515                 520                 525

Pro Gln Leu Lys Lys Asp Ile Gln Asp Gly Leu Leu Lys Met Leu Ser
    530                 535                 540

Leu Val Leu Met His Lys Pro Leu Arg His Pro Gly Met Pro Lys Gly
545                 550                 555                 560

Leu Ala His Gln Leu Ala Ser Pro Gly Leu Thr Thr Leu Pro Glu Ala
                565                 570                 575

Ser Asp Val Gly Ser Ile Thr Leu Ala Leu Arg Thr Leu Gly Ser Phe
            580                 585                 590

Glu Phe Glu Gly His Ser Leu Thr Gln Phe Val Arg His Cys Ala Asp
        595                 600                 605

His Phe Leu Asn Ser Glu His Lys Glu Ile Arg Met Glu Ala Ala Arg
    610                 615                 620

Thr Cys Ser Arg Leu Leu Thr Pro Ser Ile His Leu Ile Ser Gly His
625                 630                 635                 640

Ala His Val Val Ser Gln Thr Ala Val Gln Val Val Ala Asp Val Leu
                645                 650                 655

Ser Lys Leu Leu Val Val Gly Ile Thr Asp Pro Asp Pro Asp Ile Arg
            660                 665                 670

Tyr Cys Val Leu Ala Ser Leu Asp Glu Arg Phe Asp Ala His Leu Ala
        675                 680                 685

Gln Ala Glu Asn Leu Gln Ala Leu Phe Val Ala Leu Asn Asp Gln Val
    690                 695                 700

Phe Glu Ile Arg Glu Leu Ala Ile Cys Thr Val Gly Arg Leu Ser Ser

705                 710                 715                 720

Met Asn Pro Ala Phe Val Met Pro Phe Leu Arg Lys Met Leu Ile Gln
                725                 730                 735

Ile Leu Thr Glu Leu Glu His Ser Gly Ile Gly Arg Ile Lys Glu Gln
            740                 745                 750

Ser Ala Arg Met Leu Gly His Leu Val Ser Asn Ala Pro Arg Leu Ile
        755                 760                 765

Arg Pro Tyr Met Glu Pro Ile Leu Lys Ala Leu Ile Leu Lys Leu Lys
    770                 775                 780

Asp Pro Asp Pro Asp Pro Asn Pro Gly Val Ile Asn Asn Val Leu Ala
785                 790                 795                 800

Thr Ile Gly Glu Leu Ala Gln Val Ser Gly Leu Glu Met Arg Lys Trp
                805                 810                 815

Val Asp Glu Leu Phe Ile Ile Ile Met Asp Met Leu Gln Asp Ser Ser
            820                 825                 830

Leu Leu Ala Lys Arg Gln Val Ala Leu Trp Thr Leu Gly Gln Leu Val
        835                 840                 845

Ala Ser Thr Gly Tyr Val Val Glu Pro Tyr Arg Lys Tyr Pro Thr Leu
    850                 855                 860

Leu Glu Val Leu Leu Asn Phe Leu Lys Thr Glu Gln Asn Gln Gly Thr
865                 870                 875                 880

Arg Arg Glu Ala Ile Arg Val Leu Gly Leu Leu Gly Ala Leu Asp Pro
                885                 890                 895

Tyr Lys His Lys Val Asn Ile Gly Met Ile Asp Gln Ser Arg Asp Ala
            900                 905                 910

Ser Ala Val Ser Leu Ser Glu Ser Lys Ser Ser Gln Asp Ser Ser Asp
        915                 920                 925

Tyr Ser Thr Ser Glu Met Leu Val Asn Met Gly Asn Leu Pro Leu Asp
    930                 935                 940

Glu Phe Tyr Pro Ala Val Ser Met Val Ala Leu Met Arg Ile Phe Arg
945                 950                 955                 960

Asp Gln Ser Leu Ser His His His Thr Met Val Val Gln Ala Ile Thr
                965                 970                 975

Phe Ile Phe Lys Ser Leu Gly Leu Lys Cys Val Gln Phe Leu Pro Gln
            980                 985                 990

Val Met Pro Thr Phe Leu Asn Val Ile Arg Val Cys Asp Gly Ala Ile
        995                 1000                1005

Arg Glu Phe Leu Phe Gln Gln Leu Gly Met Leu Val Ser Phe Val
    1010                1015                1020

Lys Ser His Ile Arg Pro Tyr Met Asp Glu Ile Val Thr Leu Met
    1025                1030                1035

Arg Glu Phe Trp Val Met Asn Thr Ser Ile Gln Ser Thr Ile Ile
    1040                1045                1050

Leu Leu Ile Glu Gln Ile Val Val Ala Leu Gly Gly Glu Phe Lys
    1055                1060                1065

Leu Tyr Leu Pro Gln Leu Ile Pro His Met Leu Arg Val Phe Met
    1070                1075                1080

His Asp Asn Ser Pro Gly Arg Ile Val Ser Ile Lys Leu Leu Ala
    1085                1090                1095

Ala Ile Gln Leu Phe Gly Ala Asn Leu Asp Asp Tyr Leu His Leu
    1100                1105                1110

Leu Leu Pro Pro Ile Val Lys Leu Phe Asp Ala Pro Glu Ala Pro
    1115                1120                1125

Leu Pro Ser Arg Lys Ala Ala Leu Glu Thr Val Asp Arg Leu Thr
    1130                1135                1140

Glu Ser Leu Asp Phe Thr Asp Tyr Ala Ser Arg Ile Ile His Pro
    1145                1150                1155

Ile Val Arg Thr Leu Asp Gln Ser Pro Glu Leu Arg Ser Thr Ala
    1160                1165                1170

Met Asp Thr Leu Ser Ser Leu Val Phe Gln Leu Gly Lys Lys Tyr
    1175                1180                1185

Gln Ile Phe Ile Pro Met Val Asn Lys Val Leu Val Arg His Arg
    1190                1195                1200

Ile Asn His Gln Arg Tyr Asp Val Leu Ile Cys Arg Ile Val Lys
    1205                1210                1215

Gly Tyr Thr Leu Ala Asp Glu Glu Glu Asp Pro Leu Ile Tyr Gln
    1220                1225                1230

His Arg Met Leu Arg Ser Gly Gln Gly Asp Ala Leu Ala Ser Gly
    1235                1240                1245

Pro Val Glu Thr Gly Pro Met Lys Lys Leu His Val Ser Thr Ile
    1250                1255                1260

Asn Leu Gln Lys Ala Thr Ala Trp Gly Ala Ala Arg Arg Val Ser
    1265                1270                1275

Lys Asp Asp Trp Leu Glu Trp Leu Arg Arg Leu Ser Leu Glu Leu
    1280                1285                1290

Leu Lys Asp Ser Ser Ser Pro Ser Leu Arg Ser Cys Trp Ala Leu
    1295                1300                1305

Ala Gln Ala Tyr Asn Pro Met Ala Arg Asp Leu Phe Asn Ala Ala
    1310                1315                1320

Phe Val Ser Cys Trp Ser Glu Leu Asn Glu Asp Gln Gln Asp Glu
    1325                1330                1335

Leu Ile Arg Ser Ile Glu Leu Ala Leu Thr Ser Gln Asp Ile Ala
    1340                1345                1350

Glu Val Thr Gln Thr Leu Leu Asn Leu Ala Glu Phe Met Glu His
    1355                1360                1365

Ser Asp Lys Gly Pro Leu Pro Leu Arg Asp Asp Asn Gly Ile Val
    1370                1375                1380

Leu Leu Gly Glu Arg Ala Ala Lys Cys Arg Ala Tyr Ala Lys Ala
    1385                1390                1395

Leu His Tyr Lys Glu Leu Glu Phe Gln Lys Gly Pro Thr Pro Ala
    1400                1405                1410

Ile Leu Glu Ser Leu Ile Ser Ile Asn Asn Lys Leu Gln Gln Pro
    1415                1420                1425

Glu Ala Ala Ala Gly Val Leu Glu Tyr Ala Met Lys His Phe Gly
    1430                1435                1440

Glu Leu Glu Ile Gln Ala Thr Trp Tyr Glu Lys Leu His Glu Trp
    1445                1450                1455

Glu Asp Ala Leu Val Ala Tyr Asp Lys Lys Met Asp Thr Asn Lys
    1460                1465                1470

Asp Asp Pro Glu Leu Met Leu Gly Arg Met Arg Cys Leu Glu Ala
    1475                1480                1485

Leu Gly Glu Trp Gly Gln Leu His Gln Gln Cys Cys Glu Lys Trp
    1490                1495                1500

Thr Leu Val Asn Asp Glu Thr Gln Ala Lys Met Ala Arg Met Ala
    1505                1510                1515

Ala Ala Ala Ala Trp Gly Leu Gly Gln Trp Asp Ser Met Glu Glu
    1520                1525                1530

Tyr Thr Cys Met Ile Pro Arg Asp Thr His Asp Gly Ala Phe Tyr
    1535                1540                1545

Arg Ala Val Leu Ala Leu His Gln Asp Leu Phe Ser Leu Ala Gln
    1550                1555                1560

Gln Cys Ile Asp Lys Ala Arg Asp Leu Leu Asp Ala Glu Leu Thr
    1565                1570                1575

Ala Met Ala Gly Glu Ser Tyr Ser Arg Ala Tyr Gly Ala Met Val
    1580                1585                1590

Ser Cys His Met Leu Ser Glu Leu Glu Glu Val Ile Gln Tyr Lys
    1595                1600                1605

Leu Val Pro Glu Arg Arg Glu Ile Ile Arg Gln Ile Trp Trp Glu
    1610                1615                1620

Arg Leu Gln Gly Cys Gln Arg Ile Val Glu Asp Trp Gln Lys Ile
    1625                1630                1635

Leu Met Val Arg Ser Leu Val Val Ser Pro His Glu Asp Met Arg
    1640                1645                1650

Thr Trp Leu Lys Tyr Ala Ser Leu Cys Gly Lys Ser Gly Arg Leu
    1655                1660                1665

Ala Leu Ala His Lys Thr Leu Val Leu Leu Leu Gly Val Asp Pro
    1670                1675                1680

Ser Arg Gln Leu Asp His Pro Leu Pro Thr Val His Pro Gln Val
    1685                1690                1695

Thr Tyr Ala Tyr Met Lys Asn Met Trp Lys Ser Ala Arg Lys Ile
    1700                1705                1710

Asp Ala Phe Gln His Met Gln His Phe Val Gln Thr Met Gln Gln
    1715                1720                1725

Gln Ala Gln His Ala Ile Ala Thr Glu Asp Gln Gln His Lys Gln
    1730                1735                1740

Glu Leu His Lys Leu Met Ala Arg Cys Phe Leu Lys Leu Gly Glu
    1745                1750                1755

Trp Gln Leu Asn Leu Gln Gly Ile Asn Glu Ser Thr Ile Pro Lys

1760                    1765                    1770

Val Leu Gln Tyr Tyr Ser Ala Ala Thr Glu His Asp Arg Ser Trp
    1775                1780                1785

Tyr Lys Ala Trp His Ala Trp Ala Val Met Asn Phe Glu Ala Val
    1790                1795                1800

Leu His Tyr Lys His Gln Asn Gln Ala Arg Asp Glu Lys Lys Lys
    1805                1810                1815

Leu Arg His Ala Ser Gly Ala Asn Ile Thr Asn Ala Thr Thr Ala
    1820                1825                1830

Ala Thr Thr Ala Ala Thr Ala Thr Thr Thr Ala Ser Thr Glu Gly
    1835                1840                1845

Ser Asn Ser Glu Ser Glu Ala Glu Ser Thr Glu Asn Ser Pro Thr
    1850                1855                1860

Pro Ser Pro Leu Gln Lys Lys Val Thr Glu Asp Leu Ser Lys Thr
    1865                1870                1875

Leu Leu Met Tyr Thr Val Pro Ala Val Gln Gly Phe Phe Arg Ser
    1880                1885                1890

Ile Ser Leu Ser Arg Gly Asn Asn Leu Gln Asp Thr Leu Arg Val
    1895                1900                1905

Leu Thr Leu Trp Phe Asp Tyr Gly His Trp Pro Asp Val Asn Glu
    1910                1915                1920

Ala Leu Val Glu Gly Val Lys Ala Ile Gln Ile Asp Thr Trp Leu
    1925                1930                1935

Gln Val Ile Pro Gln Leu Ile Ala Arg Ile Asp Thr Pro Arg Pro
    1940                1945                1950

Leu Val Gly Arg Leu Ile His Gln Leu Leu Thr Asp Ile Gly Arg
    1955                1960                1965

Tyr His Pro Gln Ala Leu Ile Tyr Pro Leu Thr Val Ala Ser Lys
    1970                1975                1980

Ser Thr Thr Thr Ala Arg His Asn Ala Ala Asn Lys Ile Leu Lys
    1985                1990                1995

Asn Met Cys Glu His Ser Asn Thr Leu Val Gln Gln Ala Met Met
    2000                2005                2010

Val Ser Glu Glu Leu Ile Arg Val Ala Ile Leu Trp His Glu Met
    2015                2020                2025

Trp His Glu Gly Leu Glu Glu Ala Ser Arg Leu Tyr Phe Gly Glu
    2030                2035                2040

Arg Asn Val Lys Gly Met Phe Glu Val Leu Glu Pro Leu His Ala
    2045                2050                2055

Met Met Glu Arg Gly Pro Gln Thr Leu Lys Glu Thr Ser Phe Asn
    2060                2065                2070

Gln Ala Tyr Gly Arg Asp Leu Met Glu Ala Gln Glu Trp Cys Arg
    2075                2080                2085

Lys Tyr Met Lys Ser Gly Asn Val Lys Asp Leu Thr Gln Ala Trp
    2090                2095                2100

Asp Leu Tyr Tyr His Val Phe Arg Arg Ile Ser Lys Gln Leu Pro
    2105                2110                2115

Gln Leu Thr Ser Leu Glu Leu Gln Tyr Val Ser Pro Lys Leu Leu
    2120                2125                2130

Met Cys Arg Asp Leu Glu Leu Ala Val Pro Gly Thr Tyr Asp Pro
    2135                2140                2145

Asn Gln Pro Ile Ile Arg Ile Gln Ser Ile Ala Pro Ser Leu Gln
    2150                2155                2160

Val Ile Thr Ser Lys Gln Arg Pro Arg Lys Leu Thr Leu Met Gly
    2165                2170                2175

Ser Asn Gly His Glu Phe Val Phe Leu Leu Lys Gly His Glu Asp
    2180                2185                2190

Leu Arg Gln Asp Glu Arg Val Met Gln Leu Phe Gly Leu Val Asn
    2195                2200                2205

Thr Leu Leu Ala Asn Asp Pro Thr Ser Leu Arg Lys Asn Leu Ser
    2210                2215                2220

Ile Gln Arg Tyr Ala Val Ile Pro Leu Ser Thr Asn Ser Gly Leu
    2225                2230                2235

Ile Gly Trp Val Pro His Cys Asp Thr Leu His Ala Leu Ile Arg
    2240                2245                2250

Asp Tyr Arg Glu Lys Lys Lys Ile Leu Leu Asn Ile Glu His Arg
    2255                2260                2265

Ile Met Leu Arg Met Ala Pro Asp Tyr Asp His Leu Thr Leu Met
    2270                2275                2280

Gln Lys Val Glu Val Phe Glu His Ala Val Asn Asn Thr Ala Gly
    2285                2290                2295

Asp Asp Leu Ala Lys Leu Leu Trp Leu Lys Ser Pro Ser Ser Glu
    2300                2305                2310

Val Trp Phe Asp Arg Arg Thr Asn Tyr Thr Arg Ser Leu Ala Val
    2315                2320                2325

Met Ser Met Val Gly Tyr Ile Leu Gly Leu Gly Asp Arg His Pro
    2330                2335                2340

Ser Asn Leu Met Leu Asp Arg Leu Ser Gly Lys Ile Leu His Ile
    2345                2350                2355

Asp Phe Gly Asp Cys Phe Glu Val Ala Met Thr Arg Glu Lys Phe
    2360                2365                2370

Pro Glu Lys Ile Pro Phe Arg Leu Thr Arg Met Leu Thr Asn Ala
    2375                2380                2385

Met Glu Val Thr Gly Leu Asp Gly Asn Tyr Arg Ile Thr Cys His
    2390                2395                2400

Thr Val Met Glu Val Leu Arg Glu His Lys Asp Ser Val Met Ala
    2405                2410                2415

Val Leu Glu Ala Phe Val Tyr Asp Pro Leu Leu Asn Trp Arg Leu
    2420                2425                2430

Met Asp Thr Asn Thr Lys Gly Asn Lys Arg Ser Arg Thr Arg Thr
    2435                2440                2445

Asp Ser Tyr Ser Ala Gly Gln Ser Val Glu Ile Leu Asp Gly Val
    2450                2455                2460

Glu Leu Gly Glu Pro Ala His Lys Lys Thr Gly Thr Thr Val Pro
    2465                2470                2475

Glu Ser Ile His Ser Phe Ile Gly Asp Gly Leu Val Lys Pro Glu
    2480                2485                2490

Ala Leu Asn Lys Lys Ala Ile Gln Ile Ile Asn Arg Val Arg Asp
    2495                2500                2505

Lys Leu Thr Gly Arg Asp Phe Ser His Asp Asp Thr Leu Asp Val
    2510                2515                2520

Pro Thr Gln Val Glu Leu Leu Ile Lys Gln Ala Thr Ser His Glu
    2525                2530                2535

Asn Leu Cys Gln Cys Tyr Ile Gly Trp Cys Pro Phe Trp
    2540                2545                2550